# FASCICULE DE BREVET EUROPEEN

(11) **EP 1 235 777 B1**
(45) Date de publication et mention de la délivrance du brevet: **16.06.2004**
(21) Numéro de dépôt: 00988868.6
(22) Date de dépôt: 22.11.2000
(51) Int. Cl.: C07C 43/23, C07C 323/21, C07D 333/56, C07D 213/79, A61K 31/10, A61K 31/085, A61K 7/06, A61K 7/48, A61P 17/00

(54) **ANALOGUES DE LA VITAMINE D**
VITAMIN-D ANALOGA
VITAMIN D ANALOGUES

(30) Priorité: 24.11.1999 FR 9914781
(43) Date de publication de la demande: 04.09.2002
(73) Titulaire: Galderma Research & Development, S.N.C., 06560 Valbonne (FR)
(72) Inventeur: BERNARDON, Jean-Michel, F-06000 Nice (FR); BIADATTI, Thibaud, F-06650 Opio (FR)
(74) Mandataire: Allab, Myriam
(86) Numéro de dépôt international: PCT/FR2000/003249
(87) Numéro de publication internationale: WO 2001/038303

(56) Documents cités:
- EP-A- 0 375 368
- EP-A- 0 661 260
- EP-A- 0 728 739
- EP-A- 0 879 814
- EP-A- 0 947 496
- WO-A-95/27692
- WO-A-98/20866
- PATENT ABSTRACTS OF JAPAN vol. 1996, no. 11, 29 novembre 1996 (1996-11-29) -& JP 08 188650 A (IDEMITSU KOSAN CO LTD), 23 juillet 1996 (1996-07-23)

## Description

L'invention concerne, à titre de produits industriels nouveaux et utiles, des composés tri-aromatiques analogues de la vitamine D. Elle concerne également leur procédé de préparation et leur utilisation dans des compositions pharmaceutiques destinées à un usage en médecine humaine ou vétérinaire, ou bien encore dans des compositions cosmétiques.

Les composés selon l'invention ont une activité marquée dans les domaines de la prolifération et de la différenciation cellulaire et trouvent des applications plus particulièrement dans le traitement topique et systémique des affections dermatologiques (ou autres) liées à un désordre de la kératinisation, des affections à composante inflammatoire et/ou immunoallergique et de l'hyperprolifération des tissus d'origine ectodermique (peau, épithélium...), qu'elle soit bénigne ou maligne. Ces composés peuvent en outre être utilisés pour lutter contre le vieillissement de la peau, qu'il soit photoinduit ou chronologique et traiter les troubles de la cicatrisation.

On peut également utiliser les composés selon l'invention dans des compositions cosmétiques pour l'hygiène corporelle et capillaire.

La vitamine D est une vitamine essentielle pour la prévention et le traitement des défauts de minéralisation du cartilage (rachitisme), et de l'os (ostéomalacie), et même de certaines formes d'ostéoporose chez le sujet âgé. Mais, il est maintenant admis que ses fonctions s'étendent bien au delà de la régulation du métabolisme osseux et de l'homéostasie calcique. Parmi celles-ci, peuvent être citées ses actions sur la prolifération et sur la différenciation cellulaire et le contrôle des défenses immunitaires. Leur découverte a ouvert la voie à de nouvelles approches thérapeutiques en dermatologie, cancérologie, ainsi que dans le domaine des maladies auto-immunes et celui des transplantations d'organes ou de tissus.

Un apport thérapeutique efficace s'est longtemps heurté à la toxicité de cette vitamine (hypercalcémie parfois mortelle). Actuellement, des analogues structuraux de la vitamine D sont synthétisés, dont certains ne conservent que les propriétés différenciatrices et n'ont pas d'action sur le métabolisme calcique.

EP 0 879 814 décrit des composés triaromatiques qui ont une activité marquée dans les domaines de la différenciation et de la prolifération cellulaire.

C'est un des buts de la présente invention de proposer de nouveaux composés analogues structuraux de la vitamine D qui montrent une activité sélective sur la prolifération et sur la différenciation cellulaire sans présenter de caractère hypercalcimiant.

Un autre but de la présente invention est de proposer de nouveaux composés analogues de la vitamine D qui soient plus facilement synthétisables et donc plus économiques par rapport à ce qui était connu antérieurement.

Ainsi, la présente invention concerne des composés qui peuvent être représentés par la formule générale (I) suivante : 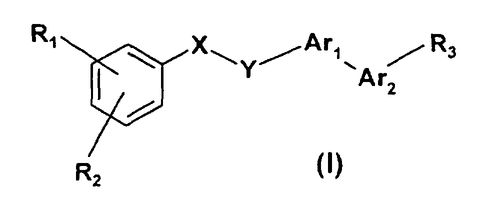 dans laquelle:
- R₁ représente un atome d' hydrogène, un radical CH₃ ou un radical -(CH₂)ᵣ-OR₄,
- R₂ représente un radical -(CH₂)ₛ-OR₅
   r, s, R₄ et R₅ ayant les significations données ci-après,
- X-Y représente une liaison choisie parmi les liaisons de formules (a) à (d) suivantes pouvant être lues de gauche à droite ou inversement: 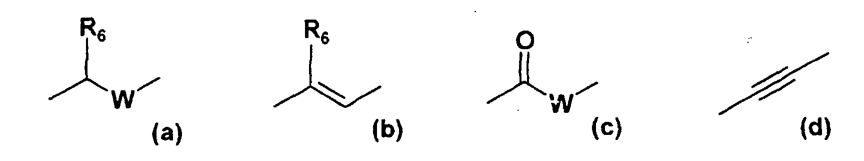 R₆ et W ayant les significations données ci-après,
- Ar₁ représente un cycle de formules (e) à (i) suivantes: 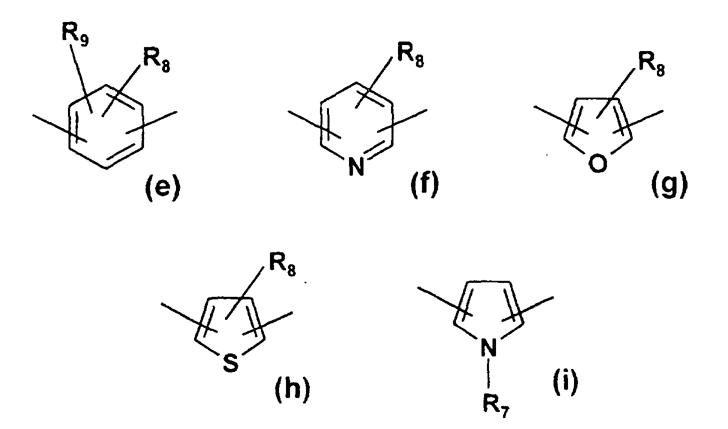 R₇, R₈ et R₉ ayant les significations données ci-après,
- Ar₂ représente un cycle de formules (j) à (n) suivantes: 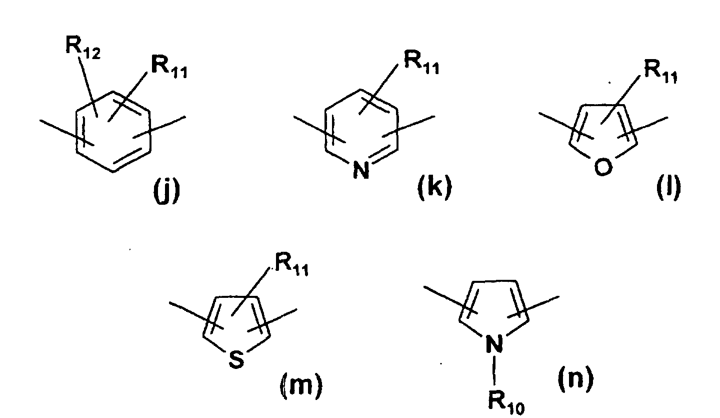 R₁₀, R₁₁ et R₁₂ ayant les significations données ci-après,
- R₃ représente un radical de formule: 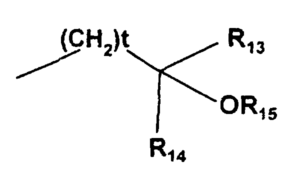 t, R₁₃, R₁₄ et R₁₅ ayant les significations données ci-après,
- r et s identiques ou différents étant 1 ou 2,
- R₄ et R₅ identiques ou différents représentent un atome d'hydrogène, un radical acétyle, un radical benzoyle, un radical triméthylsilyle, un radical tertiobutyldiméthylsilyle ou un radical tétrahydropyranyle,
- R₆ représente un atome d'hydrogène ou un radical alkyle inférieur,
- W représente un atome d'oxygène, de soufre, un radical CH₂ ou un radical NH pouvant être substitué par un radical alkyle inférieur,
- R₇ et R₁₀ identiques ou différents représentent un atome d'hydrogène ou un radical alkyle inférieur,
- R₈, R₉, R₁₁ et R₁₂ identiques ou différents représentent un atome d'hydrogène, un radical alkyle inférieur, un atome d'halogène, un radical -OR₁₆, un radical polyéther, un radical CF₃, un radical NO₂ ou un radical amino pouvant être substitué par un ou deux radicaux alkyles inférieurs,
   R₁₆ ayant les significations données ci-après,
- t étant 0 ou 1
- R₁₃ et R₁₄ identiques ou différents représentent un atome d'hydrogène, un radical alkyle inférieur, un radical cycloalkyle, un radical CF₃ ou un radical C₂F₅,
- R₁₅ représente un atome d'hydrogène, un radical acétyle, un radical triméthylsilyle, un radical tertiobutyldiméthylsilyle ou un radical tétrahydropyranyle,
- R₁₆ représente un atome d'hydrogène ou un radical alkyle inférieur,

L'invention vise également les isomères optiques et géométriques desdits composés de formule (I) ainsi que leurs sels dans le cas où X-Y représentent une liaison de formule (a) et W représente un radical -NH- éventuellement substitué par un radical alkyle inférieur.

Lorsque les composés selon l'invention se présentent sous forme de sels, il s'agit de sels pharmaceutiquement ou cosmétiquement acceptables obtenus par addition d' un acide minéral ou organique, en particulier l'acide chlorhydrique, sulfurique, acétique, fumarique, hémisuccinique, maléique ou mandélique.

Selon la présente invention on entend par radical alkyle inférieur, un radical linéaire ou ramifié ayant de 1 à 6 atomes de carbone, et de préférence, les radicaux méthyle, éthyle, isopropyle, tertiobutyle et hexyle.

Par radical cycloalkyle, on entend un radical alcane cyclique contenant de 3 à 6 atomes de carbone. De préférence le radical cycloalkyle est choisi parmi un radical cyclopropyle cyclopentyle ou cyclohexyle.

Par atome d'halogène, on entend de préférence un atome de fluor, de chlore ou de brome.

Par radical polyéther, on entend un radical ayant de 2 à 5 atomes de carbone interrompu par au moins deux atomes d'oxygène tels que les radicaux méthoxyméthoxy, méthoxyéthoxy et méthoxyéthoxyméthoxy.

Parmi les composés de formule **(I)** rentrant dans le cadre de la présente invention, on peut notamment citer les suivants:
{5-[4'-(1-Ethyl-1-hydroxy-propyl)-biphenyl-3-yloxymethyl]-2-hydroxymethyl-phenyl}-methanol
{2-Hydroxymethyl-5-[4'-(1-hydroxy-1-methyl-ethyl)-biphenyl-3-yloxymethyl]-phenyl}-methanol
{5-[4'-(1-Ethyl-1-hydroxy-propyl)-2'-methyl-biphenyl-3-yloxymethyl]-2-hydroxymethylphenyl}-methanol
{2-Hydroxymethyl-5-[4'-(1-hydroxy-1-methyl-ethyl)-2'-methyl-biphenyl-3-yloxymethyl]-phenyl}-methanol
(5- {2-[3'-(1-Ethyl-1-hydroxy-propyl)-biphenyl-3-yl]-ethyl}-2-hydroxymethyl-phenyl)-methanol
{2-Hydroxymethyl-5-[3'-(1-hydroxy-1-methyl-ethyl)-biphenyl-3-yloxymethyl]-phenyl}-methanol
{{5-[4'-(2-Ethyl-2-hydroxy-butyl)-biphenyl-3-yloxymethyl]-2-hydroxymethyl-phenyl}-methanol
{5-[3'-(2-Ethyl-2-hydroxy-butyl)-biphenyl-3-yloxymethyl]-2-hydroxymethyl-phenyl}-methanol
1-[3'-(3,4-Bis-hydroxymethyl-benzyloxy)-biphenyl-3-yl]-2-methyl-propan-2-ol
{4-[4'-(1-Ethyl-1-hydroxy-propyl)-2'-methyl-biphenyl-3-ylsulfanylmethyl]-2-hydroxymethyl-phenyl}-methanol
{4-[4'-(1-Ethyl-1-hydroxy-propyl)-2,2'-dimethyl-biphenyl-3-yloxymethyl]-2-hydroxymethyl-phenyl}-methanol
{2-Hydroxymethyl-4-[4'-(1-hydroxy-1-propyl-butyl)-2,2'-dimethyl-biphenyl-3-yloxymethyl]-phenyl}-methanol
{4-[4'-(1-Ethyl-1-hydroxy-propyl)-2-methyl-biphenyl-3-yloxymethyl]-2-hydroxymethyl-phenyl}-methanol
{4-[4'-(1-Ethyl-1-hydroxy-propyl)-2,2',6'-trimethyl-biphenyl-3-yloxymethyl]-2-hydroxymethyl-phenyl}-methanol
(4- {3-[5-(1-Ethyl-1-hydroxy-propyl)-pyridin-2-yl]-phenoxymethyl}-2-hydroxymethyl-phenyl)-methanol
{4-[4'-(1-Ethyl-1-hydroxy-propyl)-6,2'-dimethyl-biphenyl-3-yloxymethyl]-2-hydroxymethyl-phenyl}-methanol
{4-[4'-(1-Ethyl-1-hydroxy-propyl)-6,2',6'-trimethyl-biphenyl-3-yloxymethyl]-2-hydroxymethyl-phenyl}-methanol
{4-[4'-(1-Ethyl-1-hydroxy-propyl)-6-methyl-biphenyl-3-yloxymethyl]-2-hydroxymethyl-phenyl}-methanol
{4-[4'-(1-Ethyl-1-hydroxy-propyl)-2',6'-dimethyl-biphenyl-3-yloxymethyl]-2-hydroxymethyl-phenyl}-methanol
1-{4-[3-(3,4-Bis-hydroxymethyl-benzyloxy)-phenyl]-thiophen-2-yl}-propan-1-ol
(4-{3-[4-(1-Ethyl-1-hydroxy-propyl)-thiophen-2-yl]-phenoxymethyl}-2-hydroxymethyl-phenyl)-methanol
{4-[4'-(1-Ethyl-1-hydroxy-propyl)-2'-methyl-biphenyl-3-ylmethoxy]-2-hydroxymethyl-phenyl}-methanol
1-[3'-(3,4-Bis-hydroxymethyl-phenoxymethyl)-2-methyl-biphenyl-4-yl]-propan-1-ol
{4-[4'-(1-Ethyl-1-hydroxy-propyl)-3'-methyl-biphenyl-3-yloxymethyl]-2-hydroxymethyl-phenyl}-methanol
{5-[4'-(1-Ethyl-1-hydroxy-propyl)-2'-methyl-biphenyl-4-yloxymethyl]-2-hydroxymethyl-phenyl}-methanol
{4-[2'-tert-Butyl-4'-(1-ethyl-1-hydroxy-propyl)-biphenyl-3-yloxymethyl]-2-hydroxymethyl-phenyl}-methanol
1-[3'-(3,4-Bis-hydroxymethyl-benzyloxy)-2-methyl-biphenyl-4-yl]-2,2-dimethyl-propan-1-ol
1-[3'-(3,4-Bis-hydroxymethyl-benzyloxy)-2-methyl-biphenyl-4-yl]-2-methyl-propan-1-ol
{2-Hydroxymethyl-4-[methyl-(trifluoro-hydroxy-trifluoromethyl-ethyl)-biphenyl-3-yloxymethyl]-phenyl}-methanol
[5-(2-{5-[4-(1-Ethyl-1-hydroxy-propyl)-2-methyl-phenyl]-thiophen-2-yl}-ethyl)-2-hydroxymethyl-phenyl]-methanol
(5-{5-[4-(1-Ethyl-1-hydroxy-propyl)-2-methyl-phenyl]-thiophen-2-ylmethoxy}-2-hydroxymethyl-phenyl)-methanol
[5-(2-{5-[2-Ethyl-4-(1-ethyl-1-hydroxy-propyl)-phenyl]-thiophen-2-yl}-ethyl)-2-hydroxymethyl-phenyl]-methanol
(5-{5-[2-Ethyl-4-(1-ethyl-1-hydroxy-propyl)-phenyl]-thiophen-2-ylmethoxy}-2-hydroxymethyl-phenyl)-methanol
[5-(2-{5-[4-(1-Ethyl-1-hydroxy-propyl)-2-methyl-phenyl]-4-methyl-thiophen-2-yl}-ethyl)-2-hydroxymethyl-phenyl]-methanol
(5-{5-[4-(1-Ethyl-1-hydroxy-propyl)-2-methyl-phenyl]-4-methyl-thiophen-2-ylmethoxy}-2-hydroxymethyl-phenyl)-methanol
[2-Hydroxymethyl-5-(2-{5-[methyl-(trifluoro-hydroxy-trifluoromethyl-ethyl)-phenyl]-thiophen-2-yl}-ethyl)-phenyl]-methanol
(2-Hydroxymethyl-5-{5-[methyl-(trifluoro-hydroxy-trifluoromethyl-ethyl)-phenyl]-thiophen-2-ylmethoxy}-phenyl)-methanol
[5-(2-{5-[Ethyl-(trifluoro-hydroxy-trifluoromethyl-ethyl)-phenyl]-thiophen-2-yl}-ethyl)-2-hydroxymethyl-phenyl]-methanol
(5-{5-[Ethyl-(trifluoro-hydroxy-trifluoromethyl-ethyl)-phenyl]-thiophen-2-ylmethoxy}-2-hydroxymethyl-phenyl)-methanol
[2-Hydroxymethyl-5-(2-{methyl-[methyl-(trifluoro-hydroxy-trifluoromethyl-ethyl)-phenyl]-thiophen-2-yl}-ethyl)-phenyl]-methanol
(2-Hydroxymethyl-5-{methyl-[methyl-(trifluoro-hydroxy-trifluoromethyl-ethyl)-phenyl]-thiophen-2-ylmethoxy}-phenyl)-methanol
[5-(2-{5-[4-(1-Ethyl-1-hydroxy-propyl)-2-methyl-phenyl]-thiophen-3-yl}-ethyl)-2-hydroxymethyl-phenyl]-methanol
(5-{5-[4-(1-Ethyl-1-hydroxy-propyl)-2-methyl-phenyl]-thiophen-3-ylmethoxy}-2-hydroxymethyl-phenyl)-methanol
[5-(2-{5-[2-Ethyl-4-(1-ethyl-1-hydroxy-propyl)-phenyl]-thiophen-3-yl}-ethyl)-2-hydroxymethyl-phenyl]-methanol
(5-{5-[2-Ethyl-4-(1-ethyl-1-hydroxy-propyl)-phenyl]-thiophen-3-ylmethoxy}-2-hydroxymethyl-phenyl)-methanol
[2-Hydroxymethyl-5-(2-{5-[methyl-(trifluoro-hydroxy-trifluoromethyl-ethyl)-phenyl]-thiophen-3-yl}-ethyl)-phenyl]-methanol
(2-Hydroxymethyl-5-{5-[methyl-(trifluoro-hydroxy-trifluoromethyl-ethyl)-phenyl]-thiophen-3-ylmethoxy}-phenyl)-methanol
[5-(2-{5-[Ethyl-(trifluoro-hydroxy-trifluoromethyl-ethyl)-phenyl]-thiophen-3-yl}-ethyl)-2-hydroxymethyl-phenyl]-methanol
(5-{5-[Ethyl-(trifluoro-hydroxy-trifluoromethyl-ethyl)-phenyl]-thiophen-3-ylmethoxy}-2-hydroxymethyl-phenyl)-methanol
[5-(2-{4-[4-(1-Ethyl-1-hydroxy-propyl)-2-methyl-phenyl]-thiophen-2-yl}-ethyl)-2-hydroxymethyl-phenyl]-methanol
(5-{4-[4-(1-Ethyl-1-hydroxy-propyl)-2-methyl-phenyl]-thiophen-2-ylmethoxy}-2-hydroxymethyl-phenyl)-methanol
[5-(2-{4-[2-Ethyl-4-(-ethyl-1-hydroxy-propyl)-phenyl]-thiophen-2-yl}-ethyl)-2-hydroxymethyl-phenyl]-methanol
(5-{4-(2-Ethyl-4-(1-ethyl-1-hydroxy-propyl)-phenyl]-thiophen-2-ylmethoxy}-2-hydroxymethyl-phenyl)-methanol
[5-(2-{4-[4-(1-Ethyl-1-hydroxy-propyl)-2-methyl-phenyl]-5-methyl-thiophen-2-yl}-ethyl)-2-hydroxymethyl-phenyl]-methanol
(5-{4-[4-(1-Ethyl-1-hydroxy-propyl)-2-methyl-phenyl]-5-methyl-thiophen-2-ylmethoxy}-2-hydroxymethyl-phenyl)-methanol
[2-Hydroxymethyl-5-(2-{4-[methyl-(trifluoro-hydroxy-trifluoromethyl-ethyl)-phenyl]-thiophen-2-yl}-ethyl)-phenyl]-methanol
(2-Hydroxymethyl-5-{4-[methyl-(trifluoro-hydroxy-trifluoromethyl-ethyl)-phenyl]-thiophen-2-ylmethoxy}-phenyl)-methanol
[5-(2-{4-[Ethyl-(trifluoro-hydroxy-trifluoromethyl-ethyl)-phenyl]-thiophen-2-yl}-ethyl)-2-hydroxymethyl-phenyl]-methanol
(5-{4-[Ethyl-(trifluoro-hydroxy-trifluoromethyl-ethyl)-phenyl]-thiophen-2-ylmethoxy}-2-hydroxymethyl-phenyl)-methanol
[2-Hydroxymethyl-5-(2-{methyl-[methyl-(trifluoro-hydroxy-trifluoromethyl-ethyl)-phenyl]-thiophen-2-yl}-ethyl)-phenyl]-methanol
(2-Hydroxymethyl-5- {methyl-[methyl-(trifluoro-hydroxy-trifluoromethyl-ethyl)-phenyl]-thiophen-2-ylmethoxy}-phenyl)-methanol
{5-[2'-Ethyl-4'-(1-ethyl-1-hydroxy-propyl)-6-methyl-biphenyl-3-yloxymethyl]-2-hydroxymethyl-phenyl}-methanol
{5-[4'-(1-Ethyl-1-hydroxy-propyl)-2'-isopropyl-6-methyl-biphenyl-3-yloxymethyl]-2-hydroxymethyl-phenyl}-methanol
{5-[2'-*tert*-Butyl-4'-(1-ethyl-1-hydroxy-propyl)-6-methyl-biphenyl-3-yloxymethyl]-2-hydroxymethyl-phenyl}-methanol
{5-[Ethyl-methyl-(trifluoro-hydroxy-trifluoromethyl-ethyl)-biphenyl-3-yloxymethyl]-2-hydroxymethyl-phenyl}-methanol
{2-Hydroxymethyl-5-[2'-isopropyl-6-methyl-4'-(2,2,2-trifluoro-1-hydroxy-1-trifluoromethylethyl)-biphenyl-3-yloxymethyl]-phenyl}-methanol
{5-[Dimethylethyl-methyl-(trifluoro-hydroxy-trifluoromethyl-ethyl)-biphenyl-3-yloxymethyl]-2-hydroxymethyl-phenyl}-methanol
{5-[6-Ethyl-4'-(1-ethyl-1-hydroxy-propyl)-2'-methyl-biphenyl-3-yloxymethyl]-2-hydroxymethyl-phenyl}-methanol
{5-[4'-(1-Ethyl-1-hydroxy-propyl)-6-methoxy-2'-methyl-biphenyl-3-yloxymethyl]-2-hydroxymethyl-phenyl}-methanol
{5-[6-*tert*-Butyl-4'-(1-ethyl-1-hydroxy-propyl)-2'-methyl-biphenyl-3-yloxymethyl]-2-hydroxymethyl-phenyl}-methanol
{5-[Ethyl-methyl-(trifluoro-hydroxy-trifluoromethyl-ethyl)-biphenyl-3-yloxymethyl]-2-hydroxymethyl-phenyl}-methanol
{2-Hydroxymethyl-5-[6-methoxy-2'-methyl-4'-(2,2,2-trifluoro-1-hydroxy-1-trifluoromethyl-ethyl)-biphenyl-3-yloxymethyl]-phenyl}-methanol
{5-[Dimethylethyl-methyl-(trifluoro-hydroxy-trifluoromethyl-ethyl)-biphenyl-3-yloxymethyl]-2-hydroxymethyl-phenyl}-methanol
(5-{2-[4'-(1-Ethyl-1-hydroxy-propyl)-6,2'-dimethyl-biphenyl-3-yl]-ethyl}-2-hydroxymethyl-phenyl)-methanol
(5-{2-[Dimethyl-(trifluoro-hydroxy-trifluoromethyl-ethyl)-biphenyl-3-yl]-ethyl}-2-hydroxymethyl-phenyl)-methanol
(5-{[4'-(1-Ethyl-1-hydroxy-propyl)-6,2'-dimethyl-biphenyl-3-ylamino]-methyl}-2-hydroxymethyl-phenyl)-methanol
(5-{[Dimethyl-(trifluoro-hydrory-trifluoromethyl-ethyl)-biphenyl-3-ylamino]-methyl}-2-hydroxymethyl-phenyl)-methanol
[5-({[4'-(1-Ethyl-1-hydroxy-propyl)-6,2'-dimethyl-biphenyl-3-yl]-methyl-amino}-methyl)-2-hydroxymethyl-phenyl]-methanol
[5-({[Dimethyl-(trifluoro-hydroxy-trifluoromethyl-ethyl)-biphenyl-3-yl]-methyl-amino}-methyl)-2-hydroxymethyl-phenyl]-methanol

Selon la présente invention, les composés de formule (I) plus particulièrement préférés sont ceux pour lesquels l'une au moins, et de préférence toutes les conditions ci-dessous sont respectées :
- R₁ représente le radical CH₃ ou CH₂OH,
- R₂ représente le radical CH₂OH,
- X-Y représente une liaison de formule (a) ou (c),
- R₃ représente le radical C(R₁₃)(R₁₄)OH.

La présente invention a également pour objet les procédés de préparation des composés de formule (I).

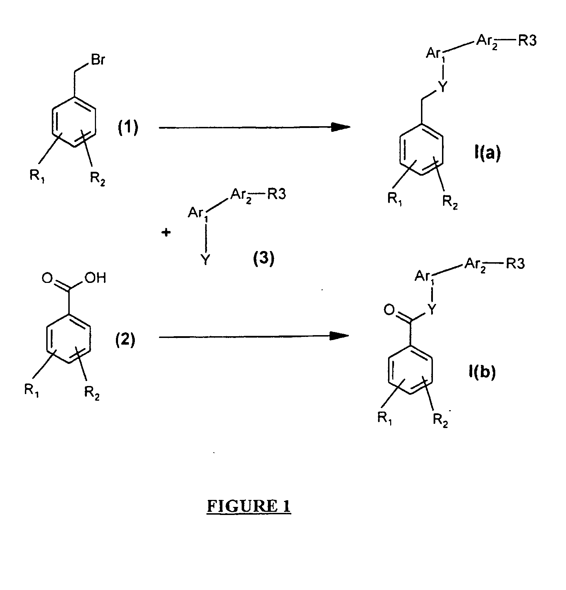

Les figures 1 à 5 représentent des schémas réactionnels qui peuvent être mis en oeuvre pour la préparation des composés selon l'invention.

Ainsi, les composés de formule **I(a)** peuvent être obtenus **(Figure 1)** par réaction d'un composé halogéné de préférence bromé **(1)** avec un dérivé **(3)** phénolique (Y=OH), thiophénolique (Y=SH), d'aniline (Y=NH-COO-tertiobutyl) en présence d'une base telle K₂CO₃ dans un solvant tel l'acétone ou la méthyléthylcétone.

Les composés de composés de formule **I(a)** peuvent être également obtenus **(Figure 1)** par réaction d'un composé halogéné de préférence bromé **(1)** avec le sel de sodium ou de potassium d'un dérivé **(3)** phénolique (Y=OH), thiophénolique (Y=SH), d'aniline (Y=NH-COO-tertiobutyl) dans un solvant tel le diméthylformamide (DMF).

Les composés de formule **I(b)** peuvent être obtenus **(Figure 1)** par réaction d'un dérivé benzoïque **(2)** avec un dérivé **(3)** phénolique (Y=OH), thiophénolique (Y=SH), d'aniline (Y=NH₂) en présence de carbonydiimidazole ou de dicyclohexylcarbodiimide dans un solvant tel le dichlorométhane ou le tétrahydrofuranne (THF).

Les composés de formule **I(b)** peuvent être également obtenus **(Figure 1)** par réaction d'un chlorure de benzoyle (obtenu par réaction d'un dérivé benzoïque **(2)** avec du chlorure de thionyle ou du chlorure d'oxalyle) avec un dérivé **(3)** phénolique (Y=OH), thiophénolique (Y=SH), d'aniline (Y=NH₂) en présence d'une base telle la triéthylamine dans un solvant tel le dichlorométhane ou le tétrahydrofuranne (THF).

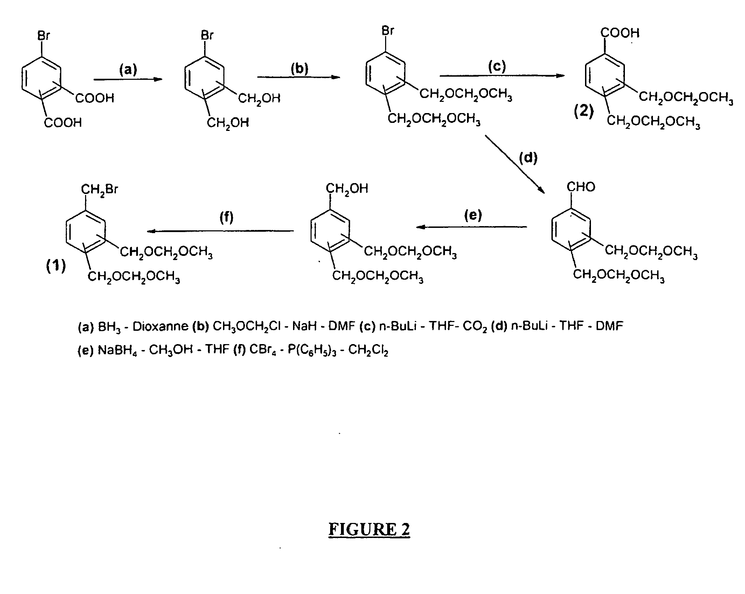

Les composés **(1)**, **(2)**, et **(3)** peuvent être obtenus selon les schémas réactionnels présentés sur **les figures 2, 3 et 4.** Les méthodes de préparations des composés (**1**) et (**2**) présentent l'avantage de limiter le nombre d'étapes de préparation.

Dans la **figure 2,** (a) représente une réaction avec BH₃ dans du Dioxanne, (b) représente une réaction avec CH₃OCH₂Cl en présence d'hydrure de sodium dans un solvant diméthylformamide, (c) représente une réaction avec le n-butyllithium en présence de CO₂ dans du tétrahydrofuranne, (d) représente une réaction une réaction avec le n-butyllithium dans du tétrahydrofuranne suivie d'une réaction avec le diméthylformamide, (e) représente une réaction de réduction avec du borohydrure de sodium dans un solvant Méthanol-tétrahydrofuranne, (f) représente un réaction avec du tétrabromure de carbone en présence de triphénylphosphine dans un solvant dichlorométhane.

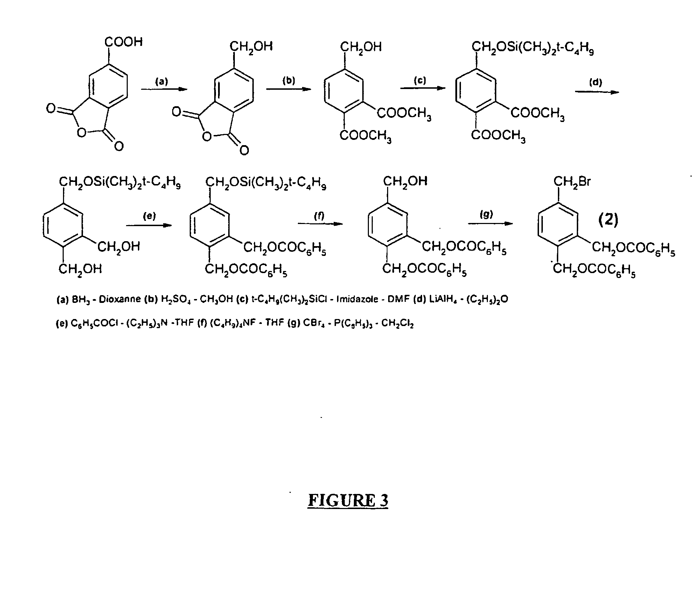

Dans la **figure 3,** (a) représente une réaction avec BH₃ dans du Dioxanne, (b) représente une réaction avec du méthanol en présence d'acide sulfurique, (c) représente une réaction avec t-C₄H₉(CH₃)₂SiCl en présence d'imidazole dans un solvant diméthylformamide, (d) représente une réaction avec LiAlH₄ dans de l'éther, (e) représente une réaction avec du chlorure de benzoyle en présence de triéthylamine dans du tétrahydrofuranne, (f) représente une réaction avec (C₄H₉)₄NF dans du tétrahydrofuranne, (g) représente un réaction avec du tétrabromure de carbone en présence de triphénylphosphine dans un solvant dichlorométhane.

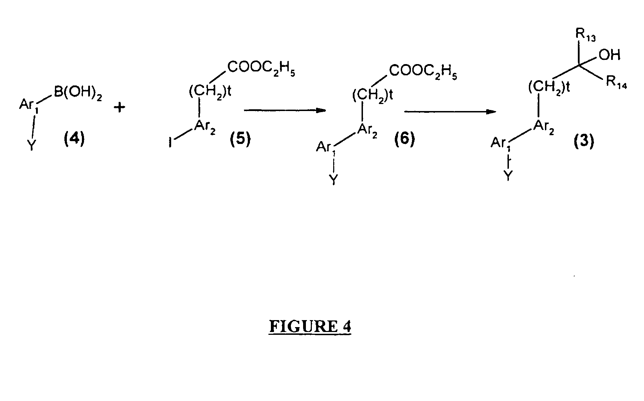

Les dérivés **(3)** peuvent être obtenus selon le schéma réactionnel indiqué sur la **figure 4.** Par une réaction de type Suzuki entre un dérivé boronique **(4)** et un dérivé iodoaryle **(5)** en présence d'un catalyseur tel le tétrakis(triphénylphosphine)palladium, on obtient le dérivé ester **(6)** qui est transformé en dérivé **(3)** par réaction avec un halogénure d'alkylmagnésium, de cycloalkylmagnésium ou un alkyllithium.

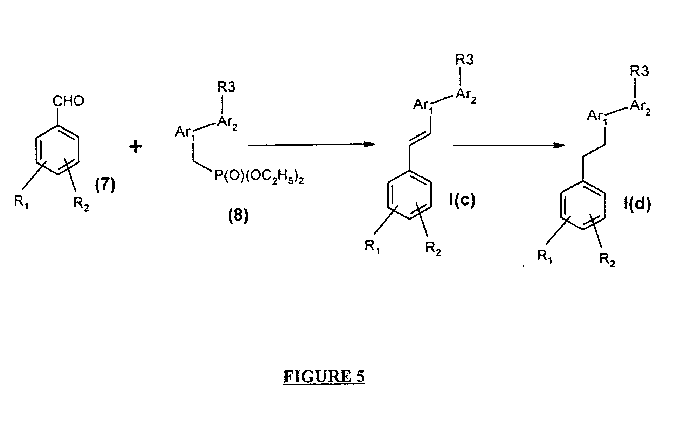

Les composés de formule **I(c)** peuvent être obtenus **(Figure 5)** par une réaction de type Horner-Emmons entre le dérivé phosphonate (8) (obtenu à partir du bromure de benzyle correspondant par une réaction de type Arbuzov) et le benzaldéhyde (7).

Les composés de formule I(d) peuvent être obtenus à partir des composés I(c) par hydrogénation de la double liaison en présence de palladium sur charbon.

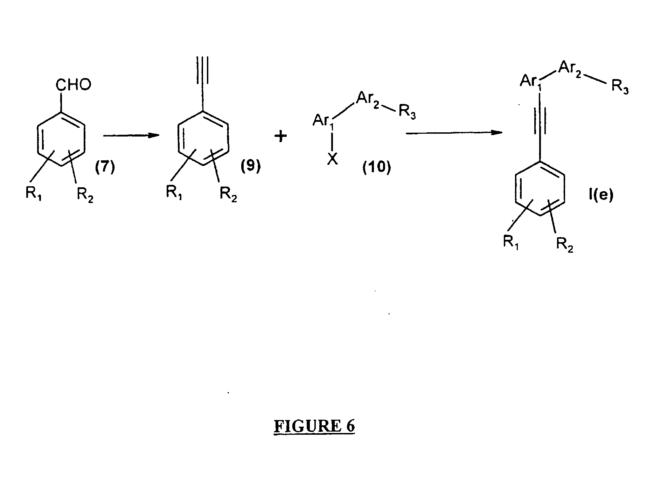

Les composés de formule **I(e)** peuvent être aussi obtenus **(Figure 6)** par une réaction de type Sonogashira entre un dérivé acétylénique **(9)** (obtenu par réaction du benzaldéhyde **(7)** par une réaction de type Corey-Fuchs) et un dérivé triflate (X=OSO₂CF₃) ou iodé (X=I) **(10)** en présence d'un catalyseur de métaux de transition tel Pd(Cl)₂(PPh₃)₂ et de CuI dans un solvant tel la triéthylamine.

Les composés de formule générale (I) présentent des propriétés biologiques analogues à celles de la vitamine D, notamment les propriétés de transactivation des éléments de réponse à la vitamine D (VDRE), telles qu'une activité agoniste ou antagoniste vis-à-vis de récepteurs de la vitamine D ou de ses dérivés. Par vitamines D ou leurs dérivés on entend par exemple les dérivés de la vitamine D₂ ou D₃ et en particulier la 1,25-dihydroxyvitamine D₃ (calcitriol).

Cette activité agoniste vis-à-vis de récepteurs de la vitamine D ou de ses dérivés peut être mise en évidence "in vitro" par dés méthodes reconnues dans le domaine de l'étude de la transcription génique (Hansen et al., The Society For Investigative Dermatologie, vol.1, N°1, April 1996).

A titre d'exemple, l'activité agoniste VDR peut être testée sur la lignée cellulaire HeLa, par cotransfection d'un vecteur d'expression du récepteur VDR humain et du plasmide rapporteur p240Hase-CAT qui contient la région - 1399 à +76 du promoteur de la 24-hydroxylase de rat, clonée en amont de la phase codante du gène de la chloramphénicol-acétyl-transférase (CAT). 18 heures après cotransfection, le produit test est ajouté dans le milieu. Après 18 heures de traitement, le dosage de l'activité CAT des lysats cellulaires est effectuée par un test Elisa. Les résultats sont exprimés en pourcentage de l'effet normalement observé avec 10⁻⁷ M de calcitriol.

L'activité agoniste peut aussi être caractérisée dans ce système de cotransfection, par la détermination de la dose nécessaire pour atteindre 50 % de l'activité maximale du produit (AC50).

Les propriétés biologiques analogues à la vitamine D peuvent être également mesurées par la capacité du produit à inhiber la prolifération des kératinocytes humaines normaux (KHN en culture). Le produit est ajouté à des KHN cultivés dans des conditions favorisant l'état prolifératif. Le produit est laissé au contact des cellules pendant cinq jours. Le nombre de cellules prolifératives est mesuré par incorporation de Bromodeoxyuridine (BRdU) dans l'ADN.

L'activité agoniste aux récepteurs de la vitamine D des composés de l'invention peut être également évaluée "in vivo" par induction de la 24-Hydroxylase chez la souris SKH. (Voorhees and al. 1997.108 : 513-518).

La présente invention a également pour objet à titre de médicament les composés de formule (I) tels que décrits ci-dessus.

Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore l'atopie cutanée, telle que l'eczéma ou l'atopie respiratoire ou encore l'hypertrophie gingivale,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides, le lymphome T, et les proliférations pouvant être induites par les ultra-violets notamment dans le cas des épithélioma baso et spinocellulaires, ainsi que toute lésion précancéreuse cutanées telles que les kératoacanthomes,
5) pour traiter d'autres désordres dermatologiques tels que les dermatoses immunes telles le lupus érythémateux, les maladies immunes bulleuses et les maladies du collagène, telle la sclérodermie,
6) dans le traitement d'affections dermatologiques ou générales à composante immunologique,
7) pour lutter contre les troubles de la fonction sébacée tels que l'hyperséborrhée de l'acné ou la séborrhée simple,
8) dans le traitement de désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique,
9) pour prévenir ou traiter les troubles de la cicatrisation ou pour prévenir ou pour réparer les vergetures,
10) dans le traitement d'affections inflammatoires telles que l'arthrite,
11) dans le traitement de toute affection d'origine virale au niveau cutané ou général, telle que le syndrome de Kaposis,
12) pour traiter certains troubles ophtalmologiques, notamment les coméopathies,
13) dans le traitement ou la prévention des états cancéreux ou précancéreux de cancers présentant ou pouvant être induits par des récepteurs de vitamine D, tels que, mais sans limitation, le cancer du sein, la leucémie, les syndromes myélodysplasiques et les lymphomes, les carcinomes des cellules de l'épithélium malpighien et les cancers gastro-intestinaux, les mélanomes, et l'ostéosarcome,
14) dans la prévention ou le traitement de l'alopécie de différentes origines, notamment l'alopécie due à la chimiothérapie ou aux rayonnements,
15) dans le traitement d'affections immunitaires, telles que les maladies auto-immunes comme le diabète sucré de type 1, la sclérose en plaques, le lupus et les affections de type lupus, l'asthme, ou la glomérulonéphrite, des dysfonctionnements sélectifs du système immunitaire tel que le SIDA, ou la prévention du rejet immun comme les rejets de greffons de rein, coeur, moelle osseuse, foie, des îlots pancréatiques, du pancréas, ou de la peau, ou la prévention de la maladie du greffon contre l'hôte,
16) dans le traitement d'affections endocriniennes, étant donné que les analogues de la vitamine D peuvent moduler la sécrétion hormonale telle que l'augmentation de la sécrétion d'insuline ou la suppression sélective de la sécrétion de l'hormone parathyroïdienne, par exemple dans l'insuffisance rénale chronique et l'hyperparathyroïdie secondaire,
17) dans le traitement d'affections caractérisées par une gestion anormale du calcium intracellulaire, et dans le traitement ou la prévention des pathologies dans lesquelles le métabolisme calcique est impliqué, telle que l'ischémie musculaire (infarctus du myocarde),
18) dans le traitement et ou la prévention des carences en vitamine D et d'autres affections de l'homéostasie des minéraux dans le plasma et les os, tel que le rachitisme, l'ostéomalacie, l'ostéoporose, notamment dans le cas des femmes monopausées, l'ostéodystrophie rénale, les troubles de la fonction parathyroïdienne,
19) dans le traitement d'affections du système cardiovasculaire telles que l'artériosclérose ou l'hypertension ainsi que le diabète non-insulino dépendant,

Dans les domaines thérapeutiques mentionnés ci-dessus, les composés selon l'invention peuvent être avantageusement employés en combinaison avec des rétinoïdes, avec des corticostéroïdes ou des oestrogènes, en association avec des anti-oxydants, avec des α-hydroxy ou α-céto acides ou leurs dérivés, avec des bloqueurs de canaux potassiques, ou encore en association avec d'autres médicaments connus pour interférer avec le système immunitaire (par exemple la cyclosporine, le FK 506, les glucocorticoïdes, les anticorps monoclonaux, les cytokines ou les facteurs de croissance ...).

Par rétinoides on entend des ligands des récepteurs RAR ou RXR, soit naturels ou soit synthétiques.

Par antiradicaux libres on entend par exemple l'α-tocophérol, la Super Oxyde Dismutase, l'Ubiquinol ou certains chélatants de métaux.

Par α-hydroxy ou α-céto acides ou leurs dérivés, on entend par exemple les acides lactique, malique, citrique, glycolique, mandélique, tartrique, glycérique, ascorbique, les dérivés d'acide salicylique, ainsi que leurs sels, amides ou esters.

Par bloqueurs de canaux potassiques, on entend par exemple le Minoxidil (2,4-diamino-6-pipéridino-pyrimidine-3-oxyde) et ses dérivés.

La présente invention a également pour objet une composition pharmaceutique comprenant au moins un composé de formule (I) tel que défini ci-dessus.

La présente invention a donc aussi pour objet une telle composition pharmaceutique destinée notamment au traitement des affections susmentionées.

L'administration des composés selon l'invention peut être effectuée par voie entérale, parentérale, topique ou oculaire.

Par voie entérale, les compositions pharmaceutiques peuvent se présenter sous forme de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, les compositions peuvent se présenter sous forme de solutions ou suspensions pour perfusion ou pour injection. Les composés selon l'invention sont généralement administrés à une dose journalière d'environ 0,001 µg/kg à 1000 µg/kg et de préférence d'environ 0,01 µg/kg à 100 µg/kg en poids corporel en 1 à 3 prises.

Par voie topique, les compositions pharmaceutiques à base de composés selon l'invention sont destinées au traitement de la peau et des muqueuses et se présentent sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent se présenter soit sous forme anhydre, soit sous forme aqueuse selon l'indication clinique.

Par voie oculaire, ce sont principalement des collyres.

Ces compositions pour la voie topique ou oculaire contiennent au moins un composé de formule (I) tel que défini ci-dessus à une concentration de préférence comprise entre 0,0001 et 5 % et de préférence entre 0,001 à 1% par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches, pour prévenir et/ou lutter contre le vieillissement photo-induit ou chronologique.

Dans le domaine cosmétique, les composés selon l'invention peuvent être avantageusement employés en combinaison avec les rétinoïdes, avec des corticostéroïdes, en association avec des anti-radicaux libres, avec des α-hydroxy ou α-céto acides ou leurs dérivés, ou encore avec des bloqueurs de canaux ioniques, les différents produits pris en association avec les composés de la présente invention étant tels que définis ci-dessus.

La présente invention vise donc également une composition cosmétique contenant, dans un support cosmétiquement acceptable, au moins un composé de formule 1 tel que défini ci-dessus. Cette composition cosmétique peut se présenter notamment sous forme d'une crème, d'un lait, d'une lotion, d'un gel, de microsphères ou nanosphères ou vésicules lipidiques ou polymériques, d'un savon ou d'un shampooing.

La concentration en composé de formule I dans les compositions cosmétiques peut être comprise entre 0,001 et 3 % en poids par rapport au poids total de la composition.

Les compositions pharmaceutiques et cosmétiques selon l'invention peuvent, en outre, contenir des additifs inertes ou même pharmacodynamiquement ou cosmétiquement actifs ou des combinaisons de ces additifs et notamment : des agents mouillants; des agents dépigmentants tels que l'hydroquinone, l'acide azelaïque, l'acide caféique ou l'acide kojique; des émollients; des agents hydratants comme le glycérol, le PEG 400, la thiamorpholinone et ses dérivés ou l'urée; des agents antiséborrhéiques ou antiacnéiques, tels que la S-carboxyméthylcystéine, la S-benzyl-cystéamine, leurs sels et leurs dérivés, ou le péroxyde de benzoyle; des antibiotiques comme l'érythromycine et ses esters, la néomycine, la clindamycine et ses esters, les tétracyclines; des agents antifongiques tels que le kétoconazole ou les polyméthylène-4,5 isothiazolinones-3 ; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4- benzothiadiazine 1,1-dioxyde) et le Phénytoïn (5,4-diphényl-imidazolidine 2,4-dione) ; des agents anti-inflammatoires non stéroïdiens; des caroténoïdes et, notamment le β-carotène; des agents anti-psoriatiques tels que l'anthraline et ses dérivés et enfin, les acides eicosa-5,8,11,14- tétraynoïque et eicosa-5,8,11-trynoïque, leurs esters et les amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des anti-oxydants, tels que l'α-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés ainsi qu'un exemple de test d'évaluation de l'activité biologique de composés de formule (I) selon l'invention.

### EXEMPLE 1

### {5-[4'-(1-Ethyl-1-hydroxy-propyl)-biphenyl-3-yloxymethyl]-2-hydroxymethyl-phenyl}-methanol

### a) 4-Hydroxymethyl-phthalate de diméthyle

1,2,4-benzenetricarboxylic anhydride (50g, 260 mmol) est dissous dans 800 mL de dioxanne anhydre. A température ambiante, BH₃.THF (260 mmol, 1 éq.) est ajouté goutte à goutte par l'intermédiaire d'une ampoule de coulée, sur une période de 1h30 environ. L'agitation est maintenue pendant 12h, puis le milieu réactionnel est versé dans un mélange contenant 600 mL d'une solution saturée de chlorure d'ammonium et 2 L de dichlorométhane. Après décantation, la phase organique est séchée et les solvants sont évaporés sous pression réduite. Le résidu obtenu est ensuite dissous dans 1L de methanol et chauffé à reflux après ajout de 5 mL d'acide sulfurique. Après 18 h de reflux, le milieu réactionnel est refroidi à température ambiante et directement versé dans un mélange eau/éther éthylique (1 L / 2 L). Après décantation, la phase aqueuse est de nouveau extraite par deux fractions d'éther éthylique (700 mL environ) puis les phases organiques sont réunies, séchées et concentrées sous pression réduite. Un mélange triester-diester/alcool est obtenu avec un rendement de 80%, contenant 65 % du produit désiré.

### b) 4-(tert-Butyl-dimethyl-silanyloxymethyl)-phthalate de diméthyle

Le mélange obtenu précédemment, contenant environ 135 mmol de produit désiré, est dissous dans 400 mL de DMF anhydre. Le chlorure de *tert*-butyldiméthylsilane (22,5 g, 150 mmol) est alors ajouté en une seule portion. Puis on ajoute en trois portions (légère exothermie) un total de 13,5 g (195 mmol) d'imidazole. Le milieu réactionnel est agité 36 heures et concentré sous pression réduite. Le résidu est alors dissous dans 500 ml d'éther éthylique, puis filtré pour éliminer le chlorhydrate d'imidazole formé. Le sel est rinçé avec 2 fractions de 150 mL d'éther éthylique, les phases organiques sont réunies, séchées et concentrées sous pression réduite. Le résidu obtenu est alors purifié par chromatographie sur colonne de silice. le premier produit collecté (éluant AcOEt 10 - heptane 90) est le 4-(*tert*-Butyl-dimethyl-silanyloxymethyl)-phthalate de diméthyle désiré. Rendement 87%, rendement global depuis l'acide de départ: 45%.

### c) [5-(tert-Butyl-dimethyl-silanyloxymethyl)-2-hydroxymethyl-phenyl]-methanol

Le diester obtenu précédemment (75 g, 220 mmol) est dissous dans 1 L d'éther éthylique et refroidi à 0°C sous pression positive d'azote. 4 fractions de 5 g de LiAlH₄ (527 mmol) sont ajoutées précautionneusement, puis le mélange est chauffé à 50°C. Après 1h30 d'agitation, le milieu réactionnel est de nouveau refroidi à 0°C, puis traité successivement avec 20 mL d'eau, 20 mL de NaOH 15%, puis 60 mL d'eau. Le milieu réactionnel est agité 30 minutes jusqu'à ce qu'on observe la disparition complète des sels d'aluminium gris et leur précipitation en flocons blancs. Le milieu est alors filtré et après rinçage des sels par trois fractions d'acétate d'éthyle (200 mL), les phases organiques sont rassemblées, séchées et concentrées sous pression réduite. Le produit obtenu représente un rendement de 97%

### d) benzoate de 2-benzoyloxymethyl-4-(tert-butyl-dimethyl-silanyloxymethyl)-benzyle

Le diol brut obtenu prédédemment (60 g, 212 mmol) est dissous dans 600 mL de THF anhydre et refroidi à 0°C. 74 mL (530 mmol) de triéthylamine sont alors ajoutés, suivis de 52 mL (448 mmol) de chlorure de benzoyle. DMAP (500 mg) est ensuite ajouté en une seule portion et le mélange est agité 30 minutes à 0°C puis 12 heures à température ambiante. Le milieu réactionnel est alors filtré pour éliminer les sels de triéthylammonium précipités, les sels sont rincés avec deux fractions de 200 mL d'acétate d'éthyle, puis le mélange des phases organiques est concentré sous pression réduite. Le résidu obtenu est repris dans le dichlorométhane, la phase organique est lavée avec une solution saturée de chlorure d'ammonium puis avec de l'eau. Après séchage sur sulfate de magnésium et concentration sous pression réduite, un résidu jaune foncé est obtenu et sera utilisé tel quel pour l'étape suivante.

### e) benzoate de 2-benzoyloxymethyl-4-hydroxymethyl-benzyle

Le résidu obtenu précédemment est dissous dans 600 mL d'acétate d'éthyle et 220 mL d'une solution de fluorure de tétrabutylammonium (1M dans le THF) sont ajoutés en une seule fraction. Après 30 minutes d'agitation à température ambiante, le milieu réactionnel est versé dans 1 L d'une solution saturé de chlorure d'ammonium . Après séparation, la phase aqueuse est de nouveau extraite avec 500 mL d'acétate d'éthyle, les phases organiques sont rassemblées, séchées et evaporées. Le produit est alors purifié par chromatographie sur colonne de silice (Acétate d'éthyle 30 / Heptane 70). Un solide blanc est obtenu (pf: 91-93°C).

### f) bromure de (3,4-bis-benzoyloxymethy)-benzyle

L'alcool précédent (65 g, 172 mmol) est dissous dans 350 mL de dichlorométhane et CBr₄ (67,7g, 202 mmol) est ajouté. Le milieu est refoidi à 0°C, puis une solution de triphénylphosphine (53 g, 202 mmol) dans 250 mL de dichlorométhane est ajoutée goutte à goutte. Le milieu réactionnel est alors réchauffé à température ambiante, puis agité 2 heures. Le milieu est alors traité par 500 mL d'eau et extrait par le dichlorométhane. Après séchage et concentration des phases organiques, le produit est purifié par chromatographie (éluant CH₂Cl₂/EtOAc), pour obtenir un solide blanc (pf: 83°C) avec un rendement de 93%.

### g) Acide 3-methoxymethoxyphenylboronique

10 g (57,8 mmol) de 3-bromophenol sont dissous dans 150 mL de DMF anhydre. 2,55 g (63,6 mmol) d'hydrure de sodium 60% sont alors ajoutés et le milieu réactionnel est agité pendant 1 heure. 4,83 mL (63,6 mmol) de chlorure de méthoxyméthyle sont alors ajoutés goutte à goutte et le mélange réactionnel est agité pendant 1 h. Après traitement par une solution saturée de chlorure d'ammonium, extraction avec de l'éther éthylique et évaporation des solvants de la phase organique, le résidu obtenu est dissous dans 200 mL de THF anhydre. Le mélange est refroidi à -78°C puis 25,4 mL (63,6 mmol) d'une solution 2,5 M de butyllithium sont ajoutés. Après agitation pendant 1 heure à -78°C, 15 mL (65 mmol) de borate de triisopropyle sont ajoutés goutte à goutte. Le milieu réactionnel est agité pendant 1 heure puis ramené à température ambiante, avant d'être traité par une solution d'acide chlorhydrique 1N. Le milieu est alors extrait avec de l'acétate d'éthyle puis les phases organiques sont réunies, séchées puis concentrées sous pression réduite. Après trituration dans l'heptane puis concentration, un solide marron est obtenu (PF = 45°C, m = 5,8 g ; R = 67%).

### (h) 3'-hydroxy-biyphenyl-4-carboxylate d'éthyle

2 g d'acide 3-methoxymethoxyphenylboronique (13,3 mmol) et 1,86 mL (11 mmol) de 4-iodobenzoate d'éthyle sont dissous dans 20 mL de DME. 13,3 mL d'une solution carbonate de potassium 2M (26,6 mmol) sont alors ajoutés et le milieu réactionnel est dégasé par un flux d'argon pendant 10 minutes. 640 mg de Pd(PPh₃)₄ sont alors ajoutés et le mélange est chauffé à 90°C pendant 14 heures. Après traitement par une solution saturée de chlorure d'ammonium, extraction avec de l'acétate d'éthyle puis séchage et évaporation des solvants de la phase organique, le résidu est dissous dans 25 mL de méthanol anhydre, puis 0,5 mL d'acide sufurique sont ajoutés. Le milieu réactionnel est porté à reflux pendant 15 h puis refroidi et enfin traité par addition d'eau. Après extraction avec de l'acétate d'éthyle, la phase organique est décantée, séchée puis concentrée sous pression réduite. Après purification par chromatographie sur colonne de silice, une huile incolore est obtenue (m = 2 g ; R = 75 %).

### (i) 3'-[3,4-Bis-(benzoyloxymethyl)-benzyloxy)-biphenyl-4-carboxylate de methyle

1 g (4,1 mmol) de 3'-hydroxy-biyphenyl-4-carboxylate d'éthyle, 1,98 g (4,5 mmol) de bromure de 3,4-Bis-(benzoyloxymethyl)-benzyle et 600 mg de carbonate de potassium sont dissous dans 40 mL de 2-butanone. Le mélange est porté à reflux (80°C) puis agité pendant 4 h. Après refroidissement, le milieu réactionnel est filtré puis concentré sous pression réduite. Le résidu est purifié par chromatographie sur colonne de silice, un solide blanc (PF 71-72°C) est obtenu (m = 2,18 g ; R = 88 %).

### j) {5-[4'-(1-Ethyl-1-hydroxy-propyl)-biphenyl-3-yloxymethyl]-2-hydroxymethyl-phenyl}-methanol

700 mg (1,16 mmol) de 3'-[3,4-bis-( benzoyloxymethyl)-benzyloxy]-biphenyl-4-carboxylate d'éthyle sont dissous dans 30 mL de THF anhydre, puis le mélange est refroidi à 0°C. 3,1 mL (9,3mmol) d'une solution de bromure d'ethylmagnesium (3 M) sont alors ajoutés, puis le milieu réactionnel est ramené à température ambiante et agité pendant 1 h. Après traitement par une solution saturée de chlorure d'ammonium puis extraction avec de l'acétate d'éthyle, les phases organiques sont réunies, séchées et concentrées sous pression réduite. Après purification par chromatographie sur colonne de silice, une huile incolore est obtenue ( m = 385 mg, R = 82% ).
**RMN** ^{**1**}**H** (CDCl₃): 0,78 (6H, t, *J*=7,3Hz), 1,77-1,93 (4H, m), 3.28 (2H, bs), 4,68 (2H, s), 4.69 (2H, s), 5,08 (2H, s), 6,95 (1H, dd), 7,19-7,42 (8H, m), 7,52 (1H, s), 7,55 (1H, s). ^{**13**}**C (DEPT)** 8.3 (2CH₃), 35,3 (CH₂), 64,2 (CH₂), 64,4 (CH₂), 70,0 (CH₂), 77,8 (C^{IV}), 113,7 (CH), 114,1 (CH), 120,3 (CH), 126,4 (2CH), 127,1 (2CH), 127,9 (CH), 129,2 (CH), 130,2 (CH), 130,4 (CH), 137,7 (C^{IV}), 139,1 (C^{IV}), 139,6 (C^{IV}), 140,2 (C^{IV}), 142,9 (C^{IV}), 145,5 (C^{IV}), 159,4 (C^{IV}).

### EXEMPLE 2

### {2-Hydroxymethyl-5-[4'-(1-hydroxy-1-methyl-ethyl)-biphenyl-3-yloxymethyl]-phenyl}-methanol

De manière analogue à l'exemple 1(j), par traitement de 3'-[3,4-bis-( benzoyloxymethyl)-benzyloxy] -biphenyl-4-carboxylate d'éthyle (700 mg, 1,16 mmol) par une solution de bromure de méthylmagnésium (3 M) (3,1 mL, 9,3 mmol), un solide blanc (PF 88-90°C) est obtenu après purification par chromatographie sur colonne de silice (m = 405 mg, R = 93 %).
**RMN** ^{**1**}**H** (CDCl₃): 1,60 (6H, s), 2,4 (1H, bs), 3,95 (2H, bs), 4,70 (2H, s), 4.71 (2H, s), 5,09 (2H, s), 6,92 (1H, dd, *J*_{*1*}*=* 2,5Hz, *J*_{*2*}= 7,2Hz), 7,17-7,19 (2H, m), 7,30-7,38 (3H, m), 7,43 (1H, s), 7,54 (4H, s). ^{**13**}**C (DEPT)** 32,1 (2CH₃), 64,1 (CH₂), 64,3 (CH₂), 70,0 (CH₂), 72,7 (C^{IV}), 113,8 (CH), 114,1 (CH), 120,3 (CH), 125,3 (2CH), 127,3 (2CH), 127,7 (CH), 129,1 (CH), 130,2 (CH), 130,3 (CH), 137,5 (C^{IV}), 139,6 (C^{IV}), 139,9 (C^{IV}), 140,5 (C^{IV}), 142,8 (C^{IV}), 149,0 (C^{IV}), 159,4 (C^{IV}).

### EXEMPLE 3

### {5-[4'-(1-Ethyl-1-hydroxy-propyl)-2'-methyl-biphenyl-3-yloxymethyl]-2-hydroxymethyl-phenyl}-methanol

### (a) 3'-Hydroxy-2-methyl-biphenyl-4-carboxylate d'éthyle

De manière analogue à l'exemple 1(h), par réaction de 2 g (13,3 mmol) d'acide 3-methoxymethoxyphenylboronique avec 1,9 g (8,9 mmol) d'acide 3-methyl-4-bromobenzoïque, on obtient 1,6 g (74%) du produit attendu.

### (b) 3'-[3,4-bis-( benzoyloxymethyl)-benzyloxy]-2-méthyl-biphenyl-4-carboxylate de méthyle

De manière analogue à l'exemple 1(i), par réaction de 1,6 g (6,6 mmol) de 3'-Hydroxy-2-methyl-biphenyl-4-carboxylate d'éthyle avec 3,47 g (7,9 mmol) de bromure de 3,4-Bis-(benzoyloxymethyl)-benzyle, on obtient 3,7 g (94%) de 3'-[3,4-bis-(1-phenyl-methanoyloxymethyl)-benzyloxy]-2-méthyl-biphenyl-4-carboxylate de méthyle

### (c) {5-[4'-(1-Ethyl-1-hydroxy-propyl)-2'-methyl-biphenyl-3-yloxymethyl]-2-hydroxymethyl-phenyl}-methanol

De manière analogue à l'exemple 1(j), par traitement de 3'-[3,4-bis benzoyloxymethyl)-benzyloxy]-2-méthyl-biphenyl-4-carboxylate de méthyle (800 mg, 1,33 mmol) par une solution de bromure d'éthylmagnésium (3 M) (3,6 mL, 10 mmol). Une huile incolore est obtenue après purification par chromatographie sur colonne de silice (m = 475 mg, R = 85 %).
**RMN** ^{**1**}**H** (CDCl₃): 0,81 (6H, t, *J*=7,5Hz), 1,74 (1H,s), 1,81-1,94 (4H, m), 2,26 (3H,s), 3.14 (2H, bs), 4,72 (4H, bs), 5,07 (2H, s), 6,91-6,95 (3H, m), 7,18 (2H, s), 7,26 (1H, s), 7,29-7,42 (4H, m). ^{**13**}**C (DEPT)** 8.3 (2CH₃), 14,5 (CH₃), 35,2 (CH₂), 64,3 (CH₂), 64,6 (CH₂), 69,9 (CH₂), 77,7 (C^{IV}), 113,6 (CH), 116,2 (CH), 122,6 (CH), 123,3 (CH), 127,8 (CH), 127,9 (CH), 129,2 (CH), 129,5 (CH), 129,7 (CH), 130,4 (CH), 135,1 (C^{IV}), 137,8 (C^{IV}), 139,5 (C^{IV}), 139,9 (C^{IV}), 140,1 (C^{IV}), 143,8 (C^{IV}), 145,3 (C^{IV}), 158,7 (C^{IV}).

### EXEMPLE 4

### {2-Hydroxymethyl-5-[4'-(1-hydroxy-1-methyl-ethyl)-2'-methyl-biphenyl-3-yloxymethyl]-phenyl}-methanol

De manière analogue à l'exemple 3(c), par traitement de 3'-[3,4-bis-( benzoyloxymethyl)-benzyloxy]-2-méthyl-biphenyl-4-carboxylate de méthyle (800 mg, 1,33 mmol) par une solution de bromure de méthylmagnésium (3 M) (3,6 mL, 10 mmol). Un solide blanc est obtenu (PF 45°C) après purification par chromatographie sur colonne de silice (m = 430 mg, R = 83 %).
**RMN** ^{**1**}**H** (CDCl₃): 1,61 (6H, s), 1,82 (1H,s), 2,26 (3H,s), 2,95 (s, 1H), 2,96 (s, 1H), 4,74 (4H, bs), 5,07 (2H, s), 6,91-6,96 (3H, m), 7,18-7,42 (7H, m). ^{**13**}**C (DEPT)** 21,0 (CH₃), 32,2 (2CH₃), 64,4 (CH₂), 64,6 (CH₂), 69,9 (CH₂), 72,8 (C^{IV}), 113,7 (CH), 116,2 (CH), 122,2 (CH), 122,6 (CH), 126,8 (CH), 127,9 (CH), 129,2 (CH), 129,5 (CH), 130,0 (CH), 130,4 (CH), 135,5 (C^{IV}), 137,8 (C^{IV}), 139,5 (C^{IV}), 140,1 (C^{IV}), 140,5 (C^{IV}), 143,6 (C^{IV}), 148,5 (C^{IV}), 158,7 (C^{IV}).

### EXEMPLE 5

### (5-{2-[3'-(1-Ethyl-1-hydroxy-propyl)-biphenyl-3-yl]-ethyl}-2-hydroxymethyl-phenyl)-methanol

### (a) 3'-hydroxy-biphenyl-3-carboxylate d'éthyle

De manière analogue à l'exemple 1(h), par réaction de 2 g (13,3 mmol) d'acide 3-methoxymethoxyphenylboronique avec 1,83 ml (11 mmol) de 3-iodobenzoate d'éthyle, on obtient 1,9 g (72%) de l'ester éthylique attendu.

### (b) 3'-[3,4-bis-(benzoyloxymethyl)-benzyloxy]-biphenyl-3-carboxylate d'éthyle

De manière analogue à l'exemple 1(i), par réaction de 1 g (4,1 mmol) de 3'-hydroxy-biphenyl-3-carboxylate d'éthyle avec 1,98 g (4,5 mmol) de bromure de 3,4-Bis-(benzoyloxymethyl)-benzyle, on obtient 2,1 g (85%) de 3'-[3,4-bis-( benzoyloxymethyl)-benzyloxy]-biphenyl-3-carboxylate d'éthyle.

### (c) (5-{2-[3'-{1-Ethyl-1-hydroxy-propyl)-biphenyl-3-yl]-ethyl}-2-hydroxymethyl-phenyl)-methanol

De manière analogue à l'exemple 1(j), par traitement de 3'-[3,4-bis-( benzoyloxymethyl)-benzyloxy]-biphenyl-3-carboxylate d'éthyle (400 mg, 0,66 mmol) par une solution de bromure d'éthylmagnésium (3M) (1.8 mL, 5.3 mmol). Une huile incolore est obtenue après purification par chromatographie sur colonne de silice (m = 217 mg, R = 81 %).
**RMN** ^{**1**}**H** (CDCl₃): 0,77 (6H, t, *J*=7,2Hz), 1,25 (1H, bs), 1,79-1,88 (4H, m), 3.37 (2H, bs), 4,68 (2H, s), 4,70 (2H, s), 5,11 (2H, s), 6,94 (1H, dd, *J*_{*1*}*=* 1,3Hz, *J*_{*2*}*=* 8,3Hz)), 7,18 (2H, s), 7,21-7,42 (8H, m), 7,53 (1H, d, *J*= 1,1Hz). ^{**13**}**C (DEPT)** 8.3 (2CH₃), 35,2 (CH₂), 64,2 (CH₂), 64,5 (CH₂), 70,1 (CH₂), 78,0 (C^{IV}), 114,1 (CH), 114,4 (CH), 120,4 (CH), 124,8 (CH), 125,1 (CH), 125,5 (CH), 127,8 (CH), 128,8 (CH), 129,0 (CH), 130,2 (CH), 130,4 (CH), 137,8 (C^{IV}), 139,6 (C^{IV}), 140,2 (C^{IV}), 141,0 (C^{IV}), 143,4 (C^{IV}), 146,7 (C^{IV}), 159,3 (C^{IV}).

### EXEMPLE 6

### {2-Hydroxymethyl-5-[3'-(1-hydroxy-1-methyl-ethyl)-biphenyl-3-yloxymethyl]-phenyl}-methanol

De manière analogue à l'exemple 5(c), par traitement de 3'-[3,4-bis-( benzoyloxymethyl)-benzyloxy]-biphenyl-3-carboxylate d'éthyle (380 mg, 0,63 mmol) par une solution de bromure de méthylmagnésium 3.0 M (1.7 mL, 5 mmol). Un solide blanc est obtenu (PF 103-104°C) après purification par chromatographie sur colonne de silice (m = 224 mg, R = 94 %).
**RMN** ^{**1**}**H** (CDCl₃+DMSO): 1,60 (6H, s), 2,96 (1H, bs), 4,29 (1H, t), 4,39 (1H, t), 4,71 (4H, t, J= 5.7Hz)), 5,12 (2H, s), 6,94 (1H, dd, *J*_{*1*}*=* 2,3Hz, *J*_{*2*}*=* 8,1Hz)), 7,18 (2H, m), 7,29-7,47 (8H, m), 7,69 (1H, s). ^{**13**}**C (DEPT)** 32,2 (2CH₃), 63,9 (CH₂), 64,1 (CH₂), 70,1 (CH₂), 72,7 (C^{IV}), 114,2 (CH), 114,2 (CH), 120,3 (CH), 123,8 (CH), 124,1 (CH), 125,6 (CH), 127,5 (CH), 128,9 (CH), 129,0 (CH), 130,2 (CH), 130,3 (CH), 137,4 (C^{IV}), 140,0 (C^{IV}), 140,7 (C^{IV}), 141,1 (C^{IV}), 143,3 (C^{IV}), 150,4 (C^{IV}), 159,3 (C^{IV}).

### EXEMPLE 7

### {{5-[4'-(2-Ethyl-2-hydroxy-butyl)-biphenyl-3-yloxymethyl]-2-hydroxymethyl-phenyl}-methanol

### (a) (3'-Hydroxy-biphenyl-4-yl)-acetate d'éthyle

De manière analogue à l'exemple 1(h), par réaction de 543 mg (3,6 mmol) d'acide 3-methoxymethoxyphenylboronique avec 700 mg (2,4 mmol) de 4-iodophénylacetate d'éthyle, on obtient 630 mg (68%) de l'ester éthylique attendu.

### (b) {3'-[3,4-bis-(benzoyloxymethyl)-benzyloxy]-biphenyl-4-yl}-acétate d'éthyle

De manière analogue à l'exemple 1(i), par réaction de 360 mg (1,4 mmol) de (3'-Hydroxy-biphenyl-4-yl)-acetate d'éthyle avec 676 mg (1,5 mmol) de bromure de 3,4-Bis-(benzoyloxymethyl)-benzyle, on obtient 810 mg (94%) de {3'-[3,4-bis-(benzoyloxymethyl)-benzyloxy]-biphenyl-4-yl}-acétate d'éthyle.

### (c) {{5-[4'-(2-Ethyl-2-hydroxy-butyl)-biphenyl-3-yloxymethyl]-2-hydroxymethyl-phenyl}-methanol

De manière analogue à l'exemple 1(j), par traitement de {3'-[3,4-bis-( benzoyloxymethyl)-benzyloxy]-biphenyl-4-yl}-acétate d'éthyle (400 mg, 0,66 mmol) par une solution de bromure d'éthylmagnésium 3.0 M (1.8 mL, 5.3 mmol). Une huile incolore est obtenue après purification par chromatographie sur colonne de silice (m = 236 mg, R = 85 %).
**RMN** ^{**1**}**H** (CDCl₃): 0,94 (6H, t, *J*=7,5Hz), 1,27 (1H, bs), 1,48 (4H, q, *J*= 7,5Hz), 2,77 (2H, s), 3.17 (2H, bs), 4,71 (4H, s), 5,09 (2H, s), 6,91-6,95 (1H, m), 7,17-7,20 (2H, m), 7,26-7,42 (6H, m), 7,49 (1H, s), 7,52 (1H, m). ^{**13**}**C (DEPT)** 8.4 (2CH₃), 30,9 (CH₂), 44,8 (CH₂), 64,3 (CH₂), 64,5 (CH₂), 70,0 (CH₂), 75,1 (C^{IV}), 113,8 (CH), 114,1 (CH), 120,3 (CH), 127,3 (2CH), 127,9 (CH), 129,2 (CH), 130,2 (CH), 130,4 (CH), 131,4 (2CH), 137,3 (C^{IV}), 137,7 (C^{IV}), 139,3 (C^{IV}), 139,6 (C^{IV}), 140,2 (C^{IV}), 142,9 (C^{IV}), 159,4 (C^{IV}).

### EXEMPLE 8

### {5-[3'-(2-Ethyl-2-hydroxy-butyl)-biphenyl-3-yloxymethyl]-2-hydroxymethyl-phenyl}-methanol

### (a) (3'-Hydroxy-biphenyl-3-yl)-acetate de méthyle

De manière analogue à l'exemple 1(h), par réaction de 2 g (13,3 mmol) d'acide 3-methoxymethoxyphenylboronique avec 2 g (9,3 mmol) d'acide 3-bromophénylacetique, on obtient 1,6 g (72%) d'ester méthylique.

### (b) {3'-[3,4-bis-( benzoyloxymethyl)-benzyloxy]-biphenyl-3-yl}-acétate de méthyle

De manière analogue à l'exemple 1(i), par réaction de 1 g (4,1 mmol) de (3'-Hydroxy-biphenyl-3-yl)-acetate de méthyle avec 2 g (4,5 mmol) de bromure de 3,4-Bis-(benzoyloxymethyl)-benzyle, on obtient 2,2 g (91%) de {3'-[3,4-bis-( benzoyloxymethyl)-benzyloxy]-biphenyl-3-yl}-acétate de méthyle

### (c) {5-[3'-(2-Ethyl-2-hydroxy-butyl)-biphenyl-3-yloxymethyl]-2-hydroxymethyl-phenyl}-methanol

De manière analogue à l'exemple 1(j), par traitement de {3'-[3,4-bis-( benzoyloxymethyl)-benzyloxy]-biphenyl-3-yl}-acétate de méthyle (700 mg, 1,16 mmol) par une solution de bromure d'éthylmagnésium 3.0 M (3,1 mL, 9,3 mmol). Une huile incolore est obtenue après purification par chromatographie sur colonne de silice (m = 419 mg, R = 86 %).
**RMN** ^{**1**}**H** (CDCl₃): 0,92 (6H, t, *J*=7,5Hz), 1,47 (4H, d, *J*=7,5 Hz), 2,78 (2H, s), 3,44 (1H, bs), 3,54 (1H, bs), 4,66 (2H, s), 4,67 (2H, s), 5,10 (2H, s), 6,94-6.96 (1H, m), 7,14-7,25 (3H, m), 7,29-7,43 (7H, m). ^{**13**}**C (DEPT)** 8.4 (2CH₃), 30,8 (CH₂), 45,3 (CH₂), 64,2 (CH₂), 64,4 (CH₂), 70,1 (CH₂), 75,2 (C^{IV}), 114,1 (CH), 114,3 (CH), 120,4 (CH), 125,6 (CH), 127,7 (CH), 128,9 (CH), 129,0 (CH), 129,7 (CH), 130,2 (CH), 130,2 (CH), 130,4 (CH), 137,8 (C^{IV}), 138,3 (C^{IV}), 139,6 (C^{IV}), 140,3 (C^{IV}), 141,2 (C^{IV}), 143,0 (C^{IV}), 159,3 (C^{IV}).

### EXEMPLE 9

### 1-[3'-(3,4-Bis-hydroxymethyl-benzyloxy)-biphenyl-3-yl]-2-methyl-propan-2-ol

De manière analogue à l'exemple 8(c), par traitement de {3'-[3,4-bis-( benzoyloxymethyl)-benzyloxy)-biphenyl-3-yl}-acétate de méthyle (700 mg, 1,13 mmol) par une solution de bromure d'éthylmagnésium (3M) (3,1 mL, 9,3 mmol). Une huile incolore est obtenue après purification par chromatographie sur colonne de silice (m = 419 mg, R = 92 %).
**RMN** ^{**1**}**H** (CDCl₃): 1,24 (6H, s), 1,65 (1H, bs), 2,80 (2H, s), 3,38 (1H, bs), 3,50 (1H, bs), 4,68 (2H, s), 4,69 (2H, s), 5,11 (2H, s), 6,94-6.96 (1H, m), 7,13-7,19 (3H, m), 7,30-7,44 (7H, m). ^{**13**}**C (DEPT)** 29,6 (2CH₃), 50,1 (CH₂), 64,2 (CH₂), 64,4 (CH₂), 70,1 (CH₂), 71,3 (C^{IV}), 114,2 (CH), 114,4 (CH), 120,4 (CH), 125,7 (CH), 127,7 (CH), 128,9 (CH), 129,0 (CH), 129,6 (CH), 130,0 (CH), 130,2 (CH), 130,4 (CH), 137,8 (C^{IV}), 138,6 (C^{IV}), 139,6 (C^{IV}), 140,3 (C^{IV}), 141,2 (C^{IV}), 142,9 (C^{IV}), 159,3 (C^{IV}).

### EXEMPLE 10

### {4-[4'-(1-Ethyl-1-hydroxy-propyl)-2'-methyl-biphenyl-3-ylsulfanylmethyl]-2-hydroxymethyl-phenyl}-methanol

### a) 4-(3-bromo-phenylsufanylmethyl)-phtalate de diméthyle

5 g (26 mmol) de 3-bromothiophenol et 9,2 g (32 mmol) de 4-bromomethyl-phthalate de diméthyle sont dissous dans 150 mL de 2-butanone. 4 g (29 mmol) de carbonate de potassium sont additionnés et le mélange est porté à reflux et agité pendant 2 h. Le milieu réactionnel est alors filtré, concentré sous pression réduite et le résidu est purifié par chromatographie sur colonne de silice. Une huile jaune (10 g) est obtenue avec un rendement de 100%.

### b) [5-(3-Bromo-phenylsulfanylmethyl)-2-hydroxymethyl-phenyl]-methanol

4-(3-bromo-phenylsufanylmethyl)-phtalate de diméthyle (10 g, 26 mmol) est dissous dans 120 mL de THF anhydre. 2,2 g de borohydrure de lithium (100 mmol) sont alors ajoutés lentement, puis le milieu réactionnel est chauffé à reflux pendant 12 h. Après refroidissement et traitement par une solution saturée de chlorure d'ammonium, le milieu réactionnel est extrait par de l'acétate d'éthyle. Les phases organiques sont rassemblées, séchées et concentrées sous pression réduite. Le résidu est purifié par chromatographie sur colonne de silice, une huile incolore est obtenue (7,8 g, R = 89%).

### (j) [2-Hydroxymethyl-5-(3-tributylstannyl-phenylsulfanylmethyl)-phenyl]-methanol

6,2 g (18 mmol ) de [5-(3-Bromo-phenylsulfanylmethyl)-2-hydroxymethyl-phenyl]-methanol sont dissous dans 120 mL de toluène anhydre. Le mélange est dégasé par un flux d'argon pendant 10 minutes, puis 18 mL (36 mmol) de hexabutyl de dietain, et 416 mg (0,36 mmol) de Pd(PPh₃)₄ sont ajoutés. Le milieu réactionnel est alors agité à 120 °C pendant 48 heures, refroidi, traité par une solution saturée de chlorure d'ammonium et extrait avec de l'acétate d'éthyle. Les phases organiques sont rassemblées, séchées puis concentrées sous pression réduite. Le résidu est purifié par chromatographie sur colonne de silice (éluant heptane pur) pour obtenir une huile jaune (m = 3,35 g, R = 34%).

### (k) 3'-(3,4-Bis-hydroxymethyl-benzylsulfanyl)-2-methyl-biphenyl-4-carboxylate de méthyle

1,67 g (3 mmol) de [2-Hydroxymethyl-5-(3-tributylstannyl-phenylsulfanylmethyl)-phenyl]-methanol sont dissous dans 30 mL de toluène et 5 mL de DME. 970 mg (4,5 mmol) d'acide 4-bromo-3-methyl-benzoique sont ajoutés et le mélange est dégasé par un flux d'argon pendant 10 minutes. Pd(PPh₃)₄ (175 mg, 0.15 mmol) est alors additionné et le milieu réactionnel est porté à reflux et agité pendant 24 h. Le milieu réactionnel est alors traité avec une solution de chlorure d'ammonium puis extrait avec de l'acétate d'éthyle. Les phases organiques sont rassemblées, séchées puis concentrées sous pression réduite. Le résidu est alors dissous dans 50 mL de méthanol, 1 mL d'acide sulfurique est ajouté puis le mélange est agité à reflux pendant 18 h. Après refroidissement et traitement par de l'eau, le mélange est extrait avec de l'acétate d'éthyle. Les phases organiques sont rassemblées, séchées puis concentrées sous pression réduite. Le résidu est alors purifié par chromatographie sur colonne de silice. Une huile incolore est obtenue (m = 180 mg, R = 15 %).

### f) {4-[4'-(1-Ethyl-1-hydroxy-propyl)-2'methyl-biphenyl-3-ylsulfanylmethyl]-2-hydroxymethyl-phenyl}-methanol

130 mg (0.3 mmol) de 3'-(3,4-Bis-hydroxymethyl-benzylsulfanyl)-2-methyl-biphenyl-4-carboxylate de méthyle sont dissous dans 10 mL de THF anhydre puis 0.7 mL (2 mmol) d'une solution de bromure d'éthylmagnesium (3M) sont ajoutés. Le milieu réactionnel est agité pendant 1 h, puis traité par une solution de chlorure d'ammonium. Après extraction avec de l'acétate d'éthyle, les phases organiques sont rassemblées, séchées et concentrées sous pression réduite. Le résidu est purifié par chromatographie sur colonne de silice. Une huile incolore est obtenue (m = 105 mg, R = 81 %).
**RMN** ^{**1**}**H** (DMSO): 0,46 (6H, t, *J*= 6,8Hz), 1,40-1,60 (4H, m), 1,97 (3H,s), 4,08 (2H, s), 4,39 (2H, s), 4,40 (2H, s), 4,85 (1H, t), 4,90 (1H, t), 6,85-7,23 (10H, m). ^{**13**}**C (DEPT)** 6.3 (2CH₃), 18,7 (CH₃), 32,8 (2CH₂), 34,7 (CH₂), 58,4 (CH₂), 58,4 (CH₂), 73,7 (C^{IV}), 121,6 (CH), 124,6 (CH), 124,8 (CH), 125,0 (CH), 125,1 (CH), 125,4 (CH), 125,9 (CH), 126,2 (CH), 126,6 (CH), 126,9 (CH), 131,8 (C^{IV}), 133,7(C^{IV}), 134,6 (C^{IV}), 136,1 (C^{IV}), 136,4 (C^{IV}), 137,8 (C^{IV}), 140,3 (C^{IV}), 144,4 (C^{IV}).

### EXEMPLE 11

### {4-[4'-(1-Ethyl-1-hydroxy-propyl)-2,2'-dimethyl-biphenyl-3-yloxymethyl]-2-hydroxymethyl-phenyl}-methanol

### a) 3-Bromo-2-methyl-phenol

20 g (107 mmol) de 3-bromo-2-methyl-phenylamine sont dissous dans 150 ml d'acide sulfurique aqueux 1,0 M et le mélange est refroidi à 0°C. Une solution de 8,9 g (129 mmol) de nitrite de sodium dans 20 ml d'eau est additionnée lentement. Le milieu est agité 15 minutes à 0°C, puis 50 ml d'acide sulfurique concentré sont ajoutés. Le milieu réactionnel est alors chauffé à 100°C pendant 1 heure, puis refroidi et dilué dans de l'eau. Après extraction à l'éther éthylique, le résidu obtenu est recristallisé dans un mélange heptane - dichlorométhane. Un solide jaune est obtenu (PF = 89°C; m = 12,3 g ; R = 61%).

### b) 1-Bromo-3-methoxymethoxy-2-methyl-benzene

13,9 g (74 mmol) de 3-bromo-2-methyl-phenol sont dissous dans 120 ml de diméthylformamide, et le mélange est refroidi à 0°C. 3,6 g (90 mmol) d'hydrure de sodium 60% sont alors ajoutés par petites portions, et le milieu est agité pendant 1 heure. 6,8 ml (90 mmol) de chlorure de méthoxyméthyle sont alors additionnés lentement, et le milieu est ramené à température ambiante, puis agité pendant 1 heure. Après le traitement usuel, le résidu est purifié par chromatographie sur gel de silice (éluant heptane 80 - acétate d'éthyle 20). Le produit désiré est obtenu sous forme d'huile incolore (m = 16,2 g ; R = 95%).

### c) Acide 3-methoxymethoxy-2-methyl-benzene-1-boronique

16,2 g (70 mmol) de 1-bromo-3-methoxymethoxy-2-methyl-benzene sont dissous dans 200 ml de THF anhydre, et le mélange est refroidi à -78°C. 33,7 ml (84 mmol) de butyllithium 2,5 M sont additionnés lentement, puis le mélange est maintenu à -78°C pendant 1 heure. Alors 19,4 ml (84 mmol) de borate de triisopropyle sont ajoutés goutte à goutte sur une période de 15 minutes. Le milieu est agité 30 minutes à la même température, puis versé dans 300 ml d'acide chlorhydrique 1 N. Après le traitement usuel, le résidu solide blanchâtre obtenu est rinçé avec de l'heptane, puis séché sous pression réduite. Un solide floconneux blanc est obtenu (m = 11,8 g ; R = 86%).

### d) 3'-Hydroxy-2,2'-dimethyl-biphenyl-4-carboxylate de méthyle

1,03 g (5,2 mmol) d'acide 3-methoxymethoxy-2-methyl-benzene-1-boronique, 1 g (4,4 mmol) de 4-bromo-3-méthyl-benzoate de méthyle et 4,4 ml d'une solution 2,0 M de carbonate de potassium sont dissous dans 20 ml d'éther diméthylique de l'éthylène glycol. Le mélange est dégazé à l'aide d'un flux d'azote pendant 10 minutes, puis 250 mg (0,22 mmol) de palladium tetrakis-triphenylphosphine sont ajoutés, et le milieu est agité pendant 24 heures à 80°C. Après refroidissement et traitement usuel, le résidu obtenu est dissous dans 30 ml de méthanol, et 0,5 ml d'acide sulfurique sont ajoutés; le milieu est agité à température ambiante pendant 6 heures, puis versé dans un mélange éther éthylique - eau. Après extraction à l'éther le résidu est purifié par chromatographie sur gel de silice (éluant heptane 9 - acétate d'éthyle 1). Le produit désiré est obtenu sous forme d'huile épaisse et incolore (m = 890 mg ; R = 79%).

### e) 3'-[3,4-Bis-(1-phenyl-methanoyloxymethyl)-benzyloxy]-2,2'-dimethyl-biphenyl-4-carboxylate de méthyle

De manière analogue à l'exemple 1(i) par réaction de 515 mg (2 mmol) de 3'-hydroxy-2,2'-dimethyl-biphenyl-4-carboxylate de méthyle avec 880 mg (2 mmol) de bromure de 3,4-bis-(benzoyloxymethyl)-benzyle et 300 mg (2,2 mmol) de carbonate de potassium. Le produit désiré est obtenu sous forme d'huile incolore (m = 1,22 g ; R = 99%).

### f) {4-[4'-(1-Ethyl-1-hydroxy-propyl)-2,2'-dimethyl-biphenyl-3-yloxymethyl]-2-hydroxymethyl-phenyl}-methanol

De manière analogue à l'exemple 1(j) par réaction de 1,22 g (1,98 mmol) de 3'-[3,4-bis-(1-phenyl-methanoyloxymethyl)-benzyloxy)-2,2'-dimethyl-biphenyl-4-carboxylate de méthyle avec 5,3 ml (16 mmol) de bromure d'éthylmagnésium 3,0 M. Le produit désiré est obtenu sous forme de poudre blanche (PF = 81-82°C ; m = 360 mg ; R = 42%).
**RMN** ^{**1**}**H** (CDCl₃): 0,82 (t, J = 7,4 Hz, 6H); 1,70-1,87 (m, 4H); 1,96 (s, 3H); 2,10 (s, 3H); 4,76 (s, 2H); 4,77 (s, 2H); 5,12 (s, 2H); 6,77 (d, J = 7,0 Hz, 1H); 6,88 (d, J = 6,9 Hz, 1H); 7,05 (d, J = 7,9 Hz, 1H); 7,17 (t, J = 7,7 Hz, 2H); 7,26 (s, 1H); 7,37-7,46 (m, 3H).

### EXEMPLE 12

### {2-Hydroxymethyl-4-[4'-(1-hydroxy-1-propyl-butyl)-2,2'-dimethyl-biphenyl-3-yloxymethyl]-phenyl}-methanol

### a) {2-Hydroxymethyl-4-[4'-(1-hydroxy-1-propyl-butyl)-2,2'-dimethyl-biphenyl-3-yloxymethyl)-phenyl}-methanol

De manière analogue à l'exemple 1(j) par réaction de 100 mg (0,25 mmol) de 3'-[3,4-bis-(1-phenyl-methanoyloxymethyl)-benzyloxy]-2,2'-dimethyl-biphenyl-4-carboxylate de méthyle (décrit à l'exemple 11(e)) avec 0,5 ml (1 mmol) de bromure d'éthylmagnésium 2,0 M. Le produit désiré est obtenu sous forme de poudre blanche (PF = 118-120°C ; m = 64 mg ; R = 56%).
**RMN** ^{**1**}**H** (CDCl₃): 0,9 (t, J = 7,2 Hz, 6H); 1,16-1,36 (m, 4H); 1,73-1,85 (m, 4H); 1,97 (s, 3H); 2,06 (s, 3H); 4,76 (s, 2H); 4,77 (s, 2H); 5,11 (s, 2H); 6,77 (d, J = 6,9 Hz, 1H); 6,89 (d, J = 6,9 Hz, 1 H); 7,04 (d, J = 7,9 Hz, 1H); 7,18 (t, J = 7,7 Hz, 2H); 7,26 (s, 1H); 7,37-7,46 (m, 3H).

### EXEMPLE 13

### {4-[4'-(1-Ethyl-1-hydroxy-propyl)-2-methyl-biphenyl-3-yloxymethyl]-2-hydroxymethyl-phenyl}-methanol

### a) 3'-Hydroxy-2'-methyl-biphenyl-4-carboxylate de méthyle

De manière analogue à l'exemple 11(d), par réaction de 3,3 g (16,7 mmol) d'acide 3-methoxymethoxy-2-methyl-benzene-1-boronique (décrit à l'exemple 11c) avec 3 g (13,9 mmol) de 4-iodobenzoate d'éthyle, 16,7 ml d'une solution 2,0 M de carbonate de potassium et 800 mg (0,69 mmol) de palladium tetrakis-triphenylphosphine, puis déprotection dans le méthanol. Le produit désiré est obtenu sous forme d'huile incolore (m = 3,07 g ; R = 77%).

### b) 3'-[3,4-Bis-(1-phenyl-methanoyloxymethyl)-benzyloxy]-2'-methyl-biphenyl-4-carboxylate de méthyle

De manière analogue à l'exemple 1(i) par réaction de 1 g (4,1 mmol) de 3'-hydroxy-2'-methyl-biphenyl-4-carboxylate de méthyle avec 2 g (4,5 mmol) de bromure de 3,4-bis-(benzoyloxymethyl)-benzyle et 650 mg (4,7 mmol) de carbonate de potassium. Le produit désiré est obtenu sous forme d'huile incolore (m = 2,4 g ; R = 97%).

### c) {4-[4'-(1-Ethyl-1-hydroxy-propyl)-2-methyl-biphenyl-3-yloxymethyl]-2-hydroxymethyl-phenyl}-methanol

De manière analogue à l'exemple 1(j) par réaction de 2,4 g (4 mmol) de 3'-[3,4-bis-(1-phenyl-methanoyloxymethyl)-benzyloxy]-2'-methyl-biphenyl-4-carboxylate de méthyle avec 10,7 ml (32 mmol) de bromure d'éthylmagnésium 3,0 M. Le produit désiré est obtenu sous forme de poudre blanche (PF = 117-118°C ; m = 1,1 mg ; R = 60%).
**RMN** ^{**1**}**H** (DMSO): 0,76 (t, J = 7,5 Hz, 6H); 1,70-1,90 (m, 4H); 2,13 (s, 3H); 4,56-4,61 (m, 5H); 5,17 (m, 4H); 6,8 (m, 1H); 7,1 (m, 1H); 7,20-7,30 (m, 3H); 7,40-7,60 (m, 4H).

### EXEMPLE 14

### {4-[4'-(1-Ethyl-1-hydroxy-propyl)-2,2',6'-trimethyl-biphenyl-3-yloxymethyl]-2-hydroxymethyl-phenyl}-methanol

### a) 3'-Hydroxy-2,6,2'-trimethyl-biphenyl-4-carboxylate de méthyle

De manière analogue à l'exemple 1 1(d), par réaction de 1,5 g (7,6 mmol) d'acide 3-methoxymethoxy-2-methyl-benzene-1-boronique (décrit à l'exemple 11c) avec 2 g (6,4 mmol) de 3,5-dimethyl-4-trifluoromethanesulfonyloxy-benzoate de méthyle, 7,7 ml d'une solution 2,0 M de carbonate de potassium et 370 mg (0,32 mmol) de palladium tetrakis-triphenylphosphine, puis déprotection dans le méthanol. Le produit désiré est obtenu sous forme de solide blanc (PF = 149°C ; m = 1,34 g ; R = 67%).

### b) 3'-[3,4-Bis-(1-phenyl-methanoyloxymethyl)-benzyloxy)-2,6,2'-trimethyl-biphenyl-4-carboxylate de méthyle

De manière analogue à l'exemple 1(i) par réaction de 980 mg (3,6 mmol) 3'-hydroxy-2,6,2'-trimethyl-biphenyl-4-carboxylate de méthyle avec 1,75 g (4,5 mmol) de bromure de 3,4-bis-(benzoyloxymethyl)-benzyle et 570 mg (4,1 mmol) de carbonate de potassium. Le produit désiré est obtenu sous forme de cristaux blancs (PF = 131-132°C ; m = 2,16 g ; R = 95%).

### c) {4-[4'-(1-Ethyl-1-hydroxy-propyl)- 2,2',6'-trimethyl -biphenyl-3-yloxymethyl]-2-hydroxymethyl-phenyl}-methanol

De manière analogue à l'exemple 1(j) par réaction de 2,1 g (3,3 mmol) 3'-[3,4-bis-(1-phenyl-methanoyloxymethyl)-benzyloxy]-2,6,2'-trimethyl-biphenyl-4-carboxylate de méthyle avec 8,9 ml (27 mmol) de bromure d'éthylmagnésium 3,0 M. Le produit désiré est obtenu sous forme de poudre blanche (PF = 105-107°C ; m = 1,1 mg ; R = 73%).
**RMN** ^{**1**}**H** (CDCl₃): 0,82 (t, J = 7,5 Hz, 6H); 1,81-1,85 (m, 4H); 1,87 (s, 3H); 1,95 (s, 6H); 2,25 (bs, 3H); 4,75 (s, 2H); 4,76 (s, 2H); 5,11 (s, 2H); 6,68 (d, J = 7,5 Hz, 1H); 6,89 (d, J = 7,5 Hz, 1H); 7,1 (s, 2H); 7,15-7,22 (m, 1H); 7,36-7,43 (m, 2H), 7,46 (s, 1H).

### EXEMPLE 15

### (4-{3-[5-(1-Ethyl-1-hydroxy-propyl)-pyridin-2-yl]-phenoxymethyl}-2-hydroxymethyl-phenyl)-methanol

### a) 6-(3-Hydroxy-phenyl)-nicotinate d'éthyle

De manière analogue à l'exemple 1(h) par réaction de 1 g (6,7 mmol) d'acide 3-methoxymethoxyphenylboronique (décrit à l'exemple 1(g)) et 1,5 g (5,6 mmol) de 6-iodonicotinate d'éthyle avec 5,6 ml de carbonate de potassium 2,0 M et 320 mg de palladium tetrakis-triphenyphosphine, puis déprotection dans l'éthanol. Le produit désiré est obtenu sous forme de solide blanc (m = 354 mg ; R = 26%).

### b) 6-{3-[3,4-Bis-(1-phenyl-methanoyloxymethyl)-benzyloxy]-phenyl}-nicotinate d'éthyle

De manière analogue à l'exemple 1(i) par réaction de 277 mg (1,1 mmol) 6-(3-hydroxy-phenyl)-nicotinate d'éthyle avec 500 mg (1,1 mmol) de bromure de 3,4-bis-(benzoyloxymethyl)-benzyle et 166 mg (1,2 mmol) de carbonate de potassium. Le produit désiré est obtenu sous forme de cristaux blancs (PF = 118-120°C ; m = 500 mg ; R = 73%).

### c) (4-{3-[5-(1-Ethyl-1-hydroxy-propyl)-pyridin-2-yl]-phenoxymethyl}-2-hydroxymethyl-phenyl)-methanol

De manière analogue à l'exemple 1(j) par réaction de 410 mg (0,68 mmol) de 6-{3-[3,4-bis-(1-phenyl-methanoyloxymethyl)-benzyloxy]-phenyl}-nicotinate d'éthyle avec 1,8 ml (5,4 mmol) de bromure d'éthylmagnésium 3,0 M. Le produit désiré est obtenu sous forme de pâte incolore (m = 143 mg ; R = 51%).
**RMN** ^{**1**}**H** (CDCl₃): 0,81 (t, J = 7,5 Hz, 6H); 1,65 (bs, 1H); 1,73 (bs, 1H); 1,85-1,97 (m, 4H); 2,90 (bs, 3H); 4,74 (s, 2H); 4,76 (s, 2H); 5,15 (s, 2H); 7,01 (d, J = 7,6 Hz, 1H); 7,34-7,45 (m, 4H); 7,55 (d, J = 7,7 Hz, 1H); 7,67-7,77 (m, 2H); 7,78-7,81 (m, 1H); 8,67 (s, 1H).

### EXEMPLE 16

### {4-(4'-(1-Ethyl-1-hydroxy-propyl)-6,2'-dimethyl-biphenyl-3-yloxymethyl]-2-hydroxymethyl-phenyl}-methanol

### a) 3-Bromo-4-methyl-phenol

De manière analogue à l'exemple 11(a), par réaction de 20 g (107 mmol) de 3-bromo-4-methyl-phenylamine avec 8,9 g (129 mmol) de nitrite de sodium. Une huile brune est obtenue, sans purification (m = 20 g ; R = 100%).

### b) 2-Bromo-4-methoxymethoxy-1-methyl-benzene

De manière analogue à l'exemple 11(b) par réaction de 20 g (107 mmol) de 3-bromo-4-methyl-phenol avec 5,6 g (139 mmol) d'hydrure de sodium 60% et 8,9 ml (139 mmol) de chlorure de méthoxyméthyle. Le produit désiré est obtenu sous forme d'huile orangée (m = 12,3 g ; R = 50%).

### c) Acide 4-methoxymethoxy-1-methyl-benzene-2-boronique

De manière analogue à l'exemple 11(c), par réaction de 12,3 g (53 mmol) de 2-bromo-4-methoxymethoxy-1-methyl-benzene avec 28 ml (69 mmol) de butyllithium 2,5 M et 18,4 ml (80 mmol) de borate de triisopropyle. Une huile brune est obtenue (m = 10,4 g ; R = 99%).

### d) 5'-Hydroxy-2,2'-dimethyl-biphenyl-4-carboxylate de méthyle

De manière analogue à l'exemple 11(d) par réaction de 3,7 g (18,8 mmol) d'acide 4-methoxymethoxy-1-methyl-benzene-2-boronique avec 3,9 g (17 mmol) de 4-bromo-3-méthyl-benzoate de méthyle, 17 ml d'une solution 2,0 M de carbonate de potassium et 1 mg (0,85 mmol) de palladium tetrakis-triphenylphosphine , puis déprotection dans le méthanol. Le produit désiré est obtenu sous forme d'huile épaisse et incolore (m = 2,87 mg ; R = 51%).

### e) 5'-[3,4-Bis-(1-phenyl-methanoyloxymethyl)-benzyloxy]-2,2'-dimethyl-biphenyl-4-carboxylate de méthyle

De manière analogue à l'exemple 1(i) par réaction de 2,24 g (8,7 mmol) de 5'-hydroxy-2,2'-dimethyl-biphenyl-4-carboxylate de méthyle avec 4,20 mg (9,6 mmol) de bromure de 3,4-bis-(benzoyloxymethyl)-benzyle et 1,26 g (9 mmol) de carbonate de potassium. Le produit désiré est obtenu sous forme d'huile incolore (m = 5,34 g ; R = 99%).

### f) {4-[4'-(1-Ethyl-1-hydroxy-propyl)-6,2'-dimethyl-biphenyl-3-yloxymethyl]-2-hydroxymethyl-phenyl}-methanol

De manière analogue à l'exemple 1(j) par réaction de 5,3 g (8,6 mmol) de 5'-[3,4-Bis-(1-phenyl-methanoyloxymethyl)-benzyloxy]-2,2'-dimethyl-biphenyl-4-carboxylate de méthyle avec 23 ml (69 mmol) de bromure d'éthylmagnésium 3,0 M. Le produit désiré est obtenu sous forme de poudre blanche (m = 1,68 g ; R = 45%).
**RMN** ^{**1**}**H** (DMSO): 0,52 (t, J = 7,4 Hz, 6H); 1,51-1,58 (m, 4H); 1,72 (s, 3H); 1,83 (s, 3H); 4,31 (s, 1H); 4,36 (s, 1H); 4,88 (s, 2H); 4,89-4,92 (m, 2H); 6,53 (s, 1H); 6,70-6,74 (dd, J₁ = 2,6 Hz; J₂ = 6,7 Hz, 1H); 6,80 (d, J = 7,8 Hz, 1H); 6,99-7,04 (m, 2H); 7,1 (s, 2H); 7,20 (d, 7,3 Hz, 1 H); 7,28 (s, 1H).

### EXEMPLE 17

### {4-[4'-(1-Ethyl-1-hydroxy-propyl)-6,2',6'-trimethyl-biphenyl-3-yloxymethyl]-2-hydroxymethyl-phenyl}-methanol

### a) 5'-Hydroxy-2,6,2'-trimethyl-biphenyl-4-carboxylate de méthyle

De manière analogue à l'exemple 11(d), par réaction de 3,7 g (18,8 mmol) d' acide 4-methoxymethoxy-1-methyl-benzene-2-boronique (décrit à l'exemple 16c) avec 5,3 g (17 mmol) de 3,5-dimethyl-4-trifluoromethanesulfonyloxy-benzoate de méthyle, 17 ml d'une solution 2,0 M de carbonate de potassium et 1 g (0,82 mmol) de palladium tetrakis-triphenylphosphine, puis déprotection dans le méthanol. Le produit désiré est obtenu sous forme d'huile jaune (m = 3,0 g ; R = 65%).

### b) 5'-[3,4-Bis-(1-phenyl-methanoyloxymethyl)-benzyloxy]-2,6,2'-trimethyl-biphenyl-4-carboxylate de méthyle

De manière analogue à l'exemple 1(i) par réaction de 1,53 g (5,6 mmol) de 5'-hydroxy-2,6,2'-trimethyl-biphenyl-4-carboxylate de méthyle avec 2,73 g (6,2 mmol) de bromure de 3,4-bis-(benzoyloxymethyl)-benzyle et 980 mg (5,8 mmol) de carbonate de potassium. Le produit désiré est obtenu sous forme d'huile jaune (m = 3,5 g ; R = 98%).

### c) {4-[4'-(1-Ethyl-1-hydroxy-propyl)-6,2',6'-trimethyl-biphenyl-3-yloxymethyl]-2-hydroxymethyl-phenyl}-methanol

De manière analogue à l'exemple 1(j) par réaction de 3,5 g (5,8 mmol) 5'-[3,4-bis-(1-phenyl-methanoyloxymethyl)-benzyloxy]-2,6,2'-trimethyl-biphenyl-4-carboxylate de méthyle avec 15,3 ml (46 mmol) de bromure d'éthylmagnésium 3,0 M. Le produit désiré est obtenu sous forme de poudre blanche (PF = 128°C ; m = 1,45 g ; R = 56%).
**RMN** ^{**1**}**H** (DMSO): 0,72 (t, J = 7,3 Hz, 6H); 1,71-1,75 (m, 4H); 1,83 (s, 3H); 1,92 (s, 6H); 4,31 (s, 1H); 4,45 (s, 1H); 4,55-4,58 (m, 4H); 5,08 (s, 2H); 5,11-5,13 (m, 1H); 6,67 (s, 1H); 6,91-6,95 (dd, J₁ = 2,6 Hz, J₂ = 6,7 Hz, 1H); 7,13 (s, 2H); 7,25 (d, 7,5 Hz, 1H); 7,32 (d, J= 7,5 Hz, 1H); 7,42 (d, J = 7,5 Hz, 1H); 7,49 (s, 1H).

### EXEMPLE 18

### {4-[4'-(1-Ethyl-1-hydroxy-propyl)-6-methyl-biphenyl-3-yloxymethyl]-2-hydroxymethyl-phenyl}-methanol

### a) 5'-Hydroxy-2'-methyl-biphenyl-4-carboxylate d'éthyle

De manière analogue à l'exemple 11(d), par réaction de 3,7 g (18,8 mmol) d' acide 4-methoxymethoxy-1-methyl-benzene-2-boronique décrit à l'exemple 16c avec 4,7 g (17 mmol) de 4-iodobenzoate d'éthyle, 17 ml d'une solution 2,0 M de carbonate de potassium et 1 g (0,8 mmol) de palladium tetrakis-triphenylphosphine, puis déprotection dans l'éthanol. Le produit désiré est obtenu sous forme d'huile incolore (m = 3,07 g ; R = 71%).

### b) 5'-[3,4-Bis-(1-phenyl-methanoyloxymethyl)-benzyloxy]-2'-methyl-biphenyl-4-carboxylate d'éthyle

De manière analogue à l'exemple 1(i) par réaction de 3,07 g (12 mmol) 5'-hydroxy-2'-methyl-biphenyl-4-carboxylate d'éthyle avec 6,7g (15 mmol) de bromure de 3,4-bis-(benzoyloxymethyl)-benzyle et 2 mg (14 mmol) de carbonate de potassium. Le produit désiré est obtenu sous forme d'huile incolore (m = 5,7 g ; R = 77%).

### c) {4-[4'-(1-Ethyl-1-hydroxy-propyl)-6-methyl-biphenyl-3-yloxymethyl]-2-hydroxymethyl-phenyl}-methanol

De manière analogue à l'exemple 1(j) par réaction de 3,8 g (6,2 mmol) de 5'-[3,4-bis-(1-phenyl-methanoyloxymethyl)-benzyloxy]-2'-methyl-biphenyl-4-carboxylate d'éthyle avec 16,7 ml (50 mmol) de bromure d'éthylmagnésium 3,0 M. Le produit désiré est obtenu sous forme de poudre blanche (m = 1,26 mg ; R = 48%).
**RMN** ^{**1**}**H** (DMSO): 0,69 (t, J = 7,2 Hz, 6H); 1,74-1,77 (m, 4H); 2,15 (s, 3H); 4,52 (s, 1H); 4,54 (s, 1H); 4,56 (s, 1H); 5,05-5,10 (m, 2H); 5,11 (s, 2H); 6,85-6,92 (m, 2H); 7,18 (d, J = 8,5 Hz, 1H); 7,25-7,31 (m, 3H); 7,37-7,48 (m, 4H).

### EXEMPLE 19

### {4-[4'-(1-Ethyl-1-hydroxy-propyl)-2',6'-dimethyl-biphenyl-3-yloxymethyl]-2-hydroxymethyl-phenyl}-methanol

### a) 3'-Hydroxy-2,6-dimethyl-biphenyl-4-carboxylate de méthyle

De manière analogue à l'exemple 1(h) par réaction de 4,48 g (24 mmol) de l'acide 3-methoxymethoxyphenylboronique (décrit à l'exemple 1(g)) avec 7 g (22,4 mmol) de 3,5-dimethyl-4-trifluoromethanesulfonyloxy-benzoate de méthyle, 24 ml de carbonate de potassium 2 M et 1,29 g (1,1 mmol) de palladium tetrakis triphenylphosphine, puis déprotection dans le méthanol, le produit attendu est obtenu.

### b) 3'-[3,4-Bis-(1-phenyl-methanoyloxymethyl)-benzyloxy]-2,6-dimethyl-biphenyl-4-carboxylate de méthyle

De manière analogue à l'exemple 1(i) par réaction de 4,49 g (17,5 mmol) de 3'-hydroxy-2,6-dimethyl-biphenyl-4-carboxylate de méthyle avec 8,46 g (19 mmol) de bromure de 3,4-bis-(benzoyloxymethyl)-benzyle et 2,54 g (18 mmol) de carbonate de potassium. Le produit désiré est obtenu sous forme d'huile jaune (m = 7,4 g ; R = 69%).

### c) {4-[4'-(1-Ethyl-1-hydroxy-propyl)-2',6'-dimethyl-biphenyl-3-yloxymethyl]-2-hydroxymethyl-phenyl}-methanol

De manière analogue à l'exemple 1(j) par réaction de 5,4 g (8,8 mmol) 3'-[3,4-bis-(1-phenyl-methanoyloxymethyl)-benzyloxy]-2,6-dimethyl-biphenyl-4-carboxylate de méthyle avec 23,3 ml (70 mmol) de bromure d'éthylmagnésium 3,0 M. Le produit désiré est obtenu sous forme de poudre blanche (PF = 133°C ; m = 2,4 g ; R = 65%).
**RMN** ^{**1**}**H** (DMSO): 0,70 (t, J = 7,3 Hz, 6H); 1,67-1,75 (m, 4H); 1,98 (s, 6H); 4,48 (s, 1H); 4,54 (s, 1H); 4,56 (s, 1H); 5,11 (s, 2H); 5,14-5,17 (m, 2H); 6,69-6,77 (m, 2H); 7,00 (d, J = 8,2 Hz, 1H); 7,1 (s, 2H); 7,30-7,42 (m, 3H); 7,49 (s, 1H).

### EXEMPLE 20

### 1-{4-[3-(3,4-Bis-hydroxymethyl-benzyloxy)-phenyl]-thiophen-2-yl}-propan-1-ol

### a) 1-(4-Bromo-thiophen-2-yl)-propan-1-ol

10 g (52 mmol) de 4-bromo-thiophene-2-carbaldehyde sont dissous dans 200 ml d'éther éthylique et 20ml de THF. 26,6 ml (78 mmol) de bromure d'éthylmagnesium 3,0 M sont additionnés lentement. Après 2 heures à température ambiante, le milieu réactionnel est versé dans une solution saturée de chlorure d'ammonium. Après extraction, une huile jaune est obtenue (m = 11,5 g ; R = 99%).

### b) 1-(4-Bromo-thiophen-2-yl)-propan-1-one

11,5 g (52 mmol) de 1-(4-bromo-thiophen-2-yl)-propan-1-ol sont dissous dans 300 ml de dichlorométhane. 60 g (690 mmol) de dioxyde de manganèse sont additionnés, et le milieu réactionnel est agité pendant 14 heures, puis filtré. Le produit désiré est obtenu sous forme d'huile orangée (m = 11,4 g ; R = 99%).

### c) 1-[4-(3-Hydroxy-phenyl)-thiophen-2-yl]-propan-1-one

De manière analogue à l'exemple 1(h) par réaction de 4,6 g (25 mmol) d'acide 3-methoxymethoxyphenylboronique (décrit à l'exemple 1(g)) et 5 g (22,8 mmol) de 1-(4-bromo-thiophen-2-yl)-propan-1-one avec 22,8 ml de carbonate de potassium 2,0 M et 1,32 g de palladium tetrakis-triphenyphosphine, puis déprotection dans le méthanol. Le produit désiré est obtenu sous forme de solide blanc (PF = 112°C ; m = 3,87 g ; R = 73%).

### d) 1-{4-[3-(3,4-Bis-(1-phenyl-methanoyloxymethyl)-benzyloxy)-phenyl]-thiophen-2-yl}-propan-1-one

De manière analogue à l'exemple 1(i) par réaction de 3,8 g (16,3 mmol) 1-[4-(3-hydroxy-phenyl)-thiophen-2-yl]-propan-1-one avec 16,9 g (18 mmol) de bromure de 3,4-bis-(benzoyloxymethyl)-benzyle et 2,36 g (17 mmol) de carbonate de potassium. Le produit désiré est obtenu sous forme de cristaux blancs (PF = 117°C ; m = 9,1 g ; R = 95%).

### e) 1-{4-[3-(3,4-Bis-hydroxymethyl-benzyloxy)-phenyl]-thiophen-2-yl}-propan-1-ol

4,3 g (7,3 mmol) de 1-{4-(3-(3,4-bis-(1-phenyl-methanoyloxymethyl)-benzyloxy)-phenyl]-thiophen-2-yl}-propan-1-one sont dissous dans 10 ml de THF et additionné goutte à goutte sur une suspension de 1,1 g (29 mmol) d'hydrure double de lithium et d'aluminium, et le mélange est agité pendant 2 heures. Après traitement du milieu réactionnel par 1,1 ml d'eau, 1,1 ml de soude 15% et 3,3 ml d'eau, le milieu est filtré. Après purification par chromatographie sur gel de silice (éluant heptane 3 - acétate d'éthyle 7), le produit désiré est obtenu sous forme d'huile incolore (m = 773 mg ; R = 28%).
**RMN** ^{**1**}**H** (DMSO): 0,91 (t, J = 7,4 Hz, 6H); 1,68-1,77 (m, 2H); 4,53-4,58 (m, 4H); 4,69-4,73 (m, 1H); 5,08-5,14 (m, 1H); 5,16 (s, 2H); 5,58 (d, J = 5,4 Hz, 2H); 6,90-6,91 (m, 1H); 7,24-7,43 (m, 6H); 7,52 (s, 1H); 7,73 (s, 1H).

### EXEMPLE 21

### (4-{3-[4-(1-Ethyl-1-hydroxy-propyl)-thiophen-2-yl]-phenoxymethyl}-2-hydroxymethyl-phenyl)-methanol

### a) (4-{3-[4-(1-Ethyl-1-hydroxy-propyl)-thiophen-2-yl]-phenoxymethyl}-2-hydroxymethyl-phenyl)-methanol

De manière analogue à l'exemple 1(j) par réaction de 4,3 g (7,3 mmol) de 1-{4-[3-(3,4-Bis-(1-phenyl-methanoyloxymethyl)-benzyloxy)-phenyl]-thiophen-2-yl}-propan-1-one (décrit à l'exemple 20 (d)) avec 17 ml (51 mmol) de bromure d'éthylmagnésium 3,0 M. Le produit désiré est obtenu sous forme de solide blanc (PF 47°C ; m = 1,54 g ; R = 51%).
**RMN** ^{**1**}**H** (DMSO): 0.74 (t, J = 7,4 Hz, 6H); 1,68-1,78 (m, 4H); 4,54-4,58 (m, 4H); 4,62 (s, 1H); 5,08-5,13 (m, 1H); 5,16 (s, 2H); 6,92-6,96 (dd, J₁ = 2,6 Hz, J₂ = 6,2 Hz, 1H); 7,19 (d, J = 7,5 Hz, 2H); 7,27-7,30 (m, 4H); 7,44 (s, 1H); 7,52 (s, 1H).

### EXEMPLE 22

### {4-[4'-(1-Ethyl-1-hydroxy-propyl)-2'-methyl-biphenyl-3-ylmethoxy]-2-hydroxymethyl-phenyl}-methanol

### a) 1-(4-Bromo-3-methyl-phenyl)-propan-1-one

25 g (116 mmol) d'acide 4-bromo-3-méthyl-benzoique sont dissous dans 250 ml de dichlorométhane puis le mélange est refroidi à 0°C. 10,1 ml (116 mmol) de chlorure d'oxalyle sont ajoutés lentement, suivis de quelques gouttes de diméthylformamide et 16 ml (116 mmol) de triéthylamine. Après 30 minutes, 12,5g (128 mmol) de chlorhydrate de N,O diméthylhydroxylamine sont additionnés, suivis de 35,5 ml (255 mmol) de triéthylamine. Le milieu est agité 14 heures, puis traité par une solution de chlorure d'ammonuim et extrait avec du dichlorométhane. Le résidu brut est alors repris dans de l'éther éthylique et rincé avec une solution de soude 1 N, puis séché et concentré. L'intermédiaire amide obtenue est dissous dans 300 ml de THF anhydre et refroidi à -78°C. 40 ml (120 mmol) de bromure d'éthylmagnésium 3,0 M sont additionnés lentement, et le milieu réactionnel est ramené à 0°C, puis agité pendant 18 heures. Après le traitement usuel et chromatographie sur gel de silice (heptane 9 - acétate d'éthyle 1), le produit désiré est obtenu sous forme d'huile épaisse incolore (m = 17 g, R = 64 %).

### b) 2-(4-Bromo-3-methyl-phenyl)-2-ethyl-[1,3]dioxolane

16 g (70 mmol) de 1-(4-bromo-3-methyl-phenyl)-propan-1-one sont dissous dans 200 ml de toluène. 20 ml (350 mmol) d'éthylène glycol et 670 mg (3,5 mmol) d'acide paratoluènesulfonique sont ajoutés. Le montage est équipé d'un appareil de distillation de type Dean-Stark, et le milieu réactionnel est chauffé à 130°C pendant 16 heures. Le milieu est refroidi, puis versé dans une solution de bicarbonate de sodium, et extrait avec de l'éther éthylique. Le produit brut obtenu est sous forme d'huile incolore (m = 18,6 g ; R = 98%).

### c) 4'-(2-Ethyl-[1,3]dioxolan-2-yl)-2'-methyl-biphenyl-3-carbaldehyde

De manière analogue à l'exemple 1(h) par réaction de 6,6 g (44 mmol) d'acide 3-formylbenzeneboronique et 10 g (37 mmol) 2-(4-bromo-3-methyl-phenyl)-2-ethyl-[1,3]dioxolane avec 45 ml de carbonate de potassium 2,0 M et 2,14 g de palladium tetrakis-triphenyphosphine. Le produit désiré est obtenu sous forme d'huile épaisse (m = 9,1 g ; R = 84%).

### d) [4'-(2-Ethyl-[1,3]dioxolan-2-yl)-2'-methyl-biphenyl-3-yl]-methanol

De manière analogue à l'exemple 20(e), par réaction de 4,5 g (15,2 mmol) de 4'-(2-ethyl-[1,3]dioxolan-2-yl)-2'-methyl-biphenyl-3-carbaldehyde avec 760mg (20 mmol) d'hydrure double de lithium et d'aluminium. Le produit désiré est obtenu sous forme d'huile incolore (m = 4,4 g, R = 97%).

### e) 4-[4'-(2-Ethyl-[1,3]dioxolan-2-yl)-2'-methyl-biphenyl-3-ylmethoxy]-phthalate de diméthyle

4,4 g (14 mmol) de [4'-(2-éthyl-[1,3]dioxolan-2-yl)-2'-methyl-biphenyl-3-yl]-methanol sont dissous dans 120 ml de dichlorométhane et le mélange est refroidi à 0°C. 2,93 ml (16,8 mmol) de diisopropylethylamine sont ajoutés, suivis par une lente addition de 1,14 ml (14,7 mmol) de chlorure de méthanesulfonyle. Après 30 minutes d'agitation, le milieu réactionnel est versé dans une solution de chlorure d'ammonium. Après traitement usuel, le résidu obtenu est dissous dans 150 ml de 2-butanone. A cette solution sont successivement ajoutés: 4,2 g (28 mmol) d'iodure de sodium, 2,13 g (15,4 mmol) de carbonate de potassium et 3,23 g (15,4 mmol) de 4-hydroxyphthalate de diméthyle. Le milieu réactionnel est chauffé à 85°C pendant 18 heures, puis filtré et concentré. Le résidu obtenu est purifié par chromatographie sur gel de silice (éluant heptane 8 - acétate d'éthyle 2). Le produit désiré est obtenu sous forme d'huile incolore (m = 6,89 g, R = 100 %).

### f) 4-(2'-Methyl-4'-propionyl-biphenyl-3-ylmethoxy)-phthalate de diméthyle

6,8 g (14 mmol) de 4-[4'-(2-Ethyl-[1,3]dioxolan-2-yl)-2'-methyl-biphenyl-3-ylmethoxy]-phthalate de diméthyle sont dissous dans 200 ml de méthanol et 50 ml d'eau. 0,5 ml d'acide sulfurique sont ajoutés, et le milieu réactionnel est agité à température ambiante pendant 10 heures. Après traitement usuel, le produit désiré est obtenu sans purification sous forme d'huile incolore (m = 6,05 g ; R = 97%).

### g) 4-[4'-(1-Ethyl-1-hydroxy-propyl)-2'-methyl-biphenyl-3-ylmethoxy]-phthalate de diméthyle

5 g (11,2 mmol) de 4-(2'-methyl-4'-propionyl-biphenyl-3-ylmethoxy)-phthalate de diméthyle sont dissous dans 15 ml de dichlorométhane, et le mélange est refroidi à 0°C. 1,42 ml (11,2 mmol) de chlorure de triméthylsilane sont ajoutés, suivis par 13,4 ml (13,4 mmol) de diethylzinc 1,0 M. Le milieu est agité pendant 6 heures, puis traité par 10 ml de méthanol. 20 ml d'une solution 1N d'acide chlorhydrique est ajoutée, et l'agitation est poursuivie 1 heure. Après extraction dans du dichlorométhane et purification sur colonne de gel de silice (éluant heptane 6 - acétate d'éthyle 4), le produit désiré est obtenu sous forme d'huile incolore (m = 3,25 g ; R = 61%).

### h) {4-[4'-(1-Ethyl-1-hydroxy-propyl)-2'-methyl-biphenyl-3-ylmethoxy]-2-hydroxymethyl-phenyl}-methanol

De manière analogue à l'exemple 20(e) par réaction de 1,5 g (3,15 mmol) de 4-[4'-(1-éthyl-1-hydroxy-propyl)-2'-methyl-biphenyl-3-ylmethoxy]-phthalate de diméthyle avec 480 mg (12,6 mmol) d'hydrure double de lithium et d'aluminium. Le produit désiré est obtenu sous forme de solide blanc (PF 87°C ; m = 1,03 g ; R = 78%).
**RMN** ^{**1**}**H** (DMSO): 0,75 (t, J = 7,5 Hz, 6H); 1,75-1,79 (m, 4H); 2,27 (s, 3H); 4,47 (d, J = 5,2 Hz, 2H); 4,56 (d, J = 5,2 Hz, 2H); 4,58 (s, 1H); 4,97 (m, 1H); 5,13 (m, 1H); 5,19 (s, 2H); 6,90-6,91 (m, 1H); 7,12-7,16 (m, 2H); 7,26-7,35 (m, 4H); 7,46 (m, 3H).

### EXEMPLE 23

### 1-[3'-(3,4-Bis-hydroxymethyl-phenoxymethyl)-2-methyl-biphenyl-4-yl]-propen-1-ol

### a) 1-[3'-(3,4-Bis-hydroxymethyl-phenoxymethyl)-2-methyl-biphenyl-4-yl]-propan-1-ol

De manière analogue à l'exemple 20(e) par réaction de 900 mg (2 mmol) de 4-(2'-methyl-4'-propionyl-biphenyl-3-ylmethoxy)-phthalate de diméthyle décrit à l'exemple 22(f)) avec 300 mg (8 mmol) d'hydrure double de lithium et d'aluminium. Le produit désiré est obtenu sous forme de solide blanc (PF 78°C ; m = 737 mg ; R = 94%).
**RMN** ^{**1**}**H** (DMSO): 0,90 (t, J = 7,5 Hz, 3H); 1,64-1,68 (m, 2H); 2,26 (s, 3H); 3,39 (m, 1H); 4,46 (s, 1H); 4,47 (d, J = 5,2 Hz, 2H); 4,56 (d, J = 5,3 Hz, 2H); 4,98 (m, 1H); 5,12-5,16 (m, 1 H); 5,19 (s, 1H); 6,90-6,91 (m, 1H); 7,12 (m, 1H); 7,19-7,29 (m, 5H); 7,43-7,49 (m, 3H).

### EXEMPLE 24

### {5-[4'-(1-Ethyl-1-hydroxy-propyl)-3'-methyl-biphenyl-3-yloxymethyl]-2-hydroxymethyl-phenyl}-methanol

### a) 3'-Hydroxy-3-methyl-biphenyl-4-carboxylate de méthyle

De manière analogue à l'exemple 1(h) par réaction de 5,35 g (29 mmol) d'acide 3-methoxymethoxyphenylboronique (décrit à l'exemple 1(g)) et 5,7 g (26,7 mmol) d'acide 4-bromo-2-methylbenzoique avec 26,7 ml de carbonate de potassium 2,0 M et 1,54 g de palladium tetrakis-triphenyphosphine, puis déprotection dans le méthanol. Le produit désiré est obtenu sous forme d'huile incolore (m = 3,22 g ; R = 50%).

### b) 3'-[3,4-Bis-(1-phenyl-methanoyloxymethyl)-benzyloxy]-3-methyl-biphenyl-4-carboxylate de méthyle

De manière analogue à l'exemple 1(i) par réaction de 1,5 g (6,2 mmol) de 3'-hydroxy-3-methyl-biphenyl-4-carboxylate de méthyle avec 3 g (6,88 mmol) de bromure de 3,4-bis-(benzoyloxymethyl)-benzyle et 900 mg (6,4 mmol) de carbonate de potassium. Le produit désiré est obtenu sous forme d'huile jaune (m = 3,69 g ; R = 99%).

### c) {5-[4'-(1-Ethyl-1-hydroxy-propyl)-3'-methyl-biphenyl-3-yloxymethyl]-2-hydroxymethyl-phenyl}-methanol

De manière analogue à l'exemple 1(j) par réaction de 4,6 g (7,6 mmol) de 3'-[3,4-bis-(1-phenyl-methanoyloxymethyl)-benzyloxy]-3-methyl-biphenyl-4-carboxylate de méthyle avec 30,6 ml (61 mmol) de chlorure d'éthylmagnésium 2,0 M. Le produit désiré est obtenu sous forme de solide blanc (PF 76°C ; m = 1,32 g ; R = 42%).
**RMN** ^{**1**}**H** (DMSO): 0,69 (t, J = 7,3 Hz, 6H); 1,68-1,76 (m, 2H); 1,79-2,01 (m, 2H); 2,50 (s, 3H); 4,89 (s, 1H); 4,53-4,57 (m, 4H); 5,07-5,70 (m, 4H); 6,97 (d, J = 5 Hz, 1H); 7,21-7,43 (m, 7H); 7,51-7,53 (m, 2H).

### EXEMPLE 25

### {4-[4'-(1-Ethyl-1-hydroxy-propyl)-2'-methyl-biphenyl-4-yloxymethyl]-2-hydroxymethyl-phenyl}-methanol

### a) Acide 4-methoxymethoxyphenylboronique

De manière analogue à l'exemple 1(g), par réaction de 10 g (57,8 mmol) de 4-bromophenol avec 2,55 g (63,6 mmol) d'hydrure de sodium 60 et 4,83 mL (63,6 mmol) de chlorure de méthoxyméthyle, puis 25,4 mL (63,6 mmol) d'une solution 2,5 M de butyllithium et 15 mL (65 mmol) de borate de triisopropyle. Un solide marron est obtenu (PF = 65°C, m = 6,2 g ; R = 73%).

### b) 4'-Hydroxy-2-methyl-biphenyl-4-carboxylate de méthyle

De manière analogue à l'exemple 1(h) par réaction de 1,61 g (8,7 mmol) d'acide 4-methoxymethoxyphenylboronique et 1,8 g (9,7 mmol) d'acide 4-bromo-3-methylbenzoique avec 26,7 ml de carbonate de potassium 2,0 M et 1,54 g de palladium tetrakis-triphenyphosphine, puis déprotection - estérification dans le méthanol. Le produit désiré est obtenu sous forme d'huile incolore (m = 2,06 g ; R = 98%).

### c) 4'-[3,4-Bis-(1-phenyl-methanoyloxymethyl)-benzyloxy]-2-methyl-biphenyl-4-carboxylate de méthyle

De manière analogue à l'exemple 1(i) par réaction de 2,0 g (8,7 mmol) de 4'-hydroxy-2-methyl-biphenyl-4-carboxylate de méthyle avec 4,3 g (9,8 mmol) de bromure de 3,4-bis-(benzoyloxymethyl)-benzyle et 1,29 g (9,3 mmol) de carbonate de potassium. Le produit désiré est obtenu sous forme d'huile jaune (m = 4,88 g ; R = 91 %).

### d) {4-[4'-(1-Ethyl-1-hydroxy-propyl)-2'-methyl-biphenyl-4-yloxymethyl]-2-hydroxymethyl-phenyl}-methanol

De manière analogue à l'exemple 1(j) par réaction de 4,9 g (8 mmol) de 4'-[3,4-bis-(1-phenyl-methanoyloxymethyl)-benzyloxy]-2-methyl-biphenyl-4-carboxylate de méthyle avec 35 ml (70 mmol) de chlorure d'éthylmagnésium 2,0 M. Le produit désiré est obtenu sous forme de solide blanc (PF 97°C ; m = 426 mg ; R = 13%).
**RMN** ^{**1**}**H** (DMSO): 0,76 (t, J = 7,34 Hz, 6H); 1,75-1,84 (m, 4H); 2,31 (s, 3H); 4,56 (s, 1H); 4,60-4,64 (m, 4H); 5,14-5,24 (m, 4H); 7,10-7,18 (m, 3H); 7,25-7,50 (m, 7H); 7,53 (s, 1H).

### EXEMPLE 26

### {4-[2'-tert-Butyl-4'-(1-ethyl-1-hydroxy-propyl)-biphenyl-3-yloxymethyl]-2-hydroxymethyl-phenyl}-methanol

### a) 3'-Hydroxy-2-tert-butyl-biphenyl-4-carboxylate d'éthyle

De manière analogue à l'exemple 1(h) par réaction de 6,8 g (37 mmol) de 3-methoxymethoxyphenylboronique (décrit à l'exemple 1(g)) avec 11 g (31 mmol) de 3-*tert*-butyl-4-trifluoromethanesulfonyloxy-benzoate d'éthyle, 37 ml de carbonate de potassium 2 M et 1,8 g de palladium tetrakis triphenylphosphine, puis déprotection dans l'éthanol. Le produit désiré est obtenu sous forme de cristaux roses (PF = 118-120°C ; m = 5,3 g ; R = 57%).

### b) 3'-[3,4-Bis-(1-phenyl-methanoyloxymethyl)-benzyloxy]-2-tert-butyl-biphenyl-4-carboxylate d'éthyle

De manière analogue à l'exemple 1(i) par réaction de 5,3 g (17,7 mmol) de 3'-hydroxy-2-*tert*-butyl-biphenyl-4-carboxylate de méthyle avec 8,46 g (19 mmol) de bromure de 3,4-bis-(benzoyloxymethyl)-benzyle et 2,54 g (18 mmol) de carbonate de potassium. Le produit désiré est obtenu sous forme d'huile jaune (m = 11 g ; R = 94%).

### c) {4-[4'-(1-Ethyl-1-hydroxy-propyl)-2'-tert-butyl-biphenyl-3-yloxymethyl]-2-hydroxymethyl-phenyl}-methanol

De manière analogue à l'exemple 1(j) par réaction de 3 g (4,6 mmol) 3'-[3,4-bis-(1-phenyl-methanoyloxymethyl)-benzyloxy]-2-*tert*-butyl-biphenyl-4-carboxylate de méthyle avec 12 ml (36 mmol) de bromure d'éthylmagnésium 3,0 M. Le produit désiré est obtenu sous forme de poudre blanche (PF = 133°C ; m = 1,7 g ; R = 80 %).
**RMN** ^{**1**}**H** (CDCl₃): 0,83 (t, J = 7,4 Hz, 6H); 1,18 (s, 9H); 1,82-1,90 (m, 4H); 2,05 (bs, 3H); 4,74 (s, 4H); 5,07 (s, 2H); 6,88-6,97 (m, 4H); 7,12-7,24 (m, 2H); 7,37 (s, 2H); 7,42 (s, 1H); 7,52 (d, J = 1,7 Hz, 1H).

### EXEMPLE 27

### 1-[3'-(3,4-Bis-hydroxymethyl-benzyloxy)-2-methyl-biphenyl-4-yl]-2,2-dimethyl-propan-1-ol

### a) 4'-Bromo-2'-methyl-biphenyl-3-ol

De manière analogue à l'exemple 1(h) par réaction de 15,3 g (84 mmol) d'acide 3-methoxymethoxyphenylboronique (décrit à l'exemple 1(g)) et 25 g (84 mmol) de 2-bromo-5-iodotoluène avec 84 ml de carbonate de potassium 2,0 M et 4,8 g de palladium tetrakis-triphenyphosphine, puis déprotection dans le méthanol. Le produit désiré est obtenu sous forme d'huile incolore (m = 11,8 g ; R = 50%).

### b) 4-Bromomethyl-phthalate de diméthyle

36 g (176 mmol) de 4-hydroxyméthyl-phthalate de diméthyle sont dissous dans 300 mL de dichlorométhane et 70 g (211 mmol) de tetrabromométhane sont ajoutés. Une solution de 55,3 g (211 mmol) de triphenylphosphine dans 200 mL de dichlorométhane est alors additionnée lentement, et le mélange réactionnel est agite 3 h à température ambiante. Après traitement avec de l'eau et extraction dans du dichlorométhane, le résidu est purifié par chromatographie sur colonne de silice (éluant dichlorométhane). Le produit désiré est obtenu sous forme d'huile incolore ( m = 25 g ; R = 50%).

### c) 4-(4'-Bromo-2'-methyl-biphenyl-3-yloxymethyl)-phthalate de diméthyle

De manière analogue à l'exemple 1(i) par réaction de 11,8 g (44,8 mmol) de 4'-bromo-2'-methyl-biphenyl-3-ol avec 14,2 g (49 mmol) de 4-bromomethyl-phthalate de diméthyle et 6,5 g (47 mmol) de carbonate de potassium. Le produit désiré est obtenu sous forme d'huile jaune (m = 16,4 g ; R = 78%).

### d)[4-(4'-Bromo-2'-methyl-biphenyl-3-yloxymethyl)-2-hydroxymethyl-phenyl]-methanol

16,4 g (35 mmol) de 4-(4'-bromo-2'-methyl-biphenyl-3-yloxymethyl)-phthalate de diméthyle sont dissous dans 200 mL de THF anhydre, et 1,5 g (70 mmol) de borohydrure de lithium sont additionnés. Le milieu réactionnel est chauffé à reflux pendant 3 h, puis refroidi et versé sur de la glace, puis dilué avec une solution saturée de chlorure d'ammonium. Après extraction, le produit désiré est obtenu sous forme d'huile incolore (m =13,3 g ; R = 100%).

### e)3'-[3,4-Bis-(tert-butyl-dimethyl-silanyloxymethyl)-benzyloxy]-4-bromo-2-methyl-biphenyl

13,3 g (34,8 mmol) de [4-(4'-bromo-2'-methyl-biphenyl-3-yloxymethyl)-2-hydroxymethyl-phenyl]-methanol sont dissous dans 100 mL de DMF. 11,5 g (76 mmol) de *tert*-butyldimethylchlorosilane, puis 6,6 g (97 mmol) d'imidazole sont additionnés. Le milieu est agité pendant 10 h, puis dilué dans 400 ml d'éther éthylique et filtré. Le filtrat est alors traité avec une solution saturée de chlorure d'ammonium et rincé à l'eau. Après chromatographie sur colonne de gel de silice, le produit désiré est obtenu sous forme d'huile incolore (m = 20,4 g ; R = 91%).

### f)1-{3'-[3,4-Bis-(tert-butyl-dimethyl-silanyloxymethyl)-benzyloxy]-2-methyl-biphenyl-4-yl}-2,2-dimethyl-propan-1-ol

2 g (3 mmol) de 3'-[3,4-bis-(*tert*-butyl-dimethyl-silanyloxymethyl)-benzyloxy]-4-bromo-2-methyl-biphenyl sont dissous dans 30 mL de THF anhydre, et le mélange est refroidi à -78°C. 1,4 mL (3,5 mmol) de butyllithium 2,5 M sont additionnés lentement, et le mélange est maintenu à -78°C pendant 1 heure. 400 µL (3,7 mmol) de 2,2-dimethyl-propionaldehyde sont additionnés goutte à goutte, et le milieu est lentement ramené à température ambiante, puis traité suivant le traitement usuel. Le produit désiré est obtenu sous forme d'huile incolore ( m = 2 g; R = 100%).

### g) 1-[3'-(3,4-Bis-hydroxymethyl-benzyloxy)-2-methyl-biphenyl-4-yl]-2,2-dimethyl-propan-1-ol

2g (3 mmol) de 1-{3'-[3,4-bis-(*tert*-butyl-dimethyl-silanyloxymethyl)-benzyloxy]-2-methyl-biphenyl-4-yl}-2,2-dimethyl-propan-1-ol sont dissous dans 20 mL de THF et 7,7 mL (7,7 mmol) de tetrabutylammonium fluoride 1M sont additionnés goutte à goutte. Après 1 heure d'agitation à température ambiante puis le traitement usuel, le résidu obtenu est purifié par chromatographie sur gel de silice. Le produit désiré est obtenu sous forme d'huile incolore (m = 1,15 g ; R = 90%).
**RMN** ^{**1**}**H (CDCl**_{**3**}**):** 0,97 (s, 9H); 2,09 (bs, 2H); 2,25 (s, 3H); 4,40 (s, 1H); 4,75 (s, 2H); 4,76 (s, 2H); 5,09 (s, 2H); 6,91-6,96 (m, 3H); 7,17-7,19 (m, 3H); 7,28-7,44 (m, 4H).

### EXEMPLE 28

### 1-[3'-(3,4-Bis-hydroxymethyl-benzyloxy)-2-methyl-biphenyl-4-yl]-2-methyl-propan-1-ol

### a) 1-{3'-[3,4-Bis-(tert-butyl-dimethyl-silanyloxymethyl)-benzyloxy]-2-methyl-biphenyl-4-yl}-2-methyl-propan-1-ol

De manière analogue à l'exemple 27(f) par réaction de 2 g (3,1 mmol) de 3'-[3,4-bis-(*tert*-butyl-dimethyl-silanyloxymethyl)-benzyloxy]-4-bromo-2-methyl-biphenyl avec 1,4 mL (3,5 mmol) de butyllithium 2,5 M et 400 µL (37 mmol) de 2-methyl-propionaldehyde. Le produit désiré est obtenu sous forme d'huile incolore ( m = 1,1 g ; R = 56%).

### b) 1-[3'-(3,4-Bis-hydroxymethyl-benzyloxy)-2-methyl-biphenyl-4-yl]-2-methyl-propan-1-ol

De manière analogue à l'exemple 27(g) par réaction de 1,1 g (1,7 mmol) de 1-{3'-[3,4-Bis-(*tert*-butyl-dimethyl-silanyloxymethyl)-benzyloxy]-2-methyl-biphenyl-4-yl}-2-methyl-propan-1-ol avec 3,8 mL (3,8 mmol) de fluorure de tetrabutylammonium 1.0 M. Le produit désiré est obtenu sous forme d'huile incolore (m = 460 mg ; R = 65%).
**RMN** ^{**1**}**H (CDCl**_{**3**}**):** 0,85 (d, J = 6,7 Hz, 3H); 1,03 (d, J = 6,6 Hz, 3H); 1,99 (m, 1H); 2,25 (s, 3H); 4,35 (d, J = 6,9 Hz, 1H); 4,73 (s, 2H); 4,74 (s, 2H); 5,08 (s, 2H); 6,92-6,95 (m, 3H); 7,14-7,20 (m, 3H); 7,28-7,43 (m, 4H).

### EXEMPLE 29

### {2-Hydroxymethyl-4-[methyl-(trifluoro-hydroxy-trifluoromethyl-ethyl)-biphenyl-3-yloxymethyl]-phenyl}-methanol

### a) 2-{3'-[3,4-Bis-(tert-butyl-dimethyl-silanyloxymethyl)-benzyloxy]-2-methyl-biphenyl-4-yl}-1,1,1,3,3,3-hexafluoro-propan-2-ol

De manière analogue à l'exemple 27(f) par réaction de 4 g (6,2 mmol) de 3'-[3,4-bis-(*tert*-butyl-dimethyl-silanyloxymethyl)-benzyloxy]-4-bromo-2-methyl-biphenyl avec 2,7 mL (6,8 mmol) de butyllithium 2,5 M et un excès d'hexafluoroacétone. Le produit désiré est obtenu sous forme d'huile incolore ( m = 2,35 g ; R = 52%).

### b) {2-Hydroxymethyl-4-[methyl-(trifluoro-hydroxy-trifluoromethyl-ethyl)-biphenyl-3-yloxymethyl]-phenyl}-methanol

De manière analogue à l'exemple 27(g) par réaction de 2,3 g (3,2 mmol) de 2-{3'-[3,4-bis-(*tert*-butyl-dimethyl-silanyloxymethyl)-benzyloxy]-2-methyl-biphenyl-4-yl}-1,1,1,3,3,3-hexafluoro-propan-2-ol avec 7 mL (7 mmol) de fluorure de tetrabutylammonium 1,0 M. Le produit désiré est obtenu sous forme d'huile incolore ( m = 1,2 g ; R = 75%).
**RMN** ^{**1**}**H (CDCl**_{**3**}**):** 2,28 (s, 3H); 2,37 (bs, 2H); 4,74 (s, 4H); 5,08 (s, 2H); 6,91-6,99 (m, 3H); 7,26-7,43 (m, 5H); 7,53-7,59 (m, 3H).

### EXEMPLE 30 : EXEMPLES DE FORMULATION

### 1) VOIE ORALE

### (a) On prépare la composition suivante sous la forme d'un comprimé de 0,2 g

| | |
|---|---|
| Composé de l'exemple 1 | 0,005 g |
| Amidon prégélatinisé | 0,065 g |
| Cellulose microcristalline | 0,075 g |
| Lactose | 0,050 g |
| Stéarate de magnésium | 0,005 g |

Pour le traitement de l'ichtyose, on administre à un individu adulte 1 à 3 comprimés par jour pendant 1 à 12 mois selon la gravité du cas traité.

### (b) On prépare une suspension buvable, destinée à être conditionnée en ampoules de 5 ml

| | |
|---|---|
| Composé de l'exemple 2 | 0,050 mg |
| Glycérine | 0,500 g |
| Sorbitol à 70 % | 0,500 g |
| Saccharinate de sodium | 0,010 g |
| Parahydroxybenzoate de méthyle | 0,040 g |
| Arôme q.s. | |
| Eau purifiée q.s.p | 5 ml |

Pour le traitement de l'acné, on administre à un individu adulte 1 ampoule par jour pendant 1 à 12 mois selon la gravité du cas traité.

### (c) On prépare la formulation suivante destinée à être conditionnée en gélules :

| | |
|---|---|
| Composé de l'exemple 4 | 0,001 mg |
| Amidon de maïs | 0,060 g |
| Lactose q.s.p. | 0,300 g |

Les gélules utilisées sont constituées de gélatine, d'oxyde de titane et d'un conservateur.

Dans le traitement du psoriasis, on administre à un individu adulte, 1 gélule par jour pendant 1 à 12 mois.

### (d) On prépare la formulation suivante destinée à être conditionnée en gélules :

| | |
|---|---|
| Composé de l'exemple 5 | 0,02 mg |
| Cyclosporine | 0,050 g |
| Amidon de maïs | 0,060 g |
| Lactose q.s.p. | 0,300 g |

Les gélules utilisées sont constituées de gélatine, d'oxyde de titane et d'un conservateur.

Dans le traitement du psoriasis, on administre à un individu adulte, 1 gélule par jour pendant 1 à 12 mois.

### 2) VOIE TOPIQUE

### (a) On prépare la crème Eau-dans l'Huile non ionique suivante :

| | |
|---|---|
| Composé de l'exemple 9 | 0,100 g |
| Mélange d'alcools de lanoline émulsifs, de cires et d'huiles raffinés, vendu par la Société BDF sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p. | 100,000 g |

Cette crème est appliquée sur une peau psoriatique 1 à 2 fois par jour pendant 1 à 12 mois.

### (b) On prépare un gel en réalisant la formulation suivante :

| | |
|---|---|
| Composé de l'exemple 28 | 0,001 g |
| Erythromycine base | 4,000 g |
| Butylhydroxytoluène | 0,050 g |
| Hydroxypropylcellulose vendue par la société Hercules sous le nom de "KLUCEL HF" | 2,000 g |
| Ethanol (à 95°) q.s.p. | 100,000 g |

Ce gel est appliqué sur une peau atteinte de dermatose ou une peau acnéique 1 à 3 fois par jour pendant 6 à 12 semaines selon la gravité du cas traité.

### (c) On prépare une lotion antiséborrhéique en procédant au mélange des ingrédients suivants :

| | |
|---|---|
| Composé de l'exemple 12 | 0,030 g |
| Propylène glycol | 5,000 g |
| Butylhydroxytoluène | 0,100 g |
| Ethanol (à 95°) q.s.p. | 100,000 g |

Cette lotion est appliquée deux fois par jour sur un cuir chevelu séborrhéique et on constate une amélioration significative dans un délai compris entre 2 et 6 semaines.

### (d) On prépare une composition cosmétique contre les effets néfastes du soleil en procédant au mélange des ingrédients suivants :

| | |
|---|---|
| Composé de l'exemple 7 | 1,000 g |
| Benzylidène camphre | 4,000 g |
| Triglycérides d'acides gras | 31,000 g |
| Monostéarate de glycérol | 6,000 g |
| Acide stéarique | 2,000 g |
| Alcool cétylique | 1,200 g |
| Lanoline | 4,000 g |
| Conservateurs | 0,300 g |
| Propylène glycol | 2,000 g |
| Triéthanolamine | 0,500 g |
| Parfum | 0,400 g |
| Eau déminéralisée q.s.p. | 100,000 g |

Cette composition est appliquée quotidiennement, elle permet de lutter contre le vieillissement photoinduit.

### (e) On prépare la crème Huile dans l'Eau non ionique suivante :

| | |
|---|---|
| Composé de l'exemple 23 | 0,500 g |
| Acide rétinoique | 0,020 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile q.s.p. | 100,000 g |

Cette crème est appliquée sur une peau psoriatique 1 à 2 fois par jour pendant 30 jours pour traitement d'attaque et indéfiniment pour entretien.

### (f) On prépare un gel topique en procédant au mélange des ingrédients suivants :

| | |
|---|---|
| Composé de l'exemple 19 | 0,050 g |
| Ethanol | 43,000 g |
| α-tocophérol | 0,050 g |
| Polymère carboxyvinylique vendu sous la dénomination | |
| "Carbopol 941" par la société "Goodrich" | 0,500 g |
| Triéthanolamine en solution aqueuse à 20 % en poids | 3,800 g |
| Eau 9,300 g | |
| Propylène glycol qsp. | 100,000 g |

Ce gel est appliqué dans le traitement de l'acné 1 à 3 fois par jour pendant 6 à 12 semaines selon la gravité du cas traité.

### (g) On prépare une lotion capillaire anti-chute et pour la repousse des cheveux en procédant au mélange des ingrédients suivants :

| | |
|---|---|
| Composé de l'exemple 13 | 0,05 g |
| Composé vendu sous la dénomination "Minoxidil" | 1,00 g |
| Propylène glycol | 20,00g |
| Ethanol | 34,92 g |
| Polyéthylèneglycol (masse moléculaire = 400) | 40,00 g |
| Butylhydroxyanisole | 0,01 g |
| Butylhydroxytoluène | 0,02 g |
| Eau qsp | 100,00 g |

On applique cette lotion 1 à 2 fois par jour pendant 3 mois sur un cuir chevelu ayant subi une chute de cheveu et indéfiniment pour traitement d'entretien.

### (h) On prépare une crème anti-acnéique en procédant au mélange des ingrédients suivants :

| | |
|---|---|
| Composé de l'exemple 5 | 0,050 g |
| Acide rétinoïque. | 0,010 g |
| Mélange de stéarates de glycérol et de polyéthylène glycol (75 moles) vendu sous le nom de "Gelot 64" ............ par la société "GATTEFOSSE" | 15,000 g |
| Huile de noyau polyoxyéthylénée à 6 moles d'oxyde .....d'éthylène vendue sous le nom de "Labrafil M2130 CS" par la société "GATTEFOSSE" | 8,000 g |
| Perhydrosqualène | 10,000 g |
| Conservateurs | qs |
| Polyéthylèneglycol (masse moléculaire = 400) | 8,000 g |
| Sel disodique de l'acide éthylène-diamine tétracétique | 0,050 g |
| Eau purifiée qsp | 100,000 g |

Cette crème est appliquée sur une peau atteinte de dermatose ou une peau acnéique 1 à 3 fois par jour pendant 6 à 12 semaines.

### (i) On prépare une crème huile dans l'eau en réalisant la formulation suivante :

| | |
|---|---|
| Composé de l'exemple 14 | 0,020 g |
| 17-valérate de bétamethasone | 0,050 g |
| S-carboxyméthyl cystéine | 3,000 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de "Tween 20" par la société "ATLAS" | 1,800 g |
| Mélange de mono et distéarate de glycérol vendu sous la dénomination de "Géléol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène ..... | 18,000 g |
| Mélange de triglycérides caprylique-caprique vendu sous la dénomination de "Miglyol 812" par la société "DYNAMIT NOBEL" | 4,000 g |
| Triéthanolamine (99 % en poids) | 2,500 g |
| Eau q.s.p. | 100,000 g |

Cette crème est appliquée 2 fois par jour sur une peau atteinte de dermatose inflammatoire pendant 30 jours.

### (j) On prépare la crème de type huile dans l'eau suivante :

| | |
|---|---|
| Acide lactique | 5,000 g |
| Composé de l'exemple 8 | 0,020 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de Tween 20" par la société "ATLAS" | 1,800 g |
| Mélange de mono et distéarate de glycérol vendu sous la dénomination de "Geleol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" ..... par la société "DYNAMIT NOBEL" | 4,000 g |
| Eau q.s.p. | 100,000 g |

Cette crème est appliquée 1 fois par jour, elle aide à lutter contre le vieillissement qu'il soit photoinduit ou chronologique.

### (k) On prépare l'onguent anhydre suivant :

| | |
|---|---|
| Composé de l'exemple 25 | 5,000 g |
| Huile de vaseline | 50,00 g |
| butylhydrotoluène | 0,050 g |
| vaseline blanche | qs 100 g |

Cet onguent est appliqué 2 fois par jour sur une peau atteinte de dermatose squameuse pendant 30 jours.

### 3) VOIE INTRALESIONNELLE

### (a) On prépare la composition suivante :

| | |
|---|---|
| Composé de l'exemple 16 | 0,002 g |
| Oléate d'éthyle | qs 10 g |

Dans le traitement du mélanome malin, on injecte la composition à un individu adulte à une fréquence de 1 à 7 fois par semaine pendant 1 à 12 mois.

### (b) On prépare la composition suivante :

| | |
|---|---|
| Composé de l'exemple 10 | 0,050 g |
| Huile d'olive | qs 2 g |

Dans le traitement du carcinome basocellulaire, on injecte la composition à un individu adulte à une fréquence de 1 à 7 fois par semaine pendant 1 à 12 mois.

### (c) On prépare la composition suivante :

| | |
|---|---|
| Composé de l'exemple 6 | 0,1 mg |
| Huile de sésame | qs 2 g |

Dans le traitement du carcinome spinocellulaire, on injecte la composition à un individu adulte à une fréquence de 1 à 7 fois par semaine pendant 1 à 12 mois.

### (d) On prépare la composition suivante :

| | |
|---|---|
| Composé de l'exemple 2 | 0,001mg |
| Benzoate de méthyle | qs 10 g |

Dans le traitement du carcinome du colon, on injecte la composition à un individu adulte à une fréquence de 1 à 7 fois par semaine pendant 1 à 12 mois.

### 4) VOIE INTRAVEINEUSE

### (a) On prépare l'émulsion lipidique injectable suivante :

| | |
|---|---|
| Composé de l'exemple 7 | 0,001 mg |
| Huile de soja | 10,000 g |
| Phospholipide d'oeuf | 1,200 g |
| Glycérine | 2,500 g |
| Eau pour injectable q.s.p. | 100,000 g |

Dans le traitement du psoriasis, on injecte la composition à un individu adulte à une fréquence de 1 à 7 fois par semaine pendant 1 à 12 mois.

### (b) On prépare l'émulsion lipidique injectable suivante :

| | |
|---|---|
| Composé de l'exemple 3 | 0,010 g |
| Huile de coton | 10,000 g |
| Lécithine de soja | 0,750 g |
| Sorbitol | 5,000 g |
| DL, α-Tocophérol | 0,100 g |
| Eau pour injectable q.s.p. | 100,000 g |

Dans le traitement de l'ichtyose, on injecte la composition à un individu adulte à une fréquence de 1 à 7 fois par semaine pendant 1 à 12 mois.

### (c) On prépare l'émulsion lipidique injectable suivante :

| | |
|---|---|
| Composé de l'exemple 21 | 0,001 g |
| Huile de soja | 15.000 g |
| Monoglycérides acétylés | 10,000 g |
| Pluronic F-108 | 1,000 g |
| Glycérol | 2,500 g |
| Eau pour injectable q.s.p. | 100,000 g |

Dans le traitement de la leucémie, on injecte la composition à un individu adulte à une fréquence de 1 à 7 fois par semaine pendant 1 à 12 mois.

### (d) On prépare la composition micelle mixte suivante :

| | |
|---|---|
| Composé de l'exemple 29 | 0,001 g |
| Lécithine | 16,930 g |
| Acide glycocholique | 8,850 g |
| Eau pour injectable q.s.p. | 100,000 g |

Dans le traitement du mélanome malin, on injecte la composition à un individu adulte à une fréquence de 1 à 7 fois par semaine pendant 1 à 12 mois.

### (e) On prépare la composition de cyclodextrine suivante :

| | |
|---|---|
| Composé de l'exemple 11 | 0, 1 mg |
| βcyclodextrine | 0,100 g |
| Eau pour injectable q.s.p. | 10,000g |

Dans le traitement du rejet de greffe, on injecte la composition à un individu adulte à une fréquence de 1 à 7 fois par semaine pendant 1 à 12 mois.

### (f) On prépare la composition de cyclodextrine suivante :

| | |
|---|---|
| Composé de l'exemple 4 | 0,010 g |
| 2-hydroxypropylβcyclodextrine | 0,100 g |
| Eau pour injectable q.s.p. | 10,000 g |

Dans le traitement du cancer du rein, on injecte la composition à un individu adulte à une fréquence de 1 à 7 fois par semaine pendant 1 à 12 mois.

### EXEMPLE 31 : Exemple de test d'évaluation de l'activité biologique des composés de l'invention

L'activité agoniste VDR a été testée sur la lignée cellulaire HeLa, par cotransfection d'un vecteur d'expression du récepteur VDR humain et du plasmide rapporteur p240Hase-CAT qui contient la région - 1399 à +76 du promoteur de la 24-hydroxylase de rat, clonée en amont de la phase codante du gène de la chloramphénicol-acétyl-transférase (CAT). 18 heures après cotransfection, le produit test est ajouté dans le milieu. Après 18 heures de traitement, le dosage de l'activité CAT des lysats cellulaires est effectuée par un test Elisa. Les résultats sont exprimés en pourcentage de l'effet normalement observé avec 10⁻⁷ M de calcitriol.

L'activité agoniste a été caractérisée dans ce système de cotransfection, par la détermination de la dose nécessaire pour atteindre 50 % de l'activité maximale du produit (AC50).

| **Composé testé** | AC 50 (nM) |
|---|---|
| **Exemple 3** | 470 |
| **Exemple 10** | 2045 |
| **Exemple 11** | 291 |
| **Exemple 16** | 46 |
| **Exemple 17** | 194 |
| **Exemple 18** | 133 |
| **Exemple 22** | 451 |

## Revendications

1. Composés, **caractérisés par le fait qu'**ils répondent à la formule générale (I) suivante : 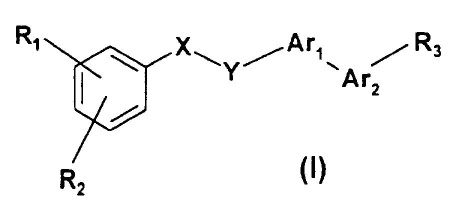 dans laquelle:
- R₁ représente un atome d' hydrogène, un radical CH₃ ou un radical -(CH₂)ᵣ-OR₄,
- R₂ représente un radical -(CH₂)ₛ-OR₅
r, s, R₄ et R₅ ayant les significations données ci-après,
- X-Y représente une liaison choisie parmi les liaisons de formules (a) à (d) suivantes pouvant être lues de gauche à droite ou inversement: 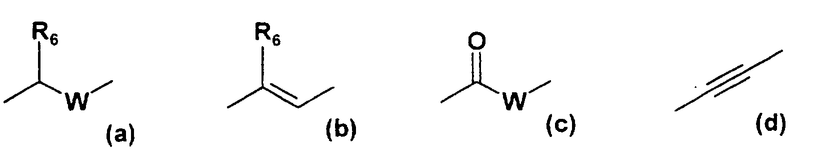 R₆ et W ayant les significations données ci-après,
- Ar₁ représente un cycle de formules (e) à (i) suivantes: 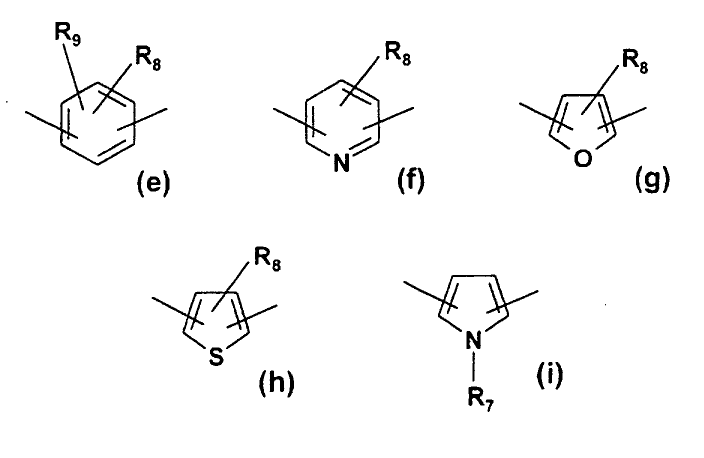 R₇, R₈ et R₉ ayant les significations données ci-après,
- Ar₂ représente un cycle de formules (j) à (n) suivantes: 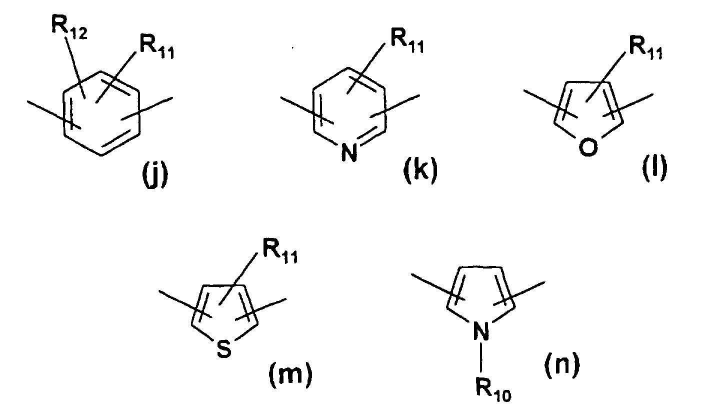 R₁₀, R₁₁ et R₁₂ ayant les significations données ci-après,
- R₃ représente un radical de formule: 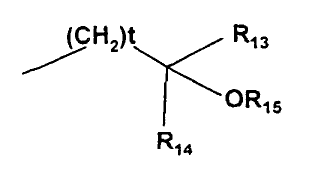 t, R₁₃, R₁₄ et R₁₅ ayant les significations données ci-après,
- r et s identiques ou différents étant 1 ou 2,
- R₄ et R₅ identiques ou différents représentent un atome d'hydrogène, un radical acétyle, un radical benzoyle, un radical triméthylsilyle, un radical tertiobutyldiméthylsilyle ou un radical tétrahydropyranyle,
- R₆ représente un atome d'hydrogène ou un radical C₁-C₆ alkyle ,
- W représente un atome d'oxygène, de soufre, un radical CH₂ ou un radical NH pouvant être substitué par un radical C₁-C₆ alkyle,
- R₇ et R₁₀ identiques ou différents représentent un atome d'hydrogène ou un radical C₁-C₆ alkyle,
- R₈, R₉, R₁₁ et R₁₂ identiques ou différents représentent un atome d'hydrogène, un radical C₁-C₆ alkyle, un atome d'halogène, un radical -OR₁₆, un radical C₂-C₅ polyéther, un radical CF₃, un radical NO₂ ou un radical amino pouvant être substitué par un ou deux radicaux C₁-C₆ alkyles ,
R₁₆ ayant les significations données ci-après,
- t étant 0 ou 1
- R₁₃ et R₁₄ identiques ou différents représentent un atome d'hydrogène, un radical C₁-C₆ alkyle, un radical C₃-C₆ cycloalkyle, un radical CF₃ ou un radical C₂F₅,
- R₁₅ représente un atome d'hydrogène, un radical acétyle, un radical triméthylsilyle, un radical tertiobutyldiméthylsilyle ou un radical tétrahydropyranyle,
- R₁₆ représente un atome d'hydrogène ou un radical C₁-C₆ alkyle, et les isomères optiques et géométriques desdits composés de formule (I) ainsi que leurs sels

2. Composés selon la revendication 1, **caractérisés par le fait qu'**ils se présentent sous forme de sels d'un acide minéral ou organique, en particulier l'acide chlorhydrique, sulfurique, acétique, fumarique, hémisuccinique, maléique ou mandélique.

3. Composés selon l'une des revendications 1 ou 2, **caractérisés par le fait que** les radicaux alkyles sont choisis parmi les radicaux méthyle, éthyle, isopropyle, tertiobutyle et hexyle.

4. Composés selon l'une des revendications précédentes, **caractérisés en ce que** le radical cycloalkyle correspond à un radical cyclopropyle cyclopentyle ou cyclohexyle.

5. Composés selon l'une des revendications précédentes, **caractérisés en ce que** l'atome d'halogène correspond à un atome de fluor, de chlore ou de brome.

6. Composés selon l'une quelconque des revendications précédentes, **caractérisés en ce que** le radical polyéther correspond à un radical méthoxyméthoxy, méthoxyéthoxy ou méthoxyéthoxyméthoxy.

7. Composés selon la revendication 1, **caractérisés par le fait qu'**ils sont pris, seuls ou en mélanges, dans le groupe constitué par :
{5-[4'-(1-Ethyl-1-hydroxy-propyl)-biphenyl-3-yloxymethyl]-2-hydroxymethyl-phenyl}-methanol
{2-Hydroxymethyl-5-[4'-(1-hydroxy-1-methyl-ethyl)-biphenyl-3-yloxymethyl]-phenyl}-methanol
{5-[4'-(1-Ethyl-1-hydroxy-propyl)-2'-methyl-biphenyl-3-yloxymethyl]-2-hydroxymethyl-phenyl}-methanol
{2-Hydroxymethyl-5-[4'-(1-hydroxy-1-methyl-ethyl)-2'-methyl-biphenyl-3-yloxymethyl]-phenyl}-methanol
(5-{2-[3'-(1-Ethyl-1-hydroxy-propyl)-biphenyl-3-yl]-ethyl}-2-hydroxymethyl-phenyl)-methanol
{2-Hydroxymethyl-5-[3'-(1-hydroxy-1-methyl-ethyl)-biphenyl-3-yloxymethyl]-phenyl}-methanol
{{5-[4'-(2-Ethyl-2-hydroxy-butyl)-biphenyl-3-yloxymethyl]-2-hydroxymethyl-phenyl}-methanol
{5-[3'-(2-Ethyl-2-hydroxy-butyl)-biphenyl-3-yloxymethyl]-2-hydroxymethyl-phenyl}-methanol
1-[3'-(3,4-Bis-hydroxymethyl-benzyloxy)-biphenyl-3-yl]-2-methyl-propan-2-ol
{4-[4'-(1-Ethyl-1-hydroxy-propyl)-2'-methyl-biphenyl-3-ylsulfanylmethyl]-2-hydroxymethyl-phenyl}-methanol
{4-[4'-(1-Ethyl-1-hydroxy-propyl)-2,2'-dimethyl-biphenyl-3-yloxymethyl]-2-hydroxymethyl-phenyl}-methanol
{2-Hydroxymethyl-4-[4'-(1-hydroxy-1-propyl-butyl)-2,2'-dimethyl-biphenyl-3-yloxymethyl]-phenyl}-methanol
{4-[4'-(1-Ethyl-1-hydroxy-propyl)-2-methyl-biphenyl-3-yloxymethyl]-2-hydroxymethyl-phenyl}-methanol
{4-[4'-(1-Ethyl-1-hydroxy-propyl)-2,2',6'-trimethyl-biphenyl-3-yloxymethyl]-2-hydroxymethyl-phenyl}-methanol
(4-{3-[5-(1-Ethyl-1-hydroxy-propyl)-pyridin-2-yl]-phenoxymethyl}-2-hydroxymethyl-phenyl)-methanol
{4-[4'-(1-Ethyl-1-hydroxy-propyl)-6,2'-dimethyl-biphenyl-3-yloxymethyl]-2-hydroxymethyl-phenyl}-methanol
{4-[4'-(1-Ethyl-1-hydroxy-propyl)-6,2',6'-trimethyl-biphenyl-3-yloxymethyl]-2-hydroxymethyl-phenyl}-methanol
{4-[4'-(1-Ethyl-1-hydroxy-propyl)-6-methyl-biphenyl-3-yloxymethyl]-2-hydroxymethyl-phenyl}-methanol
{4-[4'-(1-Ethyl-1-hydroxy-propyl)-2',6'-dimethyl-biphenyl-3-yloxymethyl]-2-hydroxymethyl-phenyl}-methanol
1-{4-[3-(3,4-Bis-hydroxymethyl-benzyloxy)-phenyl]-thiophen-2-yl}-propan-1-ol
(4-{3-[4-(1-Ethyl-1-hydroxy-propyl)-thiophen-2-yl]-phenoxymethyl}-2-hydroxymethyl-phenyl)-methanol
{4-[4'-(1-Ethyl-1-hydroxy-propyl)-2'-methyl-biphenyl-3-ylmethoxy]-2-hydroxymethyl-phenyl}-methanol
1-[3'-(3,4-Bis-hydroxymethyl-phenoxymethyl)-2-methyl-biphenyl-4-yl]-propan-1-ol
{4-[4'-(1-Ethyl-1-hydroxy-propyl)-3'-methyl-biphenyl-3-yloxymethyl]-2-hydroxymethyl-phenyl}-methanol
{5-[4'-(1-Ethyl-1-hydroxy-propyl)-2'-methyl-biphenyl-4-yloxymethyl]-2-hydroxymethyl-phenyl}-methanol
{4-[2'-tert-Butyl-4'-(1-ethyl-1-hydroxy-propyl)-biphenyl-3-yloxymethyl]-2-hydroxymethyl-phenyl}-methanol
1-[3'-(3,4-Bis-hydroxymethyl-benzyloxy)-2-methyl-biphenyl-4-yl]-2,2-dimethyl-propan-1-ol
1-[3'-(3,4-Bis-hydroxymethyl-benzyloxy)-2-methyl-biphenyl-4-yl]-2-methyl-propan-1-ol
{2-Hydroxymethyl-4-[methyl-(trifluoro-hydroxy-trifluoromethyl-ethyl)-biphenyl-3-yloxymethyl]-phenyl}-methanol
[5-(2- {5-[4-(1-Ethyl-1-hydroxy-propyl)-2-methyl-phenyl]-thiophen-2-yl}-ethyl)-2-hydroxymethyl-phenyl]-methanol
(5-{5-[4-(1-Ethyl-1-hydroxy-propyl)-2-methyl-phenyl]-thiophen-2-ylmethoxy}-2-hydroxymethyl-phenyl)-methanol
[5-(2-{5-[2-Ethyl-4-(1-ethyl-1-hydroxy-propyl)-phenyl]-thiophen-2-yl}-ethyl)-2-hydroxymethyl-phenyl]-methanol
(5-{5-[2-Ethyl-4-(1-ethyl-1-hydroxy-propyl)-phenyl]-thiophen-2-ylmethoxy}-2-hydroxymethyl-phenyl)-methanol
[5-(2-{5-[4-(1-Ethyl-1-hydroxy-propyl)-2-methyl-phenyl]-4-methyl-thiophen-2-yl}-ethyl)-2-hydroxymethyl-phenyl]-methanol
(5-{5-[4-(1-Ethyl-1-hydroxy-propyl)-2-methyl-phenyl]-4-methyl-thiophen-2-ylmethoxy}-2-hydroxymethyl-phenyl)-methanol
[2-Hydroxymethyl-5-(2-{5-[methyl-(trifluoro-hydroxy-trifluoromethyl-ethyl)-phenyl]-thiophen-2-yl}-ethyl)-phenyl]-methanol
(2-Hydroxymethyl-5-{5-[methyl-(trifluoro-hydroxy-trifluoromethyl-ethyl)-phenyl]-thiophen-2-ylmethoxy}-phenyl)-methanol
[5-(2-{5-[Ethyl-(trifluoro-hydroxy-trifluoromethyl-ethyl)-phenyl]-thiophen-2-yl}-ethyl)-2-hydroxymethyl-phenyl]-methanol
(5-{5-[Ethyl-(trifluoro-hydroxy-trifluoromethyl-ethyl)-phenyl]-thiophen-2-ylmethoxy}-2-hydroxymethyl-phenyl)-methanol
[2-Hydroxymethyl-5-(2-{methyl-[methyl-(trifluoro-hydroxy-trifluoromethyl-ethyl)-phenyl]-thiophen-2-yl}-ethyl)-phenyl]-methanol
(2-Hydroxymethyl-5-{methyl-[methyl-(trifluoro-hydroxy-trifluoromethyl-ethyl)-phenyl]-thiophen-2-ylmethoxy}-phenyl)-methanol
[5-(2-{5-[4-(-Ethyl-1-hydroxy-propyl)-2-methyl-phenyl]-thiophen-3-yl}-ethyl)-2-hydroxymethyl-phenyl]-methanol
(5-{5-[4-(1-Ethyl-1-hydroxy-propyl)-2-methyl-phenyl]-thiophen-3-ylmethoxy}-2-hydroxymethyl-phenyl)-methanol
[5-(2-{5-[2-Ethyl-4-(1-ethyl-1-hydroxy-propyl)-phenyl]-thiophen-3-yl}-ethyl)-2-hydroxymethyl-phenyl]-methanol
(5- {5-[2-Ethyl-4-(1-ethyl-1-hydroxy-propyl)-phenyl]-thiophen-3-ylmethoxy}-2-hydroxymethyl-phenyl)-methanol
[2-Hydroxymethyl-5-(2-{5-[methyl-(trifluoro-hydroxy-trifluoromethyl-ethyl)-phenyl]-thiophen-3-yl}-ethyl)-phenyl]-methanol
(2-Hydroxymethyl-5-{5-[methyl-(trifluoro-hydroxy-trifluoromethyl-ethyl)-phenyl]-thiophen-3-ylmethoxy}-phenyl)-methanol
[5-(2-{5-[Ethyl-(trifluoro-hydroxy-trifluoromethyl-ethyl)-phenyl]-thiophen-3-yl}-ethyl)-2-hydroxymethyl-phenyl]-methanol
(5-{5-[Ethyl-(trifluoro-hydroxy-trifluoromethyl-ethyl)-phenyl]-thiophen-3-ylmethoxy}-2-hydroxymethyl-phenyl)-methanol
[5-(2-{4-[4-(1-Ethyl-1-hydroxy-propyl)-2-methyl-phenyl]-thiophen-2-yl}-ethyl)-2-hydroxymethyl-phenyl]-methanol
(5-{4-[4-(1-Ethyl-1-hydroxy-propyl)-2-methyl-phenyl]-thiophen-2-ylmethoxy}-2-hydroxymethyl-phenyl)-methanol
[5-(2-{4-[2-Ethyl-4-{1-ethyl-1-hydroxy-propyl)-phenyl]-thiophen-2-yl}-ethyl)-2-hydroxymethyl-phenyl]-methanol
(5-{4-[2-Ethyl-4-(1-ethyl-1-hydroxy-propyl)-phenyl]-thiophen-2-ylmethoxy}-2-hydroxymethyl-phenyl)-methanol
[5-(2-{4-[4-(1-Ethyl-1-hydroxy-propyl)-2-methyl-phenyl]-5-methyl-thiophen-2-yl}-ethyl)-2-hydroxymethyl-phenyl]-methanol
(5-{4-[4-(1-Ethyl-1-hydroxy-propyl)-2-methyl-phenyl]-5-methyl-thiophen-2-ylmethoxy}-2-hydroxymethyl-phenyl)-methanol
[2-Hydroxymethyl-5-(2-{4-[methyl-(trifluoro-hydroxy-trifluoromethyl-ethyl)-phenyl]-thiophen-2-yl}-ethyl)-phenyl]-methanol
(2-Hydroxymethyl-5-{4-[methyl-(trifluoro-hydroxy-trifluoromethyl-ethyl)-phenyl]-thiophen-2-ylmethoxy}-phenyl)-methanol
[5-(2-{4-[Ethyl-(trifluoro-hydroxy-trifluoromethyl-ethyl)-phenyl]-thiophen-2-yl}-ethyl)-2-hydroxymethyl-phenyl]-methanol
(5-{4-[Ethyl-(trifluoro-hydroxy-trifluoromethyl-ethyl)-phenyl]-thiophen-2-ylmethoxy}-2-hydroxymethyl-phenyl)-methanol
[2-Hydroxymethyl-5-(2-{methyl-[methyl-(trifluoro-hydroxy-trifluoromethyl-ethyl)-phenyl]-thiophen-2-yl}-ethyl)-phenyl]-methanol
(2-Hydroxymethyl-5-{methyl-[methyl-(trifluoro-hydroxy-trifluoromethyl-ethyl)-phenyl]-thiophen-2-ylmethoxy}-phenyl)-methanol
{5-[2'-Ethyl-4'-(1-ethyl-1-hydroxy-propyl)-6-methyl-biphenyl-3-yloxymethyl]-2-hydroxymethyl-phenyl}-methanol
{5-[4'-(1-Ethyl-1-hydroxy-propyl)-2'-isopropyl-6-methyl-biphenyl-3-yloxymethyl]-2-hydroxymethyl-phenyl}-methanol
{5-[2'-*tert*-Butyl-4'-(1-ethyl-1-hydroxy-propyl)-6-methyl-biphenyl-3-yloxymethyl]-2-hydroxymethyl-phenyl}-methanol
{5-[Ethyl-methyl-(trifluoro-hydroxy-trifluoromethyl-ethyl)-biphenyl-3-yloxymethyl]-2-hydroxymethyl-phenyl}-methanol
{2-Hydroxymethyl-5-[2'-isopropyl-6-methyl-4'-(2,2,2-trifluoro-1-hydroxy-1-trifluoromethyl-ethyl)-biphenyl-3-yloxymethyl]-phenyl}-methanol
{5-[Dimethylethyl-methyl-(trifluoro-hydroxy-trifluoromethyl-ethyl)-biphenyl-3-yloxymethyl]-2-hydroxymethyl-phenyl}-methanol
{5-[6-Ethyl-4'-(1-ethyl-1-hydroxy-propyl)-2'-methyl-biphenyl-3-yloxymethyl]-2-hydroxymethyl-phenyl}-methanol
{5-[4'-(1-Ethyl-1-hydroxy-propyl)-6-methoxy-2'-methyl-biphenyl-3-yloxymethyl]-2-hydroxymethyl-phenyl}-methanol
{5-[6-*tert*-Butyl-4'-(1-ethyl-1-hydroxy-propyl)-2'-methyl-biphenyl-3-yloxymethyl]-2-hydroxymethyl-phenyl}-methanol
{5-[Ethyl-methyl-(trifluoro-hydroxy-trifluoromethyl-ethyl)-biphenyl-3-yloxymethyl]-2-hydroxymethyl-phenyl}-methanol
{2-Hydroxymethyl-5-[6-methoxy-2'-methyl-4'-(2,2,2-trifluoro-1-hydroxy-1-trifluoromethyl-ethyl)-biphenyl-3-yloxymethyl]-phenyl}-methanol
{5-[Dimethylethyl-methyl-(trifluoro-hydroxy-trifluoromethyl-ethyl)-biphenyl-3-yloxymethyl]-2-hydroxymethyl-phenyl}-methanol
(5-{2-[4'-(-Ethyl-1-hydroxy-propyl)-6,2'-dimethyl-biphenyl-3-yl]-ethyl}-2-hydroxymethyl-phenyl)-methanol
(5-{2-[Dimethyl-(trifluoro-hydroxy-trifluoromethyl-ethyl)-biphenyl-3-yl]-éthyl}-2-hydroxymethyl-phenyl)-methanol
(5-{[4'-(1-Ethyl-1-hydroxy-propyl)-6,2'-dimethyl-biphenyl-3-ylamino]-methyl}-2-hydroxymethyl-phenyl)-methanol
(5-{[Dimethyl-(trifluoro-hydroxy-trifluoromethyl-ethyl)-biphenyl-3-ylamino]-methyl}-2-hydroxymethyl-phenyl)-methanol
[5-({[4'-(1-Ethyl-1-hydroxy-propyl)-6,2'-dimethyl-biphenyl-3-yl]-methyl-amino}-methyl)-2-hydroxymethyl-phenyl]-methanol
[5-({[Dimethyl-(trifluoro-hydroxy-trifluoromethyl-ethyl)-biphenyl-3-yl]-methyl-amino}-methyl)-2-hydroxymethyl-phenyl]-methanol

8. Composés selon la revendication 1, **caractérisés par le fait qu'**ils présentent l'une au moins des, et de préférence toutes les, caractéristiques suivantes :
- R₁ représente le radical CH₃ ou CH₂OH,
- R₂ représente le radical CH₂OH,
- X-Y représente une liaison de formule (a) ou (c),
- R₃ représente le radical C(R₁₃)(R₁₄)OH.

9. Utilisation d'un composé selon l'une quelconque des revendications précédentes pour la fabrication d'un médicament.

10. Utilisation d'un composé selon l'une quelconque des revendications 1 à 8 pour la fabrication d'un médicament destiné au traitement :
- des affections dermatologiques liées à un désordre de la kératinisation portant sur la différenciation et sur la prolifération telles que les acnés vulgaires, comédoniennes, polymorphes, rosacées, les acnés nodulokystiques, conglobata, les acnés séniles, les acnés secondaires telles que l'acné solaire, médicamenteuse ou professionnelle ;
- d'autres types de troubles de la kératinisation, telles que les ichtyoses, les états ichtyosiformes, la maladie de Darrier, les kératodermies palmoplantaires, les leucoplasies et les états leucoplasiformes, le lichen cutané ou muqueux (buccal) ;
- d'autres affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore l'atopie cutanée, telle que l'eczéma ou l'atopie respiratoire ou encore l'hypertrophie gingivale ;
- des proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides, le lymphome T, et les proliférations pouvant être induites par les ultra-violets notamment dans le cas des épithélioma baso et spinocellulaires, ainsi que les lésions précancéreuses cutanées telles que les kératoacanthomes;
- des autres désordres dermatologiques tels que les dermatoses immunes telles le lupus érythémateux, les maladies immunes bulleuses ou les maladies du collagène, telle la sclérodermie;
- des affections dermatologiques ou générales à composante immunologique;
- des troubles de la fonction sébacée tels que l'hyperséborrhée de l'acné ou la séborrhée simple;
- des désordres cutanés dus à une exposition aux rayonnements U.V., du vieillissement de la peau, qu'il soit photo-induit ou chronologique, des pigmentations et les kératoses actiniques, ou toutes pathologies associées au vieillissement chronologique ou actinique;
- des troubles de la cicatrisation ou des vergetures,
- des affections inflammatoires telles que l'arthrite, des affections d'origine virale au niveau cutané ou général, telle que le syndrome de Kaposis;
- des troubles ophtalmologiques, notamment les coméopathies;
- des états cancéreux ou précancéreux de cancers présentant ou pouvant être induits par des récepteurs de vitamine D, tels que le cancer du sein, la leucémie, les syndromes myélodysplasiques et les lymphomes, les carcinomes des cellules de l'épithélium malpighien et les cancers gastro-intestinaux, les mélanomes, et l'ostéosarcome ;
- de l'alopécie de différentes origines, notamment l'alopécie due à la chimiothérapie ou aux rayonnements;
- des affections immunitaires, telles que les maladies auto-immunes, le diabète sucré de type 1, la sclérose en plaques, le lupus et les affections de type lupus, l'asthme, la glomérulonéphrite; des dysfonctionnements sélectifs du système immunitaire, telles que le SIDA, du rejet immun;
- des affections endocriniennes ;
- des affections **caractérisées par** une gestion anormale du calcium intracellulaire, des pathologies dans lesquelles le métabolisme calcique est impliqué, telle que l'ischémie musculaire;
- des carences en vitamine D et d'autres affections de l'homéostasie des minéraux dans le plasma et les os, tel que le rachitisme, l'ostéomalacie, l'ostéoporose, notamment dans le cas des femmes monopausées, l'ostéodystrophie rénale, ou les troubles de la fonction parathyroïdienne;
- des affections du système cardiovasculaire telles que l'artériosclérose ou l'hypertension ainsi que le diabète non-insulino dépendant,

11. Composition pharmaceutique, **caractérisée par le fait qu'**elle comprend, dans un support pharmaceutiquement acceptable, au moins l'un des composés tels que définis à l'une quelconque des revendications 1 à 8.

12. Composition selon la revendication 11, **caractérisée en ce que** la concentration en composé(s) selon l'une des revendications 1 à 8 est comprise entre 0,0001 % et 5 % en poids par rapport au poids total de la composition.

13. Composition cosmétique, **caractérisée par le fait qu'**elle comprend, dans un support cosmétiquement acceptable, au moins l'un des composés tels que définis à l'une quelconque des revendications 1 à 8.

14. Composition selon la revendication 13, **caractérisée en ce que** la concentration en composé(s) est comprise entre 0,001 % et 3 % en poids par rapport au poids total de la composition.

15. Utilisation d'une composition cosmétique telle que définie à l'une des revendications 13 ou 14 pour l'hygiène corporelle ou capillaire.

## Patentansprüche

1. Verbindungen, **dadurch gekennzeichnet, dass** sie der folgenden allgemeinen Formel (I) entsprechen: 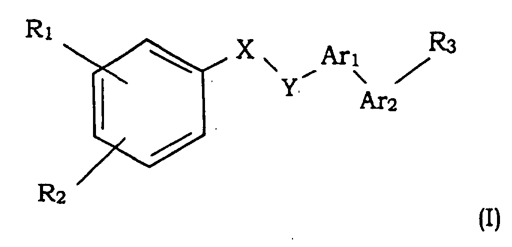 worin bedeuten:
- R₁ ein Wasserstoffatom, die Gruppe -CH₃ oder eine Gruppe -(CH₂)ᵣ-OR₄;
- R₂ eine Gruppe -(CH₂)ₛ-OR₅, wobei r, s, R₄ und R₅ die nachstehend angegebenen Bedeutungen aufweisen ;
- X-Y eine Verbindungsgruppe, die unter den Verbindungsgruppen der folgenden Formeln (a) bis (d) ausgewählt ist, wobei die Formeln von rechts nach links oder umgekehrt gelesen werden können: 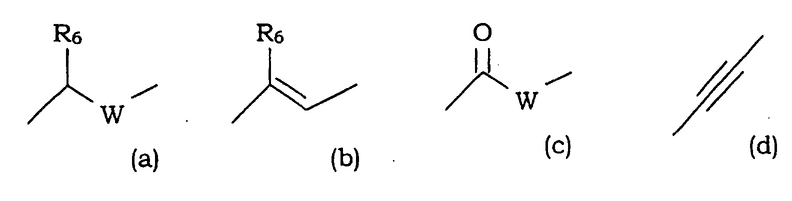
worin R₆ und W die nachstehend angegebenen Bedeutungen aufweisen;
- Ar₁ einen Ring der folgenden Formeln (e) bis (i): 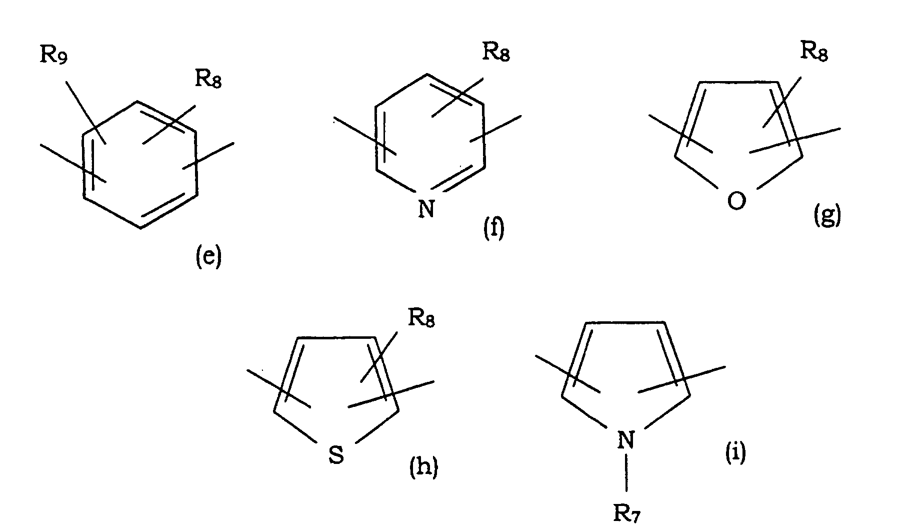
wobei R₇, R₈ und R₉ die nachstehend angegebenen Bedeutungen aufweisen;
- Ar₂ einen Ring der folgenden Formeln (j) bis (n): 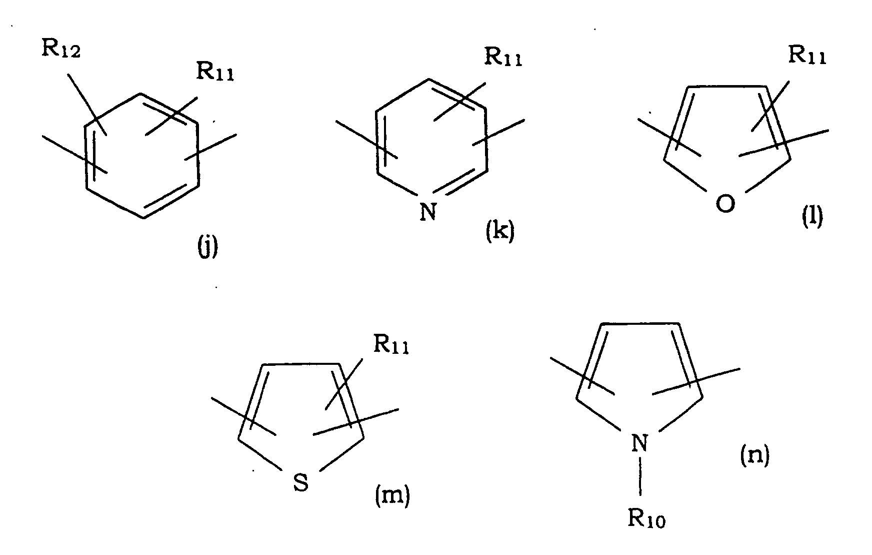
wobei R₁₀, R₁₁ und R₁₂ die nachstehend angegebenen Bedeutungen aufweisen;
- R₃ eine Gruppe der folgenden Formel: 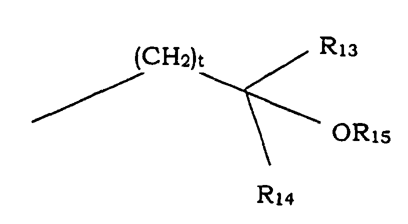
wobei t, R₁₃, R₁₄ und R₁₅ die nachstehend angegebenen Bedeutungen aufweisen;
- r und s, die gleich oder verschieden sind, 1 oder 2,
- R₄ und R₅, die gleich oder verschieden sind, Wasserstoff, Acetyl, Benzoyl, Trimethylsilyl, *t*-Butyldimethylsilyl oder Tetrahydropyranyl,
- R₆ ein Wasserstoffatom oder eine C₁₋₆-Alkylgruppe,
- W ein Sauerstoffatom, ein Schwefelatom, die Gruppe -CH₂ oder die Gruppe -NH-, die gegebenenfalls mit einer C₁₋₆-Alkylgruppe substituiert sein kann,
- R₇ und R₁₀, die gleich oder verschieden sind, ein Wasserstoffatom oder eine C₁₋₆-Alkylgruppe,
- R₈, R₉, R₁₁ und R₁₂, die gleich oder verschieden sind, ein Wasserstoffatom, eine C₁₋₆-Alkylgruppe, ein Halogenatom, eine Gruppe -OR₁₆, eine C₂₋₅-Polyethergruppe, die Gruppe -CF₃, die Gruppe NO₂ oder eine Aminogruppe, die mit einer oder zwei C₁₋₆-Alkylgruppen substituiert sein kann, wobei R₁₆ die nachstehend angegebenen Bedeutungen aufweist,
- t 0 oder 1,
- R₁₃ und R₁₄, die gleich oder verschieden sind, ein Wasserstoffatom, eine C₁₋₆-Alkylgruppe, eine C₃₋₆-Cycloalkylgruppe, die Gruppe -CF₃ oder die Gruppe -C₂F₅,
- R₁₅ Wasserstoff, Acetyl, Trimethylsilyl, *t*-Butyldimethylsilyl oder Tetrahydropyranyl,
- R₁₆ ein Wasserstoffatom oder eine C₁₋₆-Alkylgruppe,
und die optischen und geometrischen Isomere der Verbindungen der Formel (I) sowie ihre Salze.

2. Verbindungen nach Anspruch 1, **dadurch gekennzeichnet, dass** sie in Form von Salzen mit einer anorganischen oder organischen Säure, insbesondere Salzsäure, Schwefelsäure, Essigsäure, Fumarsäure, Hemibernsteinsäure, Maleinsäure oder Mandelsäure, vorliegen.

3. Verbindungen nach einem der Ansprüche 1 oder 2, **dadurch gekennzeichnet, dass** die C₁₋₆-Alkylgruppen unter Methyl, Ethyl, Isopropyl, *t*-Butyl und Hexyl ausgewählt sind.

4. Verbindungen nach einem der vorhergehenden Ansprüche, **dadurch gekennzeichnet, dass** die Cycloalkylgruppe Cyclopropyl, Cyclopentyl oder Cyclohexyl bedeutet.

5. Verbindungen nach einem der vorhergehenden Ansprüche, **dadurch gekennzeichnet, dass** das Halogenatom unter Fluor, Chlor oder Brom ausgewählt ist.

6. Verbindungen nach einem der vorhergehenden Ansprüche, **dadurch gekennzeichnet, dass** die Polyethergruppe die Gruppe Methoxymethoxy, Methoxyethoxy oder Methoxyethoxymethoxy bedeutet.

7. Verbindungen nach Anspruch 1, **dadurch gekennzeichnet, dass** sie unter den folgenden Verbindungen oder deren Gemischen ausgewählt sind:
{5-[4'-(1-Ethyl-1-hydroxy-propyl)-biphenyl-3-yloxymethyl]-2-hydroxymethyl-phenyl}-methanol,
{2-Hydroxymethyl-5-[4'-(1-hydroxy-1-methyl-ethyl)-biphenyl-3-yloxymethyl]-phenyl}-methanol,
{5-[4'-(1-Ethyl-1-hydroxy-propyl)-2'-methyl-biphenyl-3-yloxymethyl]-2-hydroxymethyl-phenyl}-methanol,
{2-Hydroxymethyl-5-[4'-(1-hydroxy-1-methyl-ethyl)-2'-methylbiphenyl-3-yloxymethyl]-phenyl}-methanol,
(5-{2-[3'-(1-Ethyl-1-hydroxy-propyl)-biphenyl-3-yl]-ethyl}-2-hydroxymethyl-phenyl)-methanol,
{2-Hydroxymethyl-5-[3'-(1-hydroxy-1-methyl-ethyl)-biphenyl-3-yloxymethyl]-phenyl}-methanol,
{{5-[4'-(2-Ethyl-2-hydroxy-butyl)-biphenyl-3-yloxymethyl]-2-hydroxymethyl-phenyl}-methanol,
{5-[3'-(2-Ethyl-2-hydroxy-butyl)-biphenyl-3-yloxymethyl]-2-hydroxymethyl-phenyl}-methanol,
1-[3'-(3,4-Bis-hydroxymethyl-benzyloxy)-biphenyl-3-yl]-2-methylpropan-2-ol,
{4-[4'-(1-Ethyl-1-hydroxy-propyl)-2'-methyl-biphenyl-3-ylsulfanylmethyl]-2-hydroxymethyl-phenyl}-methanol,
{4-[4'-(1-Ethyl-1-hydroxy-propyl)-2,2'-dimethyl-biphenyl-3-yloxymethyl]-2-hydroxymethyl-phenyl}-methanol,
{2-Hydroxymethyl-4-[4'-(1-hydroxy-1-propyl-butyl)-2,2'-dimethylbiphenyl-3-yloxymethyl]-phenyl}-methanol,
{4-[4'-(1-Ethyl-1-hydroxy-propyl)-2-methyl-biphenyl-3-yloxymethyl]-2-hydroxymethyl-phenyl}-methanol,
{4-[4'-(1-Ethyl-1-hydroxy-propyl)-2,2',6'-trimethyl-biphenyl-3-yloxymethyl]-2-hydroxymethyl-phenyl}-methanol,
(4-{3-[5-(1-Ethyl-1-hydroxy-propyl)-pyridin-2-yl]-phenoxymethyl}-2-hydroxymethyl-phenyl)-methanol,
{4-[4'-(1-Ethyl-1-hydroxy-propyl)-6,2'-dimethyl-biphenyl-3-yloxymethyl]-2-hydroxymethyl-phenyl}-methanol,
{4-[4'-(1-Ethyl-1-hydroxy-propyl)-6,2',6'-trimethyl-biphenyl-3-yloxymethyl]-2-hydroxymethyl-phenyl}-methanol,
{4-[4'-(1-Ethyl-1-hydroxy-propyl)-6-methyl-biphenyl-3-yloxymethyl]-2-hydroxymethyl-phenyl}-methanol,
{4-[4'-(1-Ethyl-1-hydroxy-propyl)-2',6'-dimethyl-biphenyl-3-yloxymethyl]-2-hydroxymethyl-phenyl}-methanol,
1-{4-[3-(3,4-Bis-hydroxymethyl-benzyloxy)-phenyl]-thiophen-2-yl}-2-methyl}-propan-1-ol,
(4-{3-[4-(1-Ethyl-1-hydroxy-propyl)-thiophen-2-yl]-phenoxymethyl}-2-hydroxymethyl-phenyl)-methanol,
{4-[4'-(1-Ethyl-1-hydroxy-propyl)-2'-methyl-biphenyl-3-ylmethoxy]-2-hydroxymethyl-phenyl}-methanol,
1-[3'-(3,4-Bis-hydroxymethyl-phenoxymethyl)-2-methyl-biphenyl-4-yl]-propan-1-ol,
{4-[4'-(1-Ethyl-1-hydroxy-propyl)-3'-methyl-biphenyl-3-yloxymethyl]-2-hydroxymethyl-phenyl}-methanol,
{5-[4'-(1-Ethyl-1-hydroxy-propyl)-2'-methyl-biphenyl-4-yloxymethyl]-2-hydroxymethyl-phenyl}-methanol,
{4-[2'-*t*-Butyl-4'-(1-ethyl-1-hydroxy-propyl)-biphenyl-3-yloxymethyl]-2-hydroxymethyl-phenyl}-methanol,
1-[3'-(3,4-Bis-hydroxymethyl-benzyloxy)-2-methyl-biphenyl-4-yl]-2,2-dimethyl-propan-1-ol,
1-[3'-(3,4-Bis-hydroxymethyl-benzyloxy)-2-methyl-biphenyl-4-yl]-2-methyl-propan-1-ol,
{2-Hydroxymethyl-4-[methyl-(trifluor-hydroxy-trifluormethylethyl)-biphenyl-3-yloxymethyl]-phenyl}-methanol,
[5-(2-{5-[4-(1-Ethyl-1-hydroxy-propyl)-2-methyl-phenyl]-thiophen-2-yl}-ethyl)-2-hydroxymethyl-phenyl]-methanol,
(5-{5-[4-(1-Ethyl-1-hydroxy-propyl)-2-methyl-phenyl]-thiophen-2-ylmethoxy}-2-hydroxymethyl-phenyl)-methanol,
[5-(2-{5-[2-Ethyl-4-(1-ethyl-1-hydroxy-propyl)-phenyl]-thiophen-2-yl}-ethyl)-2-hydroxymethyl-phenyl]-methanol,
(5-{5-[2-Ethyl-4-(1-ethyl-1-hydroxy-propyl)-phenyl]-thiophen-2-ylmethoxy}-2-hydroxymethyl-phenyl)-methanol,
[5-(2-{5-[4-(1-Ethyl-1-hydroxy-propyl)-2-methyl-phenyl]-4-methyl-thiophen-2-yl}-ethyl)-2-hydroxymethyl-phenyl]-methanol,
(5-{5-[4-(1-Ethyl-1-hydroxy-propyl)-2-methyl-phenyl]-4-methylthiophen-2-ylmethoxy}-2-hydroxymethyl-phenyl)-methanol,
[2-Hydroxymethyl-5-(2-{5-[methyl-(trifluor-hydroxytrifluormethyl-ethyl)-phenyl]-thiophen-2-yl}-ethyl)-phenyl]-methanol,
(2-Hydroxymethyl-5-{5-[methyl-(trifluor-hydroxy-trifluormethylethyl)-phenyl]-thiophen-2-ylmethoxy}-phenyl)-methanol,
[5-(2-{5-[Ethyl-(trifluor-hydroxy-trifluormethyl-ethyl)-phenyl]-thiophen-2-yl}-ethyl)-2-hydroxymethyl-phenyl]-methanol,
(5-{5-[Ethyl-(trifluor-hydroxy-trifluormethyl-ethyl)-phenyl]-thiophen-2-ylmethoxy}-2-hydroxymethyl-phenyl]-methanol,
[2-Hydroxymethyl-5-(2-{methyl-[methyl-(trifluor-hydroxytrifluormethyl-ethyl)-phenyl]-thiophen-2-yl}-ethyl)-phenyl]-methanol,
(2-Hydroxymethyl-5-{methyl-[methyl-(trifluor-hydroxytrifluormethyl-ethyl)-phenyl]-thiophen-2-ylmethoxy}-phenyl)-methanol,
[5-(2-{5-[4-(1-Ethyl-1-hydroxy-propyl)-2-methyl-phenyl]-thiophen-3-yl}-ethyl)-2-hydroxymethyl-phenyl]-methanol,
(5-{5-[4-(1-Ethyl-1-hydroxy-propyl)-2-methyl-phenyl]-thiophen-3-ylmethoxy}-2-hydroxymethyl-phenyl)-methanol,
[5-(2-{5-[2-Ethyl-4-(1-Ethyl-1-hydroxy-propyl)-phenyl]-thiophen-3-yl}-ethyl)-2-hydroxymethyl-phenyl]-methanol,
(5-{5-[2-Ethyl-4-(1-ethyl-1-hydroxy-propyl)-phenyl]-thiophen-3-ylmethoxy}-2-hydroxymethyl-phenyl)-methanol,
[2-Hydroxymethyl-5-(2-{5-[methyl-(trifluor-hydroxytrifluormethyl-ethyl)-phenyl]-thiophen-3-yl}-ethyl)-phenyl]-methanol,
(2-Hydroxymethyl-5-{5-[methyl-(trifluor-hydroxy-trifluormethylethyl)-phenyl]-thiophen-3-ylmethoxy}-phenyl)-methanol,
[5-(2-{5-[Ethyl-(trifluor-hydroxy-trifluormethyl-ethyl)-phenyl]-thiophen-3-yl}-ethyl)-2-hydroxymethyl-phenyl]-methanol,
(5-{5-[Ethyl-(trifluor-hydroxy-trifluormethyl-ethyl)-phenyl]-thiophen-3-ylmethoxy}-2-hydroxymethyl-phenyl)-methanol,
[5-(2-{4-[4-(1-Ethyl-1-hydroxy-propyl)-2-methyl-phenyl]-thiophen-2-yl}-ethyl)-2-hydroxymethyl-phenyl]-methanol,
(5-{4-[4-(1-Ethyl-1-hydroxy-propyl)-2-methyl-phenyl]-thiophen-2-ylmethoxy}-2-hydroxymethyl-phenyl)-methanol,
[5-(2-{4-[2-Ethyl-4-(1-Ethyl-1-hydroxy-propyl)-phenyl]-thiophen-2-yl}-ethyl)-2-hydroxymethyl-phenyl]-methanol,
(5-{4-[2-Ethyl-4-(1-ethyl-1-hydroxy-propyl)-phenyl]-thiophen-2-ylmethoxy}-2-hydroxymethyl-phenyl)-methanol,
[5-(2-{4-[4-(1-Ethyl-1-hydroxy-propyl)-2-methyl-phenyl]-5-methyl-thiophen-2-yl}-ethyl)-2-hydroxymethyl-phenyl]-methanol,
(5-{4-[4-(1-Ethyl-1-hydroxy-propyl)-2-methyl-phenyl]-5-methylthiophen-2-ylmethoxy}-2-hydroxymethyl-phenyl)-methanol,
[2-Hydroxymethyl-5-(2-{4-[methyl-(trifluor-hydroxytrifluormethyl-ethyl)-phenyl]-thiophen-2-yl}-ethyl)-phenyl]-methanol,
(2-Hydroxymethyl-5-{4-[methyl-(trifluor-hydroxy-trifluormethylethyl)-phenyl]-thiophen-2-ylmethoxy}-phenyl)-methanol,
[5-(2-{4-[Ethyl-(trifluor-hydroxy-trifluormethyl-ethyl)-phenyl]-thiophen-2-yl}-ethyl)-2-hydroxymethyl-phenyl]-methanol,
(5-{4-[Ethyl-(trifluor-hydroxy-trifluormethyl-ethyl)-phenyl]-thiophen-2-ylmethoxy}-2-hydroxymethyl-phenyl)-methanol,
[2-Hydroxymethyl-5-(2-{methyl-[methyl-(trifluor-hydroxytrifluormethyl-ethyl)-phenyl]-thiophen-2-yl}-ethyl)-phenyl]-methanol,
(2-Hydroxymethyl-5-{methyl-[methyl-(trifluor-hydroxytrifluormethyl-ethyl)-phenyl]-thiophen-2-ylmethoxy}-phenyl)-methanol,
{5-[2'-Ethyl-4'-(1-ethyl-1-hydroxy-propyl)-6-methyl-biphenyl-3-yloxymethyl]-2-hydroxymethyl-phenyl}-methanol,
{5-[4'-(1-Ethyl-1-hydroxy-propyl)-2'-isopropyl-6-methyl-biphenyl-3-yloxymethyl]-2-hydroxymethyl-phenyl}-methanol,
{5-[2'-*t*-Butyl-4'-(1-ethyl-1-hydroxy-propyl)-6-methyl-biphenyl-3-yloxymethyl]-2-hydroxymethyl-phenyl}-methanol,
{5-[Ethyl-methyl-(trifluor-hydroxy-trifluormethyl-ethyl)-biphenyl-3-yloxymethyl]-2-hydroxymethyl-phenyl}-methanol,
{2-Hydroxymethyl-5-[2'-isopropyl-6-methyl-4'-(2,2,2-trifluor-1-hydroxy-1-trifluormethyl-ethyl)-biphenyl-3-yloxymethyl]-phenyl}-methanol,
{5-[Dimethylethyl-methyl-(trifluor-hydroxy-trifluormethyl-ethyl)-biphenyl-3-yloxymethyl]-hydroxymethyl-phenyl}-methanol,
{5-[6-Ethyl-4'-(1-ethyl-1-hydroxy-propyl)-2'-methyl-biphenyl-3-yloxymethyl]-2-hydroxymethyl-phenyl]-methanol,
{5-[4'-(1-Ethyl-1-hydroxy-propyl)-6-methoxy-2'-methyl-biphenyl-3-yloxymethyl]-2-hydroxymethyl-phenyl}-methanol,
{5-[6-*t*-Butyl-4'-(1-ethyl-1-hydroxy-propyl)-2'-methyl-biphenyl-3-yloxymethyl]-2-hydroxymethyl-phenyl}-methanol,
{5-[Ethyl-methyl-(trifluor-hydroxy-trifluormethyl-ethyl)-biphenyl-3-yloxymethyl]-2-hydroxymethyl-phenyl}-methanol,
{2-Hydroxymethyl-5-[6-methoxy-2'-methyl-4'-(2,2,2-trifluor-1-hydroxy-1-trifluormethyl-ethyl)-biphenyl-3-yloxymethyl]-phenyl}-methanol,
{5-[Dimethylethyl-methyl-(trifluor-hydroxy-trifluormethyl-ethyl)-biphenyl-3-yloxymethyl]-2-hydroxymethyl-phenyl}-methanol,
(5-{2-[4'-(1-Ethyl-1-hydroxy-propyl)-6,2'-dimethyl-biphenyl-3-yl]-ethyl}-2-hydroxymethyl-phenyl)-methanol,
(5-{2-[Dimethyl-(trifluor-hydroxy-trifluormethyl-ethyl)-biphenyl-3-yl]-ethyl}-2-hydroxymethyl-phenyl}-methanol,
(5-{[4'-(1-Ethyl-1-hydroxy-propyl)-6,2'-dimethyl-biphenyl-3-ylamino]-methyl}-2-hydroxymethyl-phenyl)-methanol,
(5-{[Dimethyl-(trifluor-hydroxy-trifluormethyl-ethyl)-biphenyl-3-ylamino]-methyl}-2-hydroxymethyl-phenyl}-methanol,
[5-({[4'-(1-Ethyl-1-hydroxy-propyl)-6,2'-dimethyl-biphenyl-3-yl]-methyl-amino}-methyl)-2-hydroxymethyl-phenyl]-methanol,
[5-({[Dimethyl-(trifluor-hydroxy-trifluormethyl-ethyl)-biphenyl-3-yl]-methyl-amino}-methyl)-2-hydroxymethyl-phenyl]-methanol.

8. Verbindungen nach Anspruch 1, **dadurch gekennzeichnet, dass** sie mindestens eine der folgenden Eigenschaften und vorzugsweise alle folgenden Eigenschaften aufweisen:
- R₁ bedeutet die Gruppe CH₃ oder CH₂OH;
- R₂ bedeutet die Gruppe CH₂OH;
- X-Y bedeutet eine Verbindungsgruppe der Formel (a) oder (c);
- R₃ bedeutet die Gruppe C(R₁₃)(R₁₄)OH.

9. Verwendung einer Verbindung nach einem der vorhergehenden Ansprüche für die Herstellung eines Arzneimittels.

10. Verwendung einer Verbindung nach einem der Ansprüche 1 bis 8 für die Herstellung eines Arzneimittels, das für die Behandlung der folgenden Erkrankungen vorgesehen ist:
- zur Behandlung von dermatologischen Erkrankungen, die mit einer Verhornungsstörung verbunden sind, die auf der Differenzierung und Proliferation beruht, beispielsweise zur Behandlung von Acne vulgaris, Acne comedonica, polymorpher Acne, Acne rosaceae, nodulocystischer Acne, Acne conglobata, Acne senilis, sekundären Akneformen, wie Acne solaris, Acne medicamentosa oder Acne professionalis;
- zur Behandlung von weiteren Arten von Verhornungsstörungen, wie Ichtyosis, ichtyosisartigen Zuständen, der Darier Krankheit, Palmoplantarkeratosen, Leucoplakien und leucoplakieformen Zuständen und Lichen der Haut oder der Schleimhäute (buccal);
- zur Behandlung weiterer dermatologischer Erkrankungen mit entzündlicher immunoallergischer Komponente mit oder ohne Proliferationsstörung der Zellen, insbesondere aller Arten von Psoriasis der Haut, der Schleimhäute oder der Nägel, Psoriasis arthropathica, Atopie der Haut wie Ekzemen, Atopie der Atemwege oder Hypertrophie des Zahnfleisches;
- zur Behandlung von Proliferationen der Dermis oder Epidermis, die gutartig oder bösartig und gegebenenfalls viralen Ursprungs sein können, wie Verrucae vulgares, Verrucae planae und Epidermodysplasia verruciformis, Papillomatosis oralis oder florida, T-Lymphomen und Proliferationen, die von UV-Strahlung induziert sein können, insbesondere im Falle von Epithelioma basocellulare und spinocellulare, sowie präkanzerösen Lesionen der Haut, wie Keratoakanthomen;
- zur Behandlung von weiteren dermatologischen Störungen, wie Immundermatosen, beispielsweise Lupus erythematodes, bullösen Immunerkrankungen und Kollagenerkrankungen, wie Sklerodermie;
- zur Behandlung von dermatologischen Störungen oder allgemeinen Störungen mit immunologischer Komponente;
- zur Behandlung von Funktionsstörungen der Talgdrüse, wie Hyperseborrhoe bei Akne oder einfache Seborrhoe;
- zur Behandlung von Hautstörungen, die von einer Exposition gegenüber UV-Strahlung herrühren, lichtinduzierter oder altersbedingter Hautalterung, aktinischen Pigmentierungen und Keratosen oder Pathologien, die mit der altersbedingten oder aktinischen Alterung zusammenhängen;
- zur Behandlung von Störungen der Wundheilung oder Streifen;
- zur Behandlung von entzündlichen Erkrankungen, wie Arthritis, Erkrankungen viraler Herkunft der Haut oder allgemeinen Erkrankungen viraler Herkunft, wie des Kaposi-Syndrom;
- zur Behandlung von ophthalmologischen Störungen, insbesondere Corneopathien;
- zur Behandlung von kanzerösen oder präkanzerösen Zuständen, die vorhanden sind oder von Vitamin D-Rezeptoren induziert werden können, wie Brustkrebs, Leukämie, myelodysplastischen Syndromen und Lymphomen, Stachelzellkarzinomen und Magen-Darm-Krebs, Melanomen und Osteosarkom;
- zur Behandlung von Alopezie unterschiedlichen Ursprungs, insbesondere durch Chemotherapie oder Strahlung verursachter Alopezie;
- zur Behandlung von Beeinträchtigungen der Immunfunktion, wie Autoimmunkrankheiten, Diabetes vom Typ 1, multiple Sklerose, Lupus und Erkrankungen vom Lupus-Typ, Asthma, Glomerulonephritis, selektive Funktionsstörungen des Immunsystems, wie AIDS, Immunrejektion;
- zur Behandlung von endokrinen Erkrankungen;
- zur Behandlung von Erkrankungen, die durch einen anormalen Haushalt des intrazellulären Calcium gekennzeichnet sind, Erkrankungen, bei denen der Calcium-Stoffwechsel beteiligt ist, wie Ischämie der Muskeln;
- zur Behandlung von Vitamin D-Mangel und weiteren Störungen der Homöostase von Mineralien im Plasma und in den Knochen, wie Rachitis, Osteomalazie, Osteoporose, insbesondere bei Frauen in den Wechseljahren, renale Osteodystrophie oder Störungen der Nebenschilddrüsenfunktion;
- zur Behandlung von Erkrankungen des cardiovasculären Systems, beispielsweise Arteriosklerose, Bluthochdruck sowie insulinunabhängiger Diabetes.

11. Pharmazeutische Zusammensetzung, **dadurch gekennzeichnet, dass** sie in einem pharmazeutisch akzeptablen Träger mindestens eine Verbindung nach einem der Ansprüche 1 bis 8 enthält.

12. Zusammensetzung nach Anspruch 11, **dadurch gekennzeichnet, dass** die Konzentration der Verbindung(en) nach einem der Ansprüche 1 bis 8 im Bereich von 0,0001 bis 5 Gew.-%, bezogen auf das Gesamtgewicht der Zusammensetzung, liegt.

13. Kosmetische Zusammensetzung, **dadurch gekennzeichnet, dass** sie in einem kosmetisch akzeptablen Träger mindestens eine Verbindung nach einem der Ansprüche 1 bis 8 enthält.

14. Zusammensetzung nach Anspruch 13, **dadurch gekennzeichnet, dass** die Konzentration der Verbindung(en) im Bereich von 0,001 bis 3 Gew.-%, bezogen auf das Gesamtgewicht der Zusammensetzung, liegt.

15. Verwendung einer kosmetischen Zusammensetzung nach einem der Ansprüche 13 oder 14 für die Körperpflege oder Haarpflege.

## Claims

1. Compounds, **characterized in that** they correspond to the general formula (I) below: 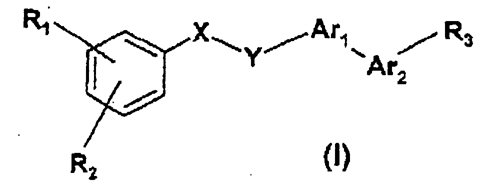 in which:
- R₁ represents a hydrogen atom, a CH₃ radical or a radical - (CH₂)ᵣ-OR₄,
- R₂ represents a radical -(CH₂)ₛ-OR₅
r, s, R₄ and R₅ having the meanings given below,
- X-Y represents a bond chosen from the bonds of formulae (a) to (d) below which can be read from left to right or vice-versa: 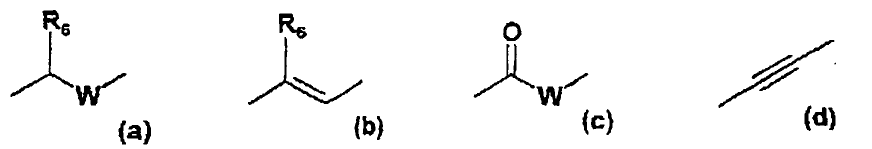 R₆ and W having the meanings given below,
- Ar₁ represents a ring of formulae (e) to (i) below: 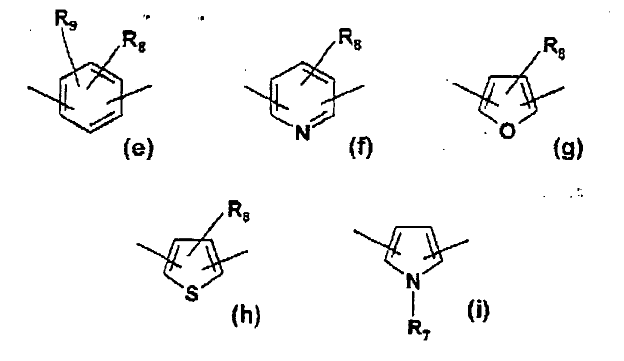 R₇, R₈ and R₉ having the meanings given below,
- Ar₂ represents a ring of formulae (j) to (n) below: 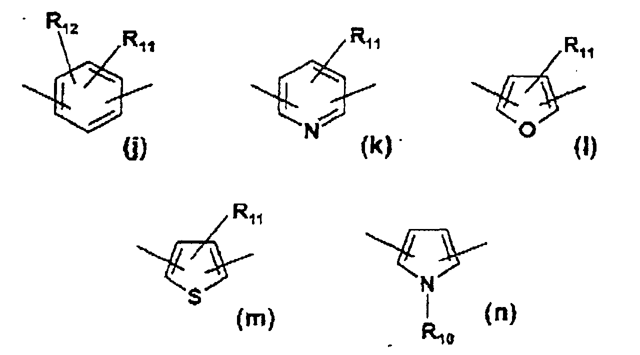 R₁₀, R₁₁ and R₁₂ having the meanings given below,
- R₃ represents a radical of formula: 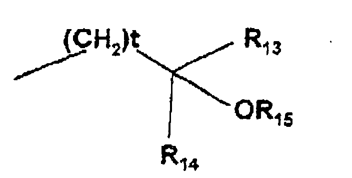 t, R₁₃, R₁₄ and R₁₅ having the meanings given below,
- r and s, which may be identical or different, being 1 or 2,
- R₄ and R₅, which may be identical or different, represent a hydrogen atom, an acetyl radical, a benzoyl radical, a trimethylsilyl radical, a tertbutyldimethylsilyl radical or a tetrahydropyranyl radical,
- R₆ represents a hydrogen atom or a C₁-C₆ alkyl radical,
- W represents an oxygen or sulphur atom, a CH₂ radical or an NH radical which can be substituted with a C₁-C₆ alkyl radical,
- R₇ and R₁₀, which may be identical or different, represent a hydrogen atom or a C₁-C₆ alkyl radical,
- R₈, R₉, R₁₁ and R₁₂, which may be identical or different, represent a hydrogen atom, a C₁-C₆ alkyl radical, a halogen atom, a radical -OR₁₆, a C₂-C₅ polyether radical, a CF₃ radical, an NO₂ radical or an amino radical which may be substituted with one or two C₁-C₆ alkyl radicals,
R₁₆ having the meanings given below,
- t being 0 or 1,
- R₁₃ and R₁₄, which may be identical or different, represent a hydrogen atom, a C₁-C₆ alkyl radical, a C₃-C₆ cycloalkyl radical, a CF₃ radical or a C₂F₅ radical,
- R₁₅ represents a hydrogen atom, an acetyl radical, a trimethylsilyl radical, a tertbutyldimethylsilyl radical or a tetrahydropyranyl radical,
- R₁₆ represents a hydrogen atom or a C₁-C₆ alkyl radical,
and the optical and geometrical isomers of the said compounds of formula (I), as well as the salts thereof.

2. Compounds according to Claim 1, **characterized in that** they are in the form of salts of an inorganic or organic acid, in particular hydrochloric acid, sulphuric acid, acetic acid, fumaric acid, hemisuccinic acid, maleic acid or mandelic acid.

3. Compounds according to either of Claims 1 and 2, **characterized in that** the alkyl radicals are chosen from methyl, ethyl, isopropyl, tert-butyl and hexyl radicals.

4. Compounds according to one of the preceding claims, **characterized in that** the cycloalkyl radical corresponds to a cyclopropyl, cyclopentyl or cyclohexyl radical.

5. Compounds according to one of the preceding claims, **characterized in that** the halogen atom corresponds to a fluorine, chlorine or bromine atom.

6. Compounds according to any one of the preceding claims, **characterized in that** the polyether radical corresponds to a methoxymethoxy, methoxyethoxy or methoxyethoxymethoxy radical.

7. Compounds according to Claim 1, **characterized in that** they are taken, alone or as mixtures, from the group consisting of:
{5-[4'-(1-ethyl-1-hydroxypropyl)biphenyl-3-yloxymethyl]-2-hydroxymethylphenyl}methanol
{2-hydroxymethyl-5-[4'-(1-hydroxy-1-methylethyl)biphenyl-3-yloxymethyl]phenyl}methanol
{5-[4'-(1-ethyl-1-hydroxypropyl)-2'-methylbiphenyl-3-yloxymethyl]-2-hydroxymethylphenyl}methanol
{2-hydroxymethyl-5-[4'-(1-hydroxy-1-methylethyl)-2'-methylbiphenyl-3-yloxymethyl]phenyl}methanol
(5-{2-[3'-(1-ethyl-1-hydroxypropyl)biphenyl-3-yl]ethyl}-2-hydroxymethylphenyl)methanol
{2-hydroxymethyl-5-[3'-(1-hydroxy-1-methylethyl)biphenyl-3-yloxymethyl]phenyl)methanol
{5-[4'-(2-ethyl-2-hydroxybutyl)biphenyl-3-yloxymethyl]-2-hydroxymethylphenyl}methanol
{5-[3'-(2-ethyl-2-hydroxybutyl)biphenyl-3-yloxymethyl]-2-hydroxymethylphenyl}methanol
1-[3'-(3,4-bis-hydroxymethyl-benzyloxy)biphenyl-3-yl]-2-methyl-2-propanol
{4-[4'-(1-ethyl-1-hydroxypropyl)-2'-methylbiphenyl-3-ylsulphanylmethyl]-2-hydroxymethylphenyl}methanol
{4-[4'-(1-ethyl-1-hydroxypropyl)-2,2'-dimethylbiphenyl-3-yloxymethyl]-2-hydroxymethylphenyl}methanol
{2-hydroxymethyl-4-[4'-(1-hydroxy-1-propylbutyl)-2,2'-dimethylbiphenyl-3-yloxymethyl]phenyl}methanol
{4-[4'-(1-ethyl-1-hydroxypropyl)-2-methylbiphenyl-3-yloxymethyl]-2-hydroxymethylphenyl}methanol
{4-[4'-(1-ethyl-1-hydroxypropyl)-2,2',6'-trimethylbiphenyl-3-yloxymethyl]-2-hydroxymethylphenyl}methanol
(4-{3-[5-(1-ethyl-1-hydroxypropyl)-2-pyridyl]phenoxymethyl}-2-hydroxymethylphenyl)methanol
{4-[4'-(1-ethyl-1-hydroxypropyl)-6,2'-dimethylbiphenyl-3-yloxymethyl]-2-hydroxymethylphenyl}methanol
{4-[4'-(1-ethyl-1-hydroxypropyl)-6,2',6'-trimethylbiphenyl-3-yloxymethyl]-2-hydroxymethylphenyl}methanol
{4-[4'-(1-ethyl-1-hydroxypropyl)-6-methylbiphenyl-3-yloxymethyl]-2-hydroxymethylphenyl}methanol
{4-[4'-(1-ethyl-1-hydroxypropyl)-2',6'-dimethylbiphenyl-3-yloxymethyl]-2-hydroxymethylphenyl}methanol
1-{4-[3-(3,4-bis-hydroxymethyl-benzyloxy)phenyl]-2-thienyl}-1-propanol
(4-{3-[4-(1-ethyl-1-hydroxypropyl)-2-thienyl]phenoxymethyl}-2-hydroxymethylphenyl)methanol
{4-[4'-(1-ethyl-1-hydroxypropyl)-2'-methylbiphenyl-3-ylmethoxy]-2-hydroxymethylphenyl}methanol
1-[3'-(3,4-bis-hydroxymethyl-phenoxymethyl)-2-methylbiphenyl-4-yl]-1-propanol
{4-[4'-(1-ethyl-1-hydroxypropyl)-3'-methylbiphenyl-3-yloxymethyl]-2-hydroxymethylphenyl}methanol
{5-[4'-(1-ethyl-1-hydroxypropyl)-2'-methylbiphenyl-4-yloxymethyl]-2-hydroxymethylphenyl}methanol
{4-[2'-tert-butyl-4'-(1-ethyl-2-hydroxypropyl)biphenyl-3-yloxymethyl]-2-hydroxymethylphenyl}methanol
1-[3'-(3,4-bis-hydroxymethyl-benzyloxy)-2-methylbiphenyl-4-yl]-2,2-dimethyl-1-propanol
1-[3'-(3,4-bis-hydroxymethyl-benzyloxy)-2-methylbiphenyl-4-yl]-2-methyl-1-propanol
{2-hydroxymethyl-4-[methyl(trifluorohydroxytrifluoromethylethyl)biphenyl-3-yloxymethyl]phenyl}methanol
[5-(2-{5-[4-(1-ethyl-1-hydroxypropyl)-2-methylphenyl]-2-thienyl}ethyl)-2-hydroxymethylphenyl]methanol
(5-{5-[4-(1-ethyl-1-hydroxypropyl)-2-methylphenyl]-2-thienylmethoxy}-2-hydroxymethylphenyl)methanol
[5-(2-{5-[2-ethyl-4-(1-ethyl-1-hydroxypropyl)phenyl]-2-thienyl}ethyl)-2-hydroxymethylphenyl]methanol
(5-{5-[2-ethyl-4-(1-ethyl-1-hydroxypropyl)phenyl]-2-thienylmethoxy}-2-hydroxymethylphenyl)methanol
[5-(2-{5-[4-(1-ethyl-1-hydroxypropyl)-2-methylphenyl]-4-methyl-2-thienyl}ethyl)-2-hydroxymethylphenyl]methanol
(5-{5-[4-(1-ethyl-1-hydroxypropyl)-2-methylphenyl]-4-methyl-2-thienylmethoxy}-2-hydroxymethylphenyl)methanol
[2-hydroxymethyl-5-(2-(5-[methyl(trifluorohydroxytrifluoromethylethyl)phenyl]-2-thienyl}ethyl)phenyl]methanol
(2-hydroxymethyl-5-{5-[methyl(trifluorohydroxytrifluoromethylethyl)phenyl]-2-thienylmethoxy}phenyl)methanol
[5-(2-{5-[ethyl(trifluorohydroxytrifluoromethylethyl)phenyl]-2-thienyl}ethyl)-2-hydroxymethylphenyl]methanol
(5-{5-[ethyl(trifluorohydroxytrifluoromethylethyl)phenyl]-2-thienylmethoxy}-2-hydroxymethylphenyl)methanol
[2-hydroxymethyl-5-(2-{methyl[methyl(trifluorohydroxytrifluoromethylethyl)phenyl]-2-thienyl}ethyl)phenyl]methanol
(2-hydroxymethyl-5-{methyl[methyl(trifluorohydroxytrifluoromethylethyl)phenyl]-2-thienylmethoxy}phenyl)methanol
[5-(2-{5-[4-(1-ethyl-1-hydroxypropyl)-2-methylphenyl]-3-thienyl}ethyl)-2-hydroxymethylphenyl]methanol
(5-{5-[4-(1-ethyl-1-hydroxypropyl)-2-methylphenyl]-3-thienylmethoxy}-2-hydroxymethylphenyl)methanol
[5-(2-{5-[2-ethyl-4-(1-ethyl-1-hydroxypropyl)phenyl]-3-thienyl}ethyl)-2-hydroxymethylphenyl]methanol
(5-{5-[2-ethyl-4-(1-ethyl-1-hydroxypropyl)phenyl]-3-thienylmethoxy}-2-hydroxymethylphenyl)methanol
[2-hydroxymethyl-5-(2-{5-[methyl(trifluorohydroxytrifluoromethylethyl)phenyl]-3-thienyl}ethyl)phenyl]methanol
(2-hydroxymethyl-5-{5-[methyl(trifluorohydroxytrifluoromethylethyl)phenyl]-3-thienylmethoxy}phenyl)methanol
[5-(2-{5-[ethyl(trifluorohydroxytrifluoromethylethyl)phenyl]-3-thienyl}ethyl)-2-hydroxymethylphenyl]methanol
(5-{5-[ethyl(trifluorohydroxytrifluoromethylethyl)phenyl]-3-thienylmethoxy}-2-hydroxymethylphenyl)methanol
[5-(2-{4-[4-(1-ethyl-1-hydroxypropyl)-2-methylphenyl]-2-thienyl}ethyl)-2-hydroxymethylphenyl]methanol
(5-{4-[4-(1-ethyl-1-hydroxypropyl)-2-methylphenyl]-2-thienylmethoxy}-2-hydroxymethylphenyl)methanol
[5-(2-{4-[2-ethyl-4-(1-ethyl-1-hydroxypropyl)phenyl]-2-thienyl}ethyl)-2-hydroxymethylphenyl]methanol
(5-{4-[2-ethyl-4-(1-ethyl-1-hydroxypropyl)phenyl]-2-thienylmethoxy}-2-hydroxymethylphenyl)methanol
[5-(2-{4-[4-(1-ethyl-1-hydroxypropyl)-2-methylphenyl]-5-methyl-2-thienyl}ethyl)-2-hydroxymethylphenyl]methanol
(5-{4-[4-(1-ethyl-1-hydroxypropyl)-2-methylphenyl]-5-methyl-2-thienylmethoxy}-2-hydroxymethylphenyl)methanol
[2-hydroxymethyl-5-(2-{4-[methyl(trifluorohydroxytrifluoxomethylethyl)phenyl]-2-thienyl}ethyl)phenyl]methanol
(2-hydroxymethyl-5-{4-[methyl(trifluorohydroxytrifluoromethylethyl)phenyl]-2-thienylmethoxy}phenyl)methanol
[5-(2-{4-[ethyl(trifluorohydroxytrifluoromethylethyl)phenyl]-2-thienyl}ethyl)-2-hydroxymethylphenyl]methanol
(5-{4-[ethyl(trifluorohydroxytrifluoromethylethyl)phenyl]-2-thienylmethoxy}-2-hydroxymethylphenyl)methanol
[2-hydroxymethyl-5-(2-{methyl[methyl(trifluorohydroxytrifluoromethylethyl)phenyl]-2-thienyl}ethyl)phenyl]methanol
(2-hydroxymethyl-5-{methyl[methyl(trifluorohydroxytrifluoromethylethyl)phenyl]-2-thienylmethoxy}phenyl)methanol
{5-[2'-ethyl-4'-(1-ethyl-1-hydroxypropyl)-6-methylbiphenyl-3-yloxymethyl]-2-hydroxymethylphenyl}methanol
{5-[4'-(1-ethyl-1-hydroxypropyl)-2'-isopropyl-6-methylbiphenyl-3-yloxymethyl]-2-hydroxymethylphenyl}methanol
{5-[2'-tert-butyl-4'-(1-ethyl-1-hydroxypropyl)-6-methylbiphenyl-3-yloxymethyl]-2-hydroxymethylphenyl}methanol
{5-[ethylmethyl(trifluorohydroxytrifluoromethylethyl)biphenyl-3-yloxymethyl]-2-hydroxymethylphenyl}methanol
{2-hydroxymethyl-5-[2'-isopropyl-6-methyl-4'-(2,2,2-trifluoro-1-hydroxy-1-trifluoromethylethyl)biphenyl-3-yloxymethyl]phenyl}methanol
{5-[dimethylethylmethyl(trifluorohydroxytrifluoromethylethyl)biphenyl-3-yloxymethyl]-2-hydroxymethylphenyl}methanol
{5-[6-ethyl-4'-(1-ethyl-1-hydroxypropyl)-2'-methylbiphenyl-3-yloxymethyl]-2-hydroxymethylphenyl}methanol
{5-[4'-(1-ethyl-1-hydroxypropyl)-6-methoxy-2'-methylbiphenyl-3-yloxymethyl]-2-hydroxymethylphenyl}methanol
{5-[6-tert-butyl-4'-(1-ethyl-1-hydroxypropyl)-2'-methylbiphenyl-3-yloxymethyl]-2-hydroxymethylphenyl}methanol
{5-[ethylmethyl(trifluorohydroxytrifluoromethylethyl)biphenyl-3-yloxymethyl]-2-hydroxymethylphenyl}methanol
{2-hydroxymethyl-5-[6-methoxy-2'-methyl-4'-(2,2,2-trifluoro-1-hydroxy-1-trifluoromethylethyl)biphenyl-3-yloxymethyl]phenyl}methanol
{5-[dimethylethylmethyl(trifluorohydroxytrifluoromethylethyl)biphenyl-3-yloxymethyl]-2-hydroxymethylphenyl}methanol
(5-{2-[4'-(1-ethyl-1-hydroxypropyl)-6,2'-dimethylbiphenyl-3-yl]ethyl}-2-hydroxymethylphenyl)methanol
(5-{2-[dimethyl(trifluorohydroxytrifluoromethylethyl)biphenyl-3-yl]ethyl}-2-hydroxymethylphenyl}methanol
(5-{[4'-(1-ethyl-1-hydroxypropyl)-6,2'-dimethylbiphenyl-3-ylamino]methyl}-2-hydroxymethylphenyl)methanol
(5-{[dimethyl(trifluorohydroxytrifluoromethylethyl)biphenyl-3-ylamino]methyl}-2-hydroxymethylphenyl)methanol
[5-({[4'-(1-ethyl-1-hydroxypropyl)-6,2'-dimethylbiphenyl-3-yl]methylamino}methyl)-2-hydroxymethylphenyl]methanol
[5-({[dimethyl(trifluorohydroxytrifluoromethylethyl)biphenyl-3-yl]methylamino}methyl)-2-hydroxymethylphenyl)methanol.

8. Compounds according to Claim 1, **characterized in that** they have at least one, and preferably all, of the following characteristics:
- R₁ represents a CH₃ or CH₂OH radical,
- R₂ represents a CH₂OH radical,
- X-Y represents a bond of formula (a) or (c),
- R₃ represents a radical C(R₁₃) (R₁₄)OH.

9. Use of a compound according to any one of the preceding claims, for the manufacture of a medicinal product.

10. Use of a compound according to any one of Claims 1 to 8, for the manufacture of a medicinal product intended for the treatment:
- of dermatological complaints associated with a keratinization disorder which has a bearing on differentiation and on proliferation, such as simple acne, comedones, polymorphonuclear leukocytes, rosacea, nodulocystic acne, acne conglobata, senile acne and secondary acnes such as solar, medication-related or professional acne;
- of other types of keratinization disorder, such as ichthyosis, ichthyosiform states, Darier's disease, palmoplantar keratoderma, leukoplasias and leukoplasiform states, and cutaneous or mucous (buccal) lichen;
- of other dermatological complaints with an inflammatory and/or immunoallergic component, with or without cell proliferation disorders, and, in particular, all forms of psoriasis, whether this is cutaneous, mucous or ungual psoriasis, and even psoriatic rheumatism, or alternatively cutaneous atopy, such as eczema or respiratory atopy or alternatively gingival hypertrophy;
- of dermal or epidermal proliferations, whether benign or malignant and whether they are of viral origin or otherwise, such as common warts, flat warts and verruciform epidermodysplasia, oral or florid papillomatoses, T lymphoma, and proliferations which can be induced by ultraviolet radiation, in particular in the case of basocellular and spinocellular epithelioma, as well as any pre-cancerous skin lesions such as keratoacanthomas;
- of other dermatological disorders such as immune dermatitis such as lupus erythematosus, immune bullosis and collagen diseases such as scleroderma;
- of dermatological or general complaints with an immunological component;
- of disorders of sebaceous function such as the hyperseborrhoea of acne or simple seborrhoea;
- of skin disorders due to exposure to UV radiation, of ageing of the skin, whether it is light-induced or chronological ageing, of pigmentations and actinic keratoses, or any pathologies associated with chronological or actinic ageing;
- of cicatrization disorders or stretchmarks;
- of inflammatory complaints such as arthritis, of complaints of viral origin on the skin or generally, such as Kaposi's syndrome;
- of ophthalmological disorders, in particular corneopathies;
- of cancerous or pre-cancerous states of cancers presenting or possibly being induced by vitamin D receptors, such as breast cancer, leukaemia, myelodysplasic syndromes and lymphomas, carcinomas of the Malpighian epithelial cells and gastrointestinal cancers, melanomas and osteosarcoma;
- of alopecia of various origins, in particular alopecia due to chemotherapy or radiation;
- of immune complaints, such as autoimmune diseases, type 1 diabetes mellitus, multiple sclerosis, lupus and lupus-type complaints, asthma, glomerulonephritis; of selective dysfunctions of the immune system such as AIDS, or of immune rejection;
- of endocrine complaints;
- of complaints **characterized by** abnormal management of intracellular calcium, and of pathologies in which calcium metabolism is involved, such as muscular ischaemia;
- of vitamin D deficiencies and other mineral homeostasis complaints in plasma and bone, such as rickets, osteomalacia, osteoporosis, in particular in the case of menopausal women, renal osteodystrophy and parathyroid function disorders;
- of disorders of the cardiovascular system such as arteriosclerosis or hypertension, as well as non-insulin-dependent diabetes.

11. Pharmaceutical composition, **characterized in that** it comprises, in a pharmaceutically acceptable support, at least one of the compounds as defined in any one of Claims 1 to 8.

12. Composition according to Claim 11, **characterized in that** the concentration of compound(s) according to one of Claims 1 to 8 is between 0.0001% and 5% by weight relative to the total weight of the composition.

13. Cosmetic composition, **characterized in that** it comprises, in a cosmetically acceptable support, at least one of the compounds as defined in any one of Claims 1 to 8.

14. Composition according to Claim 13, **characterized in that** the concentration of compound(s) is between 0.001% and 3% by weight relative to the total weight of the composition.

15. Use of a cosmetic composition as defined in either of Claims 13 and 14, for body or hair hygiene.
